# EUROPEAN PATENT SPECIFICATION

(11) **EP 1 825 005 B1**
(45) Date of publication and mention of the grant of the patent: **15.07.2015**
(21) Application number: 05852182.4
(22) Date of filing: 23.11.2005
(51) Int. Cl.: A61K 39/395, G01N 33/53, G01N 33/574, C07K 16/30, C07K 16/28, G01N 33/86

(54) **MER DIAGNOSTIC AND THERAPEUTIC AGENTS**
MER-DIAGNOSTISCHE UND -THERAPEUTISCHE MITTEL
AGENTS DIAGNOSTIQUES ET THERAPEUTIQUES A BASE DE MER

(30) Priority: 24.11.2004 US 630192 P
(43) Date of publication of application: 29.08.2007
(73) Proprietor: The Regents of the University of Colorado, a body corporate, Denver, CO 80203 (US)
(72) Inventor: GRAHAM, Douglas, Kim, Aurora, Colorado 80016 (US); SATHER, Susan, Louise, Denver, Colorado 80209 (US)
(74) Representative: Mallalieu, Catherine Louise
(86) International application number: PCT/US2005/042724
(87) International publication number: WO 2006/058202

(56) References cited:
- WO-A-95/33499
- US-A1- 2003 068 759
- EK SARA ET AL: "Mantle cell lymphomas express a distinct genetic signature affecting lymphocyte trafficking and growth regulation as compared with subpopulations of normal human B cells." CANCER RESEARCH 1 AUG 2002, vol. 62, no. 15, 1 August 2002 (2002-08-01), pages 4398-4405, XP002515126 ISSN: 0008-5472
- FENG WEI ET AL: "Mertk triggers uptake of photoreceptor outer segments during phagocytosis by cultured retinal pigment epithelial cells." THE JOURNAL OF BIOLOGICAL CHEMISTRY 10 MAY 2002, vol. 277, no. 19, 10 May 2002 (2002-05-10), pages 17016-17022, XP002515127 ISSN: 0021-9258
- COHEN PHILIP L ET AL: "Delayed apoptotic cell clearance and lupus-like autoimmunity in mice lacking the c-mer membrane tyrosine kinase." THE JOURNAL OF EXPERIMENTAL MEDICINE 1 JUL 2002, vol. 196, no. 1, 1 July 2002 (2002-07-01), pages 135-140, XP002515128 ISSN: 0022-1007
- GRAHAM DOUGLAS K ET AL: "Ectopic expression of the proto-oncogene Mer in pediatric T-cell acute lymphoblastic leukemia." CLINICAL CANCER RESEARCH : AN OFFICIAL JOURNAL OF THE AMERICAN ASSOCIATION FOR CANCER RESEARCH 1 MAY 2006, vol. 12, no. 9, 1 May 2006 (2006-05-01), pages 2662-2669, XP002515129 ISSN: 1078-0432
- SALZBERG DANA B ET AL: "Lymphoblastic leukemia/lymphoma in mice overexpressing the Mer receptor tyrosine kinase." BLOOD, vol. 106, no. 11, Part 1, November 2005 (2005-11), page 736A, XP002515130 & 47TH ANNUAL MEETING OF THE AMERICAN-SOCIETY-OF-HEMATOLOGY; ATLANTA, GA, USA; DECEMBER 10 -13, 2005 ISSN: 0006-4971
- CAMENISCH T.D. ET AL.: 'A Novel Receptor Tyrosine Kinase, Mer, Inhibits TNF-Production and Lipopolysaccharide-Induced Endotoxic Shock' JOURNAL OF IMMUNOLOGY vol. 162, no. 6, March 1999, pages 3498 - 3503, XP003013485
- NANDROT E. ET AL.: 'Homozygous Deletion in the Coding Sequence of the c-Mer Gene in RCS Rats Unravels General Mechanisms of Physiological Cell Adhesion and Apoptosis' NEUROBIOL. DIS. vol. 7, no. 6, PART B, December 2000, pages 586 - 599, XP003013486
- GEORGESCU M.-M. ET AL.: 'Biological Effects of the c-Mer Receptor Tyrosine Kinase in Hematopoietic Cells Depends on the Grb2 Binding Site in the Receptor and Activation of the NF-kappaB' MOL. CELL BIOL. vol. 19, no. 2, February 1999, pages 1171 - 1181, XP003013487
- DUNCAN JACQUE L ET AL: "An RCS-like retinal dystrophy phenotype in mer knockout mice.", INVESTIGATIVE OPHTHALMOLOGY & VISUAL SCIENCE FEB 2003 LNKD- PUBMED:12556419, vol. 44, no. 2, February 2003 (2003-02), pages 826-838, ISSN: 0146-0404
- MCHENRY CHRISTINA L ET AL: "MERTK arginine-844-cysteine in a patient with severe rod-cone dystrophy: loss of mutant protein function in transfected cells.", INVESTIGATIVE OPHTHALMOLOGY & VISUAL SCIENCE MAY 2004 LNKD- PUBMED:15111602, vol. 45, no. 5, May 2004 (2004-05), pages 1456-1463, ISSN: 0146-0404

### SEQUENCE LISTING

<110> Graham, Douglas K.. Sather, Susan L.
<120> MER DIAGNOSTIC AND THERAPEUTIC AGENTS
<130> 2848-86-PCT
<140> Not Yet Assigned
   <141> 2005-11-23
<150> 60/630,192 <151> 2004-11-24
<160> 8
<170> PatentIn version 3.3
<210> 1
   <211> 3632
   <212> DNA
   <213> Homo sapiens
<220>
   <221> CDS
   <222> (123)..(3122)
<400> 1
actcactgcc cgggccgccc ggacagggag cttcgctggc gcgcttggcc ggcgacagga    60
   caggttcggg acgtccatct gtccatccgt ccggagagaa attacagatc cgcagccccg   120
   gg atg ggg ccg gcc ccg ctg ccg ctg ctg ctg ggc ctc ttc ctc ccc      167
   gcg ctc tgg cgt aga gct atc act gag gca agg gaa gaa gcc aag cct    215
   tac ccg cta ttc ccg gga cct ttt cca ggg agc ctg caa act gac cac    263
   aca ccg ctg tta tcc ctt cct cac gcc agt ggg tac cag cct gcc ttg    311
   atg ttt tca cca acc cag cct gga aga cca cat aca gga aac gta gcc    359
   att ccc cag gtg acc tct gtc gaa tca aag ccc cta ccg cct ctt gcc    407
   ttc aaa cac aca gtt gga cac ata ata ctt tct gaa cat aaa ggt gtc    455
   aaa ttt aat tgc tca atc agt gta cct aat ata tac cag gac acc aca    503
   att tct tgg tgg aaa gat ggg aag gaa ttg ctt ggg gca cat cat gca    551
   att aca cag ttt tat cca gat gat gaa gtt aca gca ata atc gct tcc    599
   ttc agc ata acc agt gtg cag cgt tca gac aat ggg tcg tat atc tgt    647
   aag atg aaa ata aac aat gaa gag atc gtg tct gat ccc atc tac atc    695
   gaa gta caa gga ctt cct cac ttt act aag cag cct gag agc atg aat    743
   gtc acc aga aac aca gcc ttc aac ctc acc tgt cag gct gtg ggc ccg    791
   cct gag ccc gtc aac att ttc tgg gtt caa aac agt agc cgt gtt aac       839
   gaa cag cct gaa aaa tcc ccc tcc gtg cta act gtt cca ggc ctg acg       887
   gag atg gcg gtc ttc agt tgt gag gcc cac aat gac aaa ggg ctg acc       935
   gtg tcc aag gga gtg cag atc aac atc aaa gca att ccc tcc cca cca       983
   act gaa gtc agc atc cgt aac agc act gca cac agc att ctg atc tcc      1031
   tgg gtt cct ggt ttt gat gga tac tcc ccg ttc agg aat tgc agc att      1079
   cag gtc aag gaa gct gat ccg ctg agt aat ggc tca gtc atg att ttt      1127
   aac acc tct gcc tta cca cat ctg tac caa atc aag cag ctg caa gcc      1175
   ctg gct aat tac agc att ggt gtt tcc tgc atg aat gaa ata ggc tgg      1223
   tct gca gtg agc cct tgg att cta gcc agc acg act gaa gga gcc cca      1271
   tca gta gca cct tta aat gtc act gtg ttt ctg aat gaa tct agt gat      1319
   aat gtg gac atc aga tgg atg aag cct ccg act aag cag cag gat gga      1367
   gaa ctg gtg ggc tac cgg ata tcc cac gtg tgg cag agt gca ggg att      1415
   tcc aaa gag ctc ttg gag gaa gtt ggc cag aat ggc agc cga gct cgg      1463
   atc tct gtt caa gtc cac aat gct acg tgc aca gtg agg att gca gcc      1511
   gtc acc aga ggg gga gtt ggg ccc ttc agt gat cca gtg aaa ata ttt      1559
   atc cct gca cac ggt tgg gta gat tat gcc ccc tct tca act ccg gcg      1607
   cct ggc aac gca gat cct gtg ctc atc atc ttt ggc tgc ttt tgt gga      1655
   ttt att ttg att ggg ttg att tta tac atc tcc ttg gcc atc aga aaa      1703
   aga gtc cag gag aca aag ttt ggg aat gca ttc aca gag gag gat tct      1751
   gaa tta gtg gtg aat tat ata gca aag aaa tcc ttc tgt cgg cga gcc      1799
   att gaa ctt acc tta cat agc ttg gga gtc agt gag gaa cta caa aat      1847
   aaa cta gaa gat gtt gtg att gac agg aat ctt cta att ctt gga aaa      1895
   att ctg ggt gaa gga gag ttt ggg tct gta atg gaa gga aat ctt aag      1943
   cag gaa gat ggg acc tct ctg aaa gtg gca gtg aag acc atg aag ttg      1991
   gac aac tct tca cag cgg gag atc gag gag ttt ctc agt gag gca gcg      2039
   tgc atg aaa gac ttc agc cac cca aat gtc att cga ctt cta ggt gtg      2087
   tgt ata gaa atg agc tct caa ggc atc cca aag ccc atg gta att tta      2135
   ccc ttc atg aaa tac ggg gac ctg cat act tac tta ctt tat tcc cga      2183
   ttg gag aca gga cca aag cat att cct ctg cag aca cta ttg aag ttc      2231
   atg gtg gat att gcc ctg gga atg gag tat ctg agc aac agg aat ttt      2279
   ctt cat cga gat tta gct gct cga aac tgc atg ttg cga gat gac atg      2327
   act gtc tgt gtt gcg gac ttc ggc ctc tct aag aag att tac agt ggc      2375
   gat tat tac cgc caa ggc cgc att gct aag atg cct gtt aaa tgg atc      2423
   gcc ata gaa agt ctt gca gac cga gtc tac aca agt aaa agt gat gtg      2471
   tgg gca ttt ggc gtg acc atg tgg gaa ata gct acg cgg gga atg act      2519
   ccc tat cct ggg gtc cag aac cat gag atg tat gac tat ctt ctc cat      2567
   ggc cac agg ttg aag cag ccc gaa gac tgc ctg gat gaa ctg tat gaa      2615
   ata atg tac tct tgc tgg aga acc gat ccc tta gac cgc ccc acc ttt      2663
   tca gta ttg agg ctg cag cta gaa aaa ctc tta gaa agt ttg cct gac      2711
   gtt cgg aac caa gca gac gtt att tac gtc aat aca cag ttg ctg gag      2759
   agc tct gag ggc ctg gcc cag ggc tcc acc ctt gct cca ctg gac ttg      2807
   aac atc gac cct gac tct ata att gcc tcc tgc act ccc cgc gct gcc      2855
   atc agt gtg gtc aca gca gaa gtt cat gac agc aaa cct cat gaa gga      2903
   cgg tac atc ctg aat ggg ggc agt gag gaa tgg gaa gat ctg act tct      2951
   gcc ccc tct gct gca gtc aca gct gaa aag aac agt gtt tta ccg ggg      2999
   gag aga ctt gtt agg aat ggg gtc tcc tgg tcc cat tcg agc atg ctg      3047
   ccc ttg gga agc tca ttg ccc gat gaa ctt ttg ttt gct gac gac tcc      3095
   tca gaa ggc tca gaa gtc ctg atg tga ggagaggtgc ggggagacat            3142
   tccaaaaatc aagccaattc ttctgctgta ggagaatcca attgtacctg atgtttttgg   3202
   tatttgtctt ccttaccaag tgaactccat ggccccaaag caccagatga atgttgttaa   3262
   gtaagctgtc attaaaaata cataatatat atttatttaa agagaaaaaa tatgtgtata   3322
   tcatggaaaa agacaaggat attttaataa aacattactt atttcatttc acttatcttg   3382
   catatcttaa aattaagctt cagctgctcc ttgatattaa catttgtaca gagttgaagt   3442
   tgttttttca agttcttttc tttttcatga ctattaaatg taaaaatatt tgtaaaatga   3502
   aatgccatat ttgacttggc ttctggtctt gatgtatttg ataagaatga ttcattcaat   3562
   gtttaaagtt gtataactga ttaattttct gatatggctt cctaataaaa tatgaataag   3622
   gaagaaaaaa                                                           3632
<210> 2
   <211> 999
   <212> PRT
   <213> Homo sapiens
<400> 2
<210> 3
   <211> 3564
   <212> DNA
   <213> Mus musculus
<220>
   <221> CDS
   <222> (50)..(3034)
<400> 3
   tggatacgtg catctgtccg gagagaactg ccagatccgc ggccccgcg atg gtt ctg      58
                                                         Met Val Leu
                                                         1
   gcc cca ctg cta ctg ggg ctg ctg ctg cta ccc gcg ctc tgg agt gga       106
   Ala Pro Leu Leu Leu Gly Leu Leu Leu Leu Pro Ala Leu Trp Ser Gly
       5                   10                  15
   ggc act gcc gag aag tgg gaa gag acc gag cta gat cag cta ttt tca       154
   Gly Thr Ala Glu Lys Trp Glu Glu Thr Glu Leu Asp Gln Leu Phe Ser
   20                  25                  30                  35
   ggg cct tta cca ggg aga ctc cca gtc aac cac agg cca ttc tct gct       202
   Gly Pro Leu Pro Gly Arg Leu Pro Val Asn His Arg Pro Phe Ser Ala
                   40                  45                  50
   cct cac tcc agc cgg gac cag ctg cca cca ccc cag act gga aga tca       250
   Pro His Ser Ser Arg Asp Gln Leu Pro Pro Pro Gln Thr Gly Arg Ser
               55                  60                  65
   cat cca gca cac aca gcc gct ccc cag gtg acc tcc aca gca tca aag       298
   His Pro Ala His Thr Ala Ala Pro Gln Val Thr Ser Thr Ala Ser Lys
           70                  75                  80
   ctc cta cct cct gtt gcg ttt aat cac acc att gga cac ata gta ctg       346
   Leu Leu Pro Pro Val Ala Phe Asn His Thr Ile Gly His Ile Val Leu
       85                  90                  95
   tcg gaa cat aaa aat gtc aaa ttt aat tgc tcc atc aat att cct aac       394
   Ser Glu His Lys Asn Val Lys Phe Asn Cys Ser Ile Asn Ile Pro Asn
   100                 105                 110                 115
   aca tac caa gaa aca gct ggc att tca tgg tgg aaa gat gga aag gaa      442
   Thr Tyr Gln Glu Thr Ala Gly Ile Ser Trp Trp Lys Asp Gly Lys Glu
                   120                 125                 130
   ttg ctc ggg gca cat cat tca atc aca cag ttt tat cct gat gag gaa      490
   Leu Leu Gly Ala His His Ser Ile Thr Gln Phe Tyr Pro Asp Glu Glu
               135                 140                 145
   ggg gta tca ata att gca ttg ttc agc ata gcc agt gtg cag cgc tca      538
   Gly Val Ser Ile Ile Ala Leu Phe Ser Ile Ala Ser Val Gln Arg Ser
           150                 155                 160
   gac aat ggg tcg tac ttc tgt aag atg aag gtg aac aat aga gag att      586
   Asp Asn Gly Ser Tyr Phe Cys Lys Met Lys Val Asn Asn Arg Glu Ile
       165                 170                 175
   gta tct gat ccc ata tac gtg gaa gtt caa gga ctc cct tac ttt att      634
   Val Ser Asp Pro Ile Tyr Val Glu Val Gln Gly Leu Pro Tyr Phe Ile
   180                 185                 190                 195
   aag cag cct gag agt gtg aat gtc acc aga aac aca gcc ttc aac ctc      682
   Lys Gln Pro Glu Ser Val Asn Val Thr Arg Asn Thr Ala Phe Asn Leu
                   200                 205                 210
   acc tgc cag gcc gtg ggc cct cct gag ccc gtc aat atc ttc tgg gtt      730
   Thr Cys Gln Ala Val Gly Pro Pro Glu Pro Val Asn Ile Phe Trp Val
               215                 220                 225
   caa aat agc agc cgt gtt aat gaa aaa ccg gaa agg tcc ccg tct gtc      778
   Gln Asn Ser Ser Arg Val Asn Glu Lys Pro Glu Arg Ser Pro Ser Val
           230                 235                 240
   cta acc gta cct ggt ctg aca gag aca gca gtc ttc agc tgt gag gcc      826
   Leu Thr Val Pro Gly Leu Thr Glu Thr Ala Val Phe Ser Cys Glu Ala
       245                 250                 255
   cac aat gac aaa gga ctg acg gtg tcc aag ggt gta cat atc aac atc      874
   His Asn Asp Lys Gly Leu Thr Val Ser Lys Gly Val His Ile Asn Ile
   260                 265                 270                 275
   aaa gta atc ccc tcc ccg ccc act gaa gtc cat atc ctc aac agt aca      922
   Lys Val Ile Pro Ser Pro Pro Thr Glu Val His Ile Leu Asn Ser Thr
                   280                 285                 290
   gca cac agc atc ctg gtc tcc tgg gtc cct ggt ttt gat ggc tac tcc      970
   Ala His Ser Ile Leu Val Ser Trp Val Pro Gly Phe Asp Gly Tyr Ser
               295                 300                 305
   cca ctt cag aac tgc agc att cag gtc aag gaa gct gac cgg ctg agt     1018
   Pro Leu Gln Asn Cys Ser Ile Gln Val Lys Glu Ala Asp Arg Leu Ser
           310                 315                 320
   aat ggc tca gtc atg gtt ttt aat acc tct gct tcg cca cat ctg tat     1066
   Asn Gly Ser Val Met Val Phe Asn Thr Ser Ala Ser Pro His Leu Tyr
       325                 330                 335
   gag atc cag cag ctg caa gcc ctg gct aat tac agc atc gct gtg tcc     1114
   Glu Ile Gln Gln Leu Gln Ala Leu Ala Asn Tyr Ser Ile Ala Val Ser
   340                 345                 350                 355
   tgt cgg aat gag att ggc tgg tct gca gta agc cct tgg att ctg gcc     1162
   Cys Arg Asn Glu Ile Gly Trp Ser Ala Val Ser Pro Trp Ile Leu Ala
                   360                 365                 370
   agc aca aca gaa gga gct cca tct gta gca cct tta aac atc act gtg     1210
   Ser Thr Thr Glu Gly Ala Pro Ser Val Ala Pro Leu Asn Ile Thr Val
               375                 380                 385
   ttt ctg aac gaa tct aac aat atc ctg gat att aga tgg acg aag cct     1258
   Phe Leu Asn Glu Ser Asn Asn Ile Leu Asp Ile Arg Trp Thr Lys Pro
           390                 395                 400
   cca att aag cgg cag gat ggg gaa ctg gtg ggc tac cgg ata tct cac     1306
   Pro Ile Lys Arg Gln Asp Gly Glu Leu Val Gly Tyr Arg Ile Ser His
       405                 410                 415
   gtg tgg gaa agc gca ggg act tac aaa gag ctt tct gaa gaa gtc agc     1354
   Val Trp Glu Ser Ala Gly Thr Tyr Lys Glu Leu Ser Glu Glu Val Ser
   420                 425                 430                 435
   cag aat ggc agc tgg gct cag att cct gtc caa atc cac aat gcc acc     1402
   Gln Asn Gly Ser Trp Ala Gln Ile Pro Val Gln Ile His Asn Ala Thr
                   440                 445                 450
   tgc aca gtg aga atc gcg gcc att act aaa ggg ggc atc ggg ccc ttc      1450
   Cys Thr Val Arg Ile Ala Ala Ile Thr Lys Gly Gly Ile Gly Pro Phe
               455                 460                 465
   agt gag cca gtg aat atc atc att cct gaa cac agt aag gta gat tac      1498
   Ser Glu Pro Val Asn Ile Ile Ile Pro Glu His Ser Lys Val Asp Tyr
           470                 475                 480
   gca ccc tcg tca acc cca gcc cct ggc aac acc gac tct atg ttc atc      1546
   Ala Pro Ser Ser Thr Pro Ala Pro Gly Asn Thr Asp Ser Met Phe Ile
       485                 490                 495
   atc ctc ggc tgc ttc tgt gga ttc att tta atc ggg tta att ttg tgt      1594
   Ile Leu Gly Cys Phe Cys Gly Phe Ile Leu Ile Gly Leu Ile Leu Cys
   500                 505                 510                 515
   att tct ctg gcc ctc aga agg aga gtc cag gaa aca aag ttt ggg gga      1642
   Ile Ser Leu Ala Leu Arg Arg Arg Val Gln Glu Thr Lys Phe Gly Gly
                   520                 525                 530
   gca ttc tct gag gag gat tcc caa ctg gtc gta aat tat aga gcg aag      1690
   Ala Phe Ser Glu Glu Asp Ser Gln Leu Val Val Asn Tyr Arg Ala Lys
               535                 540                 545
   aag tcc ttc tgc cgg cga gcc atc gag ctt acc ttg cag agc ctg gga      1738
   Lys Ser Phe Cys Arg Arg Ala Ile Glu Leu Thr Leu Gln Ser Leu Gly
           550                 555                 560
   gtg agc gag gag ctg cag aat aag ctg gaa gat gtt gtg att gac aga      1786
   Val Ser Glu Glu Leu Gln Asn Lys Leu Glu Asp Val Val Ile Asp Arg
       565                 570                 575
   aac ctt ctg gtt ctc ggc aaa gtt ctg ggt gaa gga gag ttt ggg tct      1834
   Asn Leu Leu Val Leu Gly Lys Val Leu Gly Glu Gly Glu Phe Gly Ser
   580                 585                 590                 595
   gta atg gaa gga aat ttg aag caa gaa gat ggg act tct cag aag gtg      1882
   Val Met Glu Gly Asn Leu Lys Gln Glu Asp Gly Thr Ser Gln Lys Val
                   600                 605                 610
   gca gtg aag acc atg aag ttg gac aac ttt tct caa cgg gag atc gag      1930
   Ala Val Lys Thr Met Lys Leu Asp Asn Phe Ser Gln Arg Glu Ile Glu
               615                 620                 625
   gag ttt ctc agc gaa gca gca tgc atg aaa gac ttc aac cac cca aat      1978
   Glu Phe Leu Ser Glu Ala Ala Cys Met Lys Asp Phe Asn His Pro Asn
           630                 635                 640
   gtc atc cga ctt cta ggc gtg tgt ata gaa ctg agc tct caa ggc atc      2026
   Val Ile Arg Leu Leu Gly Val Cys Ile Glu Leu Ser Ser Gln Gly Ile
       645                 650                 655
   ccg aag ccc atg gtg att tta ccc ttc atg aaa tac gga gac ctc cac      2074
   Pro Lys Pro Met Val Ile Leu Pro Phe Met Lys Tyr Gly Asp Leu His
   660                 665                 670                 675
   acc ttc ctg tta tat tcc cga tta aac aca gga ccc aag tac att cac      2122
   Thr Phe Leu Leu Tyr Ser Arg Leu Asn Thr Gly Pro Lys Tyr Ile His
                   680                 685                 690
   ctg cag aca cta ctg aag ttc atg atg gac att gcc cag gga atg gag      2170
   Leu Gln Thr Leu Leu Lys Phe Met Met Asp Ile Ala Gln Gly Met Glu
               695                 700                 705
   tat ctg agc aac agg aat ttt ctt cat agg gat ttg gca gct cga aac      2218
   Tyr Leu Ser Asn Arg Asn Phe Leu His Arg Asp Leu Ala Ala Arg Asn
           710                 715                 720
   tgc atg ttg cgg gat gac atg act gtc tgc gtg gca gac ttt ggc ctc      2266
   Cys Met Leu Arg Asp Asp Met Thr Val Cys Val Ala Asp Phe Gly Leu
       725                 730                 735
   tca aag aag att tac agt ggt gat tat tac cgc caa ggc cgc att gcc      2314
   Ser Lys Lys Ile Tyr Ser Gly Asp Tyr Tyr Arg Gln Gly Arg Ile Ala
   740                 745                 750                 755
   aaa atg cct gtg aag tgg atc gcc atc gag agc ctg gcg gac cga gtc      2362
   Lys Met Pro Val Lys Trp Ile Ala Ile Glu Ser Leu Ala Asp Arg Val
                   760                 765                 770
   tac aca agc aaa agt gac gtg tgg gct ttt ggc gtg acc atg tgg gaa      2410
   Tyr Thr Ser Lys Ser Asp Val Trp Ala Phe Gly Val Thr Met Trp Glu
               775                 780                 785
   ata aca aca cgg gga atg act ccc tat ccc gga gtt cag aac cat gag      2458
   Ile Thr Thr Arg Gly Met Thr Pro Tyr Pro Gly Val Gln Asn His Glu
           790                 795                 800
   atg tac gac tac ctt ctc cac ggc cac agg ctg aag cag cct gag gac      2506
   Met Tyr Asp Tyr Leu Leu His Gly His Arg Leu Lys Gln Pro Glu Asp
       805                 810                 815
   tgc ttg gat gaa ctg tat gac atc atg tac tct tgc tgg agt gct gat      2554
   Cys Leu Asp Glu Leu Tyr Asp Ile Met Tyr Ser Cys Trp Ser Ala Asp
   820                 825                 830                 835
   ccc ttg gat cga ccc acc ttc tct gtg ttg agg ctg cag ctg gaa aag      2602
   Pro Leu Asp Arg Pro Thr Phe Ser Val Leu Arg Leu Gln Leu Glu Lys
                   840                 845                 850
   ctc tcc gag agt ttg cct gat gcg cag gac aaa gaa tcc atc atc tac      2650
   Leu Ser Glu Ser Leu Pro Asp Ala Gln Asp Lys Glu Ser Ile Ile Tyr
               855                 860                 865
   atc aat acc cag ttg cta gag agc tgc gag ggc ata gcc aat ggg ccc      2698
   Ile Asn Thr Gln Leu Leu Glu Ser Cys Glu Gly Ile Ala Asn Gly Pro
           870                 875                 880
   tca ctc acg ggg cta gac atg aac att gac cct gac tcc atc att gcc      2746
   Ser Leu Thr Gly Leu Asp Met Asn Ile Asp Pro Asp Ser Ile Ile Ala
       885                 890                 895
   tct tgc aca cca ggc gct gcc gtc agc gtg gtc acg gca gaa gtt cac      2794
   Ser Cys Thr Pro Gly Ala Ala Val Ser Val Val Thr Ala Glu Val His
   900                 905                 910                 915
   gag aac aac ctt cgt gag gaa aga tac atc ttg aat ggg ggc aat gag      2842
   Glu Asn Asn Leu Arg Glu Glu Arg Tyr Ile Leu Asn Gly Gly Asn Glu
                   920                 925                 930
   gaa tgg gaa gat gtg tcc tcc act cct ttt gct gca gtc aca cct gaa      2890
   Glu Trp Glu Asp Val Ser Ser Thr Pro Phe Ala Ala Val Thr Pro Glu
               935                 940                 945
   aag gat ggt gtc tta ccg gag gac aga ctc acc aaa aat ggc gtc tcc      2938
   Lys Asp Gly Val Leu Pro Glu Asp Arg Leu Thr Lys Asn Gly Val Ser
           950                 955                 960
   tgg tct cac cat agt aca cta ccc ttg ggg agc cca tca cca gat gaa      2986
   Trp Ser His His Ser Thr Leu Pro Leu Gly Ser Pro Ser Pro Asp Glu
       965                 970                 975
   ctt tta ttt gta gat gac tcc ttg gaa gac tct gaa gtt ctg atg tga      3034
   Leu Leu Phe Val Asp Asp Ser Leu Glu Asp Ser Glu Val Leu Met
   980                 985                 990
   agccagctga gaggaggcat gagagaacca agcaaataca gcttcctggg atctggtggt    3094
   cttagatact ttgttattgc tctgataaaa catcatgacc aaggcaatct tcaagagaaa    3154
   gtgtttaact aggtttactg tttcaggggg ttagagtcta tgattgcaga aggaagttat    3214
   gatggcagga acagctgagt gcttatatct ttaagagcaa gcaggagaca gagagcactc    3274
   tgggaaatgg cacctatctt ttgaaacctc aaagcctgct cccagtgaca aaagtccttc    3334
   aacaggccat acctcctaat ccttcccaaa caattccacc aactggagac caaacattca    3394
   aatgtatgcg cgtattgggg ccattcttaa gcaaaccact acactggtta tacctgaggt    3454
   tttggtactt gttttcctta ccaagtagag ttcatggccg gacagcacca ggtgaaagct    3514
   gtcaagtcag gtttgcaaat acataaccaa ggtcttgaga gctcgtgccg                3564
<210> 4
   <211> 994
   <212> PRT
   <213> Mus musculus
<400> 4
   Met Val Leu Ala Pro Leu Leu Leu Gly Leu Leu Leu Leu Pro Ala Leu
   1               5                   10                  15
   Trp Ser Gly Gly Thr Ala Glu Lys Trp Glu Glu Thr Glu Leu Asp Gln
               20                  25                  30
   Leu Phe Ser Gly Pro Leu Pro Gly Arg Leu Pro Val Asn His Arg Pro
           35                  40                  45
   Phe Ser Ala Pro His Ser Ser Arg Asp Gln Leu Pro Pro Pro Gln Thr
       50                  55                  60
   Gly Arg Ser His Pro Ala His Thr Ala Ala Pro Gln Val Thr Ser Thr
   65                  70                  75                  80
   Ala Ser Lys Leu Leu Pro Pro Val Ala Phe Asn His Thr Ile Gly His
                   85                  90                  95
   Ile Val Leu Ser Glu His Lys Asn Val Lys Phe Asn Cys Ser Ile Asn
               100                 105                 110
   Ile Pro Asn Thr Tyr Gln Glu Thr Ala Gly Ile Ser Trp Trp Lys Asp
           115                 120                 125
   Gly Lys Glu Leu Leu Gly Ala His His Ser Ile Thr Gln Phe Tyr Pro
       130                 135                 140
   Asp Glu Glu Gly Val Ser Ile Ile Ala Leu Phe Ser Ile Ala Ser Val
   145                 150                 155                 160
   Gln Arg Ser Asp Asn Gly Ser Tyr Phe Cys Lys Met Lys Val Asn Asn
                   165                 170                 175
   Arg Glu Ile Val Ser Asp Pro Ile Tyr Val Glu Val Gln Gly Leu Pro
               180                 185                 190
   Tyr Phe Ile Lys Gln Pro Glu Ser Val Asn Val Thr Arg Asn Thr Ala
           195                 200                 205
   Phe Asn Leu Thr Cys Gln Ala Val Gly Pro Pro Glu Pro Val Asn Ile
       210                 215                 220
   Phe Trp Val Gln Asn Ser Ser Arg Val Asn Glu Lys Pro Glu Arg Ser
   225                 230                 235                 240
   Pro Ser Val Leu Thr Val Pro Gly Leu Thr Glu Thr Ala Val Phe Ser
                   245                 250                 255
   Cys Glu Ala His Asn Asp Lys Gly Leu Thr Val Ser Lys Gly Val His
               260                 265                 270
   Ile Asn Ile Lys Val Ile Pro Ser Pro Pro Thr Glu Val His Ile Leu
           275                 280                 285
   Asn Ser Thr Ala His Ser Ile Leu Val Ser Trp Val Pro Gly Phe Asp
       290                 295                 300
   Gly Tyr Ser Pro Leu Gln Asn Cys Ser Ile Gln Val Lys Glu Ala Asp
   305                 310                 315                 320
   Arg Leu Ser Asn Gly Ser Val Met Val Phe Asn Thr Ser Ala Ser Pro
                   325                 330                 335
   His Leu Tyr Glu Ile Gln Gln Leu Gln Ala Leu Ala Asn Tyr Ser Ile
               340                 345                 350
   Ala Val Ser Cys Arg Asn Glu Ile Gly Trp Ser Ala Val Ser Pro Trp
           355                 360                 365
   Ile Leu Ala Ser Thr Thr Glu Gly Ala Pro Ser Val Ala Pro Leu Asn
       370                 375                 380
   Ile Thr Val Phe Leu Asn Glu Ser Asn Asn Ile Leu Asp Ile Arg Trp
   385                 390                 395                 400
   Thr Lys Pro Pro Ile Lys Arg Gln Asp Gly Glu Leu Val Gly Tyr Arg
                   405                 410                 415
   Ile Ser His Val Trp Glu Ser Ala Gly Thr Tyr Lys Glu Leu Ser Glu
               420                 425                 430
   Glu Val Ser Gln Asn Gly Ser Trp Ala Gln Ile Pro Val Gln Ile His
           435                 440                 445
   Asn Ala Thr Cys Thr Val Arg Ile Ala Ala Ile Thr Lys Gly Gly Ile
       450                 455                 460
   Gly Pro Phe Ser Glu Pro Val Asn Ile Ile Ile Pro Glu His Ser Lys
   465                 470                 475                 480
   Val Asp Tyr Ala Pro Ser Ser Thr Pro Ala Pro Gly Asn Thr Asp Ser
                   485                 490                 495
   Met Phe Ile Ile Leu Gly Cys Phe Cys Gly Phe Ile Leu Ile Gly Leu
               500                 505                 510
   Ile Leu Cys Ile Ser Leu Ala Leu Arg Arg Arg Val Gln Glu Thr Lys
           515                 520                 525
   Phe Gly Gly Ala Phe Ser Glu Glu Asp Ser Gln Leu Val Val Asn Tyr
       530                 535                 540
   Arg Ala Lys Lys Ser Phe Cys Arg Arg Ala Ile Glu Leu Thr Leu Gln
   545                 550                 555                 560
   Ser Leu Gly Val Ser Glu Glu Leu Gln Asn Lys Leu Glu Asp Val Val
                   565                 570                 575
   Ile Asp Arg Asn Leu Leu Val Leu Gly Lys Val Leu Gly Glu Gly Glu
               580                 585                 590
   Phe Gly Ser Val Met Glu Gly Asn Leu Lys Gln Glu Asp Gly Thr Ser
           595                 600                 605
   Gln Lys Val Ala Val Lys Thr Met Lys Leu Asp Asn Phe Ser Gln Arg
       610                 615                 620
   Glu Ile Glu Glu Phe Leu Ser Glu Ala Ala Cys Met Lys Asp Phe Asn
   625                 630                 635                 640
   His Pro Asn Val Ile Arg Leu Leu Gly Val Cys Ile Glu Leu Ser Ser
                   645                 650                 655
   Gln Gly Ile Pro Lys Pro Met Val Ile Leu Pro Phe Met Lys Tyr Gly
               660                 665                 670
   Asp Leu His Thr Phe Leu Leu Tyr Ser Arg Leu Asn Thr Gly Pro Lys
           675                 680                 685
   Tyr Ile His Leu Gln Thr Leu Leu Lys Phe Met Met Asp Ile Ala Gln
       690                 695                 700
   Gly Met Glu Tyr Leu Ser Asn Arg Asn Phe Leu His Arg Asp Leu Ala
   705                 710                 715                 720
   Ala Arg Asn Cys Met Leu Arg Asp Asp Met Thr Val Cys Val Ala Asp
                   725                 730                 735
   Phe Gly Leu Ser Lys Lys Ile Tyr Ser Gly Asp Tyr Tyr Arg Gln Gly
               740                 745                 750
   Arg Ile Ala Lys Met Pro Val Lys Trp Ile Ala Ile Glu Ser Leu Ala
           755                 760                 765
   Asp Arg Val Tyr Thr Ser Lys Ser Asp Val Trp Ala Phe Gly Val Thr
       770                 775                 780
   Met Trp Glu Ile Thr Thr Arg Gly Met Thr Pro Tyr Pro Gly Val Gln
   785                 790                 795                 800
   Asn His Glu Met Tyr Asp Tyr Leu Leu His Gly His Arg Leu Lys Gln
                   805                 810                 815
   Pro Glu Asp Cys Leu Asp Glu Leu Tyr Asp Ile Met Tyr Ser Cys Trp
               820                 825                 830
   Ser Ala Asp Pro Leu Asp Arg Pro Thr Phe Ser Val Leu Arg Leu Gln
           835                 840                 845
   Leu Glu Lys Leu Ser Glu Ser Leu Pro Asp Ala Gln Asp Lys Glu Ser
       850                 855                 860
   Ile Ile Tyr Ile Asn Thr Gln Leu Leu Glu Ser Cys Glu Gly Ile Ala
   865                 870                 875                 880
   Asn Gly Pro Ser Leu Thr Gly Leu Asp Met Asn Ile Asp Pro Asp Ser
                   885                 890                 895
   Ile Ile Ala Ser Cys Thr Pro Gly Ala Ala Val Ser Val Val Thr Ala
               900                 905                 910
   Glu Val His Glu Asn Asn Leu Arg Glu Glu Arg Tyr Ile Leu Asn Gly
           915                 920                 925
   Gly Asn Glu Glu Trp Glu Asp Val Ser Ser Thr Pro Phe Ala Ala Val
       930                 935                 940
   Thr Pro Glu Lys Asp Gly Val Leu Pro Glu Asp Arg Leu Thr Lys Asn
   945                 950                 955                 960
   Gly Val Ser Trp Ser His His Ser Thr Leu Pro Leu Gly Ser Pro Ser
                   965                 970                 975
   Pro Asp Glu Leu Leu Phe Val Asp Asp Ser Leu Glu Asp Ser Glu Val
               980                 985                 990
   Leu Met
<210> 5
   <211> 3024
   <212> DNA
   <213> Rattus norvegicus
<220>
   <221> CDS
   <222> (40)..(3024)
<400> 5
   catctgtccg gagagaactg ccagatccgc ggccccgcg atg gtt ctg gcc cca       54
                                              Met Val Leu Ala Pro
                                              1               5
   cta ctg ctg ggg ctg ctg ctg cta tcc gca ctc tgg aat gga ggc act     102
   Leu Leu Leu Gly Leu Leu Leu Leu Ser Ala Leu Trp Asn Gly Gly Thr
                   10                  15                  20
   gct gaa aag gag gaa gaa atc aag cca gat cag cca ttt tca ggg cct      150
   Ala Glu Lys Glu Glu Glu Ile Lys Pro Asp Gln Pro Phe Ser Gly Pro
               25                  30                  35
   tta cca ggg agc cta cca gct gac cac agg cca ttc ttc gcc cct cac      198
   Leu Pro Gly Ser Leu Pro Ala Asp His Arg Pro Phe Phe Ala Pro His
           40                  45                  50
   tcc agt ggg gac cag ctg tca cca tcc cag act gga aga tca cat cca      246
   Ser Ser Gly Asp Gln Leu Ser Pro Ser Gln Thr Gly Arg Ser His Pro
       55                  60                  65
   gca cac aca gcc act ccc cag atg acc tct gca gca tca aac ctc ctg      294
   Ala His Thr Ala Thr Pro Gln Met Thr Ser Ala Ala Ser Asn Leu Leu
   70                  75                  80                  85
   cct cct gtt gca ttt aaa aac aca att gga cgc ata gta ctt tcg gaa      342
   Pro Pro Val Ala Phe Lys Asn Thr Ile Gly Arg Ile Val Leu Ser Glu
                   90                  95                  100
   cat aaa agt gtc aaa ttt aat tgc tcg atc aac att cct aac gtg tat      390
   His Lys Ser Val Lys Phe Asn Cys Ser Ile Asn Ile Pro Asn Val Tyr
               105                 110                 115
   caa gaa aca gct ggc att tcg tgg tgg aaa gat gga aag gaa ctg ctt      438
   Gln Glu Thr Ala Gly Ile Ser Trp Trp Lys Asp Gly Lys Glu Leu Leu
           120                 125                 130
   ggg gca cat cat tca atc aca cag ttt tat cct gat gag gaa ggg gta      486
   Gly Ala His His Ser Ile Thr Gln Phe Tyr Pro Asp Glu Glu Gly Val
       135                 140                 145
   tca ata att gca ttg ttc agc ata acc agt gtg cag cgc tca gac aat      534
   Ser Ile Ile Ala Leu Phe Ser Ile Thr Ser Val Gln Arg Ser Asp Asn
   150                 155                 160                 165
   ggg tcg tac atc tgt aag atg aag gtg aac gat aga gag gtt gtg tct      582
   Gly Ser Tyr Ile Cys Lys Met Lys Val Asn Asp Arg Glu Val Val Ser
                   170                 175                 180
   gat ccc ata tac gtg gaa gtc caa gga ctc cct tac ttt act aag cag      630
   Asp Pro Ile Tyr Val Glu Val Gln Gly Leu Pro Tyr Phe Thr Lys Gln
               185                 190                 195
   cct gag agc gtg aat gtc acc aga aac aca gcc ttc aac ctc acc tgc      678
   Pro Glu Ser Val Asn Val Thr Arg Asn Thr Ala Phe Asn Leu Thr Cys
           200                 205                 210
   cag gct gtg gga ccc cct gag ccc gtc aac atc ttc tgg gtt caa aat      726
   Gln Ala Val Gly Pro Pro Glu Pro Val Asn Ile Phe Trp Val Gln Asn
       215                 220                 225
   agc agc cgt gtt aat gaa aat cca gaa agg tcc ccg tct gtc cta act      774
   Ser Ser Arg Val Asn Glu Asn Pro Glu Arg Ser Pro Ser Val Leu Thr
   230                 235                 240                 245
   gtc gct ggt ctg aca gag acc gca gtc ttc agc tgt gag gcc cac aac      822
   Val Ala Gly Leu Thr Glu Thr Ala Val Phe Ser Cys Glu Ala His Asn
                   250                 255                 260
   gac aaa gga ctg acg gtg tcc aag ggt gta cag atc aac atc aaa gtc      870
   Asp Lys Gly Leu Thr Val Ser Lys Gly Val Gln Ile Asn Ile Lys Val
               265                 270                 275
   atc cct tcc cca ccc act gaa gtc cat atc ctc aac agc aca gcg cac      918
   Ile Pro Ser Pro Pro Thr Glu Val His Ile Leu Asn Ser Thr Ala His
           280                 285                 290
   agc atc ctg gtc tcc tgg gtc cca ggg ttt gat ggc tac tcc cca ctt      966
   Ser Ile Leu Val Ser Trp Val Pro Gly Phe Asp Gly Tyr Ser Pro Leu
       295                 300                 305
   cag aac tgc agc att cag gtc aag gaa gct gac cag ctg agt aat ggc     1014
   Gln Asn Cys Ser Ile Gln Val Lys Glu Ala Asp Gln Leu Ser Asn Gly
   310                 315                 320                 325
   tca gtc atg gtt ttt aat acc tct gct tcg cca cat ctg tat gaa gtc     1062
   Ser Val Met Val Phe Asn Thr Ser Ala Ser Pro His Leu Tyr Glu Val
                   330                 335                 340
   cag cag ctg caa gcc ctg gct aat tac agc gtc act gtg tcc tgt cgg     1110
   Gln Gln Leu Gln Ala Leu Ala Asn Tyr Ser Val Thr Val Ser Cys Arg
                       345                      350                      355
   aat gag att ggc tgg tct gca gtg agc cct tgg atc ctg gcc agc act      1158
   Asn Glu Ile Gly Trp Ser Ala Val Ser Pro Trp Ile Leu Ala Ser Thr
               360                      365                      370
   aca gaa gga gct cca gca gtg gcg cct cta aac atc act gtg ttt ctg      1206
   Thr Glu Gly Ala Pro Ala Val Ala Pro Leu Asn Ile Thr Val Phe Leu
           375                      380                      385
   aac gaa tcc agc aac aac ctg gaa atc aga tgg acg aag cct cca att      1254
   Asn Glu Ser Ser Asn Asn Leu Glu Ile Arg Trp Thr Lys Pro Pro Ile
   390                      395                      400                      405
   aag cgg cag gat ggg gaa ctg gtg ggc tac cgg ata tct cac gtg tgg      1302
   Lys Arg Gln Asp Gly Glu Leu Val Gly Tyr Arg Ile Ser His Val Trp
                   410                      415                      420
   gag agt gcg ggg act tcc aaa gag ctt tct gaa gaa gtc agc cag aat      1350
   Glu Ser Ala Gly Thr Ser Lys Glu Leu Ser Glu Glu Val Ser Gln Asn
                       425                      430                      435
   ggc agc tgg gct cag gtt cct gtc caa atg cac aat gcc acc tgc aca      1398
   Gly Ser Trp Ala Gln Val Pro Val Gln Met His Asn Ala Thr Cys Thr
               440                      445                      450
   gtg aga atc gcc gtc atc act aaa ggg ggc att ggg ccc ttc agt gag      1446
   Val Arg Ile Ala Val Ile Thr Lys Gly Gly Ile Gly Pro Phe Ser Glu
           455                      460                      465
   cca gtg gac gta gcc att ccg gag cac agt agg gta gat tac gca ccc      1494
   Pro Val Asp Val Ala Ile Pro Glu His Ser Arg Val Asp Tyr Ala Pro
   470                      475                      480                      485
   tca tca acc cca gcc cct ggc aac acc gag tct atg ctc atc atc ctg      1542
   Ser Ser Thr Pro Ala Pro Gly Asn Thr Glu Ser Met Leu Ile Ile Leu
                   490                      495                      500
   ggc tgc ttc tgc ggg ttt gtt ctc atg ggg ttg att ctg tat ctt tct      1590
   Gly Cys Phe Cys Gly Phe Val Leu Met Gly Leu Ile Leu Tyr Leu Ser
                   505                      510                      515
   ctg gcc atc aaa agg aga gtc cag gaa aca aag ttt ggg ggt gca ttc      1638
   Leu Ala Ile Lys Arg Arg Val Gln Glu Thr Lys Phe Gly Gly Ala Phe
               520                      525                      530
   tcc gag gag gac tcc caa tta gtt gta aat tac aga gca aag aag tcc      1686
   Ser Glu Glu Asp Ser Gln Leu Val Val Asn Tyr Arg Ala Lys Lys Ser
           535                      540                      545
   ttc tgt cgg cga gcc atc gag ctt acc ttg caa agc ctg ggg gtg agc      1734
   Phe Cys Arg Arg Ala Ile Glu Leu Thr Leu Gln Ser Leu Gly Val Ser
   550                      555                      560                      565
   gag gag ctg caa aac aag cta gaa gat gtt gtg gtt gac aga aat ctt      1782
   Glu Glu Leu Gln Asn Lys Leu Glu Asp Val Val Val Asp Arg Asn Leu
                   570                      575                      580
   cta att ctt ggg aaa gtt ctg ggc gaa gga gag ttt ggg tct gta atg      1830
   Leu Ile Leu Gly Lys Val Leu Gly Glu Gly Glu Phe Gly Ser Val Met
                   585                      590                      595
   gaa gga aat ctg aag cag gaa gat ggg act tct cag aag gtg gca gtg      1878
   Glu Gly Asn Leu Lys Gln Glu Asp Gly Thr Ser Gln Lys Val Ala Val
               600                      605                      610
   aag acc atg aag ttg gac aac ttt tct cta cgg gag atc gag gag ttt      1926
   Lys Thr Met Lys Leu Asp Asn Phe Ser Leu Arg Glu Ile Glu Glu Phe
           615                      620                      625
   ctc agc gaa gca gcg tgc atg aaa gac ttc aat cac cca aac gtc atc      1974
   Leu Ser Glu Ala Ala Cys Met Lys Asp Phe Asn His Pro Asn Val Ile
   630                      635                      640                      645
   cgg ctt cta ggc gtg tgt ata gaa ctg agc tct caa ggc atc ccg aag      2022
   Arg Leu Leu Gly Val Cys Ile Glu Leu Ser Ser Gln Gly Ile Pro Lys
                   650                      655                      660
   ccc atg gtg att tta ccc ttc atg aaa tac gga gac ctc cac acc ttc      2070
   Pro Met Val Ile Leu Pro Phe Met Lys Tyr Gly Asp Leu His Thr Phe
                   665                      670                      675
   ctg cta tat tcc cgg ata gaa tca gta ccg aag tcc atc ccc ctg cag      2118
   Leu Leu Tyr Ser Arg Ile Glu Ser Val Pro Lys Ser Ile Pro Leu Gln
               680                      685                      690
   aca ctg ctg aag ttc atg gtg gac att gcc cag gga atg gag tac ctg      2166
   Thr Leu Leu Lys Phe Met Val Asp Ile Ala Gln Gly Met Glu Tyr Leu
       695                 700                 705
   agc agc agg aat ttt ctc cac agg gat tta gct gct cgg aat tgc atg      2214
   Ser Ser Arg Asn Phe Leu His Arg Asp Leu Ala Ala Arg Asn Cys Met
   710                 715                 720                 725
   ttg cgg gat gac atg act gtc tgc gtg gca gac ttt ggc ctc tct aag      2262
   Leu Arg Asp Asp Met Thr Val Cys Val Ala Asp Phe Gly Leu Ser Lys
                   730                 735                 740
   aag att tac agt ggt gat tat tac cgc caa ggc cgc att gcc aaa atg      2310
   Lys Ile Tyr Ser Gly Asp Tyr Tyr Arg Gln Gly Arg Ile Ala Lys Met
               745                 750                 755
   cct gtg aag tgg atc gcc ata gag agc ctg gcg gac cga gtc tac aca      2358
   Pro Val Lys Trp Ile Ala Ile Glu Ser Leu Ala Asp Arg Val Tyr Thr
           760                 765                 770
   agc aag agt gac gtg tgg gct ttt ggc gtg acc atg tgg gaa ata gca      2406
   Ser Lys Ser Asp Val Trp Ala Phe Gly Val Thr Met Trp Glu Ile Ala
       775                 780                 785
   aca cgg gga atg act ccc tat cct gga gtc cag aac cat gag atg tac      2454
   Thr Arg Gly Met Thr Pro Tyr Pro Gly Val Gln Asn His Glu Met Tyr
   790                 795                 800                 805
   gat tac ctt ctc cac ggc cac agg ctg aag cag ccc gag gac tgc ctg      2502
   Asp Tyr Leu Leu His Gly His Arg Leu Lys Gln Pro Glu Asp Cys Leu
                   810                 815                 820
   gat gat ctg tat gaa atc atg tac tct tgc tgg agt gct gat ccc ctg      2550
   Asp Asp Leu Tyr Glu Ile Met Tyr Ser Cys Trp Ser Ala Asp Pro Leu
               825                 830                 835
   gac cga ccc acc ttc tca gtg ctg agg ctg cag ctg gaa aag ctc tcc      2598
   Asp Arg Pro Thr Phe Ser Val Leu Arg Leu Gln Leu Glu Lys Leu Ser
           840                 845                 850
   gag agt ttg cct gat gcc cag gac aaa gaa tcc atc atc tac atc aat      2646
   Glu Ser Leu Pro Asp Ala Gln Asp Lys Glu Ser Ile Ile Tyr Ile Asn
       855                 860                 865
   aca cag tta cta gag agc tgc gag ggc cta gcc aac agg tcc tcc ctc      2694
   Thr Gln Leu Leu Glu Ser Cys Glu Gly Leu Ala Asn Arg Ser Ser Leu
   870                 875                 880                 885
   gca ggg cta gac atg aac att gat cct gac tcc atc att gcc tct tgc      2742
   Ala Gly Leu Asp Met Asn Ile Asp Pro Asp Ser Ile Ile Ala Ser Cys
                   890                 895                 900
   aca gca ggt gct gct gtc agc gtg gtc atg gcg gaa gtt cac gag aac      2790
   Thr Ala Gly Ala Ala Val Ser Val Val Met Ala Glu Val His Glu Asn
               905                 910                 915
   aac ctt cat gag gaa aga tac atc tta aat ggg ggc aac gag gaa tgg      2838
   Asn Leu His Glu Glu Arg Tyr Ile Leu Asn Gly Gly Asn Glu Glu Trp
           920                 925                 930
   gaa gat gtg gcc tcc act cca ttt gct aca gtc aca gct gga aaa gat      2886
   Glu Asp Val Ala Ser Thr Pro Phe Ala Thr Val Thr Ala Gly Lys Asp
       935                 940                 945
   ggt gtg tta cca gag gac aga ctc acc aaa aat ggc atc tcc tgg tct      2934
   Gly Val Leu Pro Glu Asp Arg Leu Thr Lys Asn Gly Ile Ser Trp Ser
   950                 955                 960                 965
   cac cat agt acg cta ccc ttg ggg agc cca tca cca gat gaa ctt ctg      2982
   His His Ser Thr Leu Pro Leu Gly Ser Pro Ser Pro Asp Glu Leu Leu
                   970                 975                 980
   ttt gca gac gac tcc tcg gga gac tct gaa gtt ctg atg tga              3024
   Phe Ala Asp Asp Ser Ser Gly Asp Ser Glu Val Leu Met
               985                 990
<210> 6
   <211> 994
   <212> PRT
   <213> Rattus norvegicus
<400> 6
   Met Val Leu Ala Pro Leu Leu Leu Gly Leu Leu Leu Leu Ser Ala Leu
   1               5                   10                  15
   Trp Asn Gly Gly Thr Ala Glu Lys Glu Glu Glu Ile Lys Pro Asp Gln
               20                  25                  30
   Pro Phe Ser Gly Pro Leu Pro Gly Ser Leu Pro Ala Asp His Arg Pro
           35                  40                  45
   Phe Phe Ala Pro His Ser Ser Gly Asp Gln Leu Ser Pro Ser Gln Thr
       50                  55                  60
   Gly Arg Ser His Pro Ala His Thr Ala Thr Pro Gln Met Thr Ser Ala
   65                  70                  75                  80
   Ala Ser Asn Leu Leu Pro Pro Val Ala Phe Lys Asn Thr Ile Gly Arg
                   85                  90                  95
   Ile Val Leu Ser Glu His Lys Ser Val Lys Phe Asn Cys Ser Ile Asn
               100                 105                 110
   Ile Pro Asn Val Tyr Gln Glu Thr Ala Gly Ile Ser Trp Trp Lys Asp
           115                 120                 125
   Gly Lys Glu Leu Leu Gly Ala His His Ser Ile Thr Gln Phe Tyr Pro
       130                 135                 140
   Asp Glu Glu Gly Val Ser Ile Ile Ala Leu Phe Ser Ile Thr Ser Val
   145                 150                 155                 160
   Gln Arg Ser Asp Asn Gly Ser Tyr Ile Cys Lys Met Lys Val Asn Asp
                   165                 170                 175
   Arg Glu Val Val Ser Asp Pro Ile Tyr Val Glu Val Gln Gly Leu Pro
               180                 185                 190
   Tyr Phe Thr Lys Gln Pro Glu Ser Val Asn Val Thr Arg Asn Thr Ala
           195                 200                 205
   Phe Asn Leu Thr Cys Gln Ala Val Gly Pro Pro Glu Pro Val Asn Ile
       210                 215                 220
   Phe Trp Val Gln Asn Ser Ser Arg Val Asn Glu Asn Pro Glu Arg Ser
   225                 230                 235                 240
   Pro Ser Val Leu Thr Val Ala Gly Leu Thr Glu Thr Ala Val Phe Ser
                   245                 250                 255
   Cys Glu Ala His Asn Asp Lys Gly Leu Thr Val Ser Lys Gly Val Gln
               260                 265                 270
   Ile Asn Ile Lys Val Ile Pro Ser Pro Pro Thr Glu Val His Ile Leu
           275                 280                 285
   Asn Ser Thr Ala His Ser Ile Leu Val Ser Trp Val Pro Gly Phe Asp
       290                 295                 300
   Gly Tyr Ser Pro Leu Gln Asn Cys Ser Ile Gln Val Lys Glu Ala Asp
   305                 310                 315                 320
   Gln Leu Ser Asn Gly Ser Val Met Val Phe Asn Thr Ser Ala Ser Pro
                   325                 330                 335
   His Leu Tyr Glu Val Gln Gln Leu Gln Ala Leu Ala Asn Tyr Ser Val
               340                 345                 350
   Thr Val Ser Cys Arg Asn Glu Ile Gly Trp Ser Ala Val Ser Pro Trp
           355                 360                 365
   Ile Leu Ala Ser Thr Thr Glu Gly Ala Pro Ala Val Ala Pro Leu Asn
       370                 375                 380
   Ile Thr Val Phe Leu Asn Glu Ser Ser Asn Asn Leu Glu Ile Arg Trp
   385                 390                 395                 400
   Thr Lys Pro Pro Ile Lys Arg Gln Asp Gly Glu Leu Val Gly Tyr Arg
                   405                 410                 415
   Ile Ser His Val Trp Glu Ser Ala Gly Thr Ser Lys Glu Leu Ser Glu
               420                 425                 430
   Glu Val Ser Gln Asn Gly Ser Trp Ala Gln Val Pro Val Gln Met His
           435                 440                 445
   Asn Ala Thr Cys Thr Val Arg Ile Ala Val Ile Thr Lys Gly Gly Ile
       450                 455                 460
   Gly Pro Phe Ser Glu Pro Val Asp Val Ala Ile Pro Glu His Ser Arg
   465                 470                 475                 480
   Val Asp Tyr Ala Pro Ser Ser Thr Pro Ala Pro Gly Asn Thr Glu Ser
                   485                 490                 495
   Met Leu Ile Ile Leu Gly Cys Phe Cys Gly Phe Val Leu Met Gly Leu
               500                 505                 510
   Ile Leu Tyr Leu Ser Leu Ala Ile Lys Arg Arg Val Gln Glu Thr Lys
           515                 520                 525
   Phe Gly Gly Ala Phe Ser Glu Glu Asp Ser Gln Leu Val Val Asn Tyr
       530                 535                 540
   Arg Ala Lys Lys Ser Phe Cys Arg Arg Ala Ile Glu Leu Thr Leu Gln
   545                 550                 555                 560
   Ser Leu Gly Val Ser Glu Glu Leu Gln Asn Lys Leu Glu Asp Val Val
                   565                 570                 575
   Val Asp Arg Asn Leu Leu Ile Leu Gly Lys Val Leu Gly Glu Gly Glu
               580                 585                 590
   Phe Gly Ser Val Met Glu Gly Asn Leu Lys Gln Glu Asp Gly Thr Ser
           595                 600                 605
   Gln Lys Val Ala Val Lys Thr Met Lys Leu Asp Asn Phe Ser Leu Arg
       610                 615                 620
   Glu Ile Glu Glu Phe Leu Ser Glu Ala Ala Cys Met Lys Asp Phe Asn
   625                 630                 635                 640
   His Pro Asn Val Ile Arg Leu Leu Gly Val Cys Ile Glu Leu Ser Ser
                   645                 650                 655
   Gln Gly Ile Pro Lys Pro Met Val Ile Leu Pro Phe Met Lys Tyr Gly
               660                 665                 670
   Asp Leu His Thr Phe Leu Leu Tyr Ser Arg Ile Glu Ser Val Pro Lys
           675                 680                 685
   Ser Ile Pro Leu Gln Thr Leu Leu Lys Phe Met Val Asp Ile Ala Gln
       690                 695                 700
   Gly Met Glu Tyr Leu Ser Ser Arg Asn Phe Leu His Arg Asp Leu Ala
   705                 710                 715                 720
   Ala Arg Asn Cys Met Leu Arg Asp Asp Met Thr Val Cys Val Ala Asp
                   725                 730                 735
   Phe Gly Leu Ser Lys Lys Ile Tyr Ser Gly Asp Tyr Tyr Arg Gln Gly
               740                 745                 750
   Arg Ile Ala Lys Met Pro Val Lys Trp Ile Ala Ile Glu Ser Leu Ala
           755                 760                 765
   Asp Arg Val Tyr Thr Ser Lys Ser Asp Val Trp Ala Phe Gly Val Thr
       770                 775                 780
   Met Trp Glu Ile Ala Thr Arg Gly Met Thr Pro Tyr Pro Gly Val Gln
   785                 790                 795                 800
   Asn His Glu Met Tyr Asp Tyr Leu Leu His Gly His Arg Leu Lys Gln
                   805                 810                 815
   Pro Glu Asp Cys Leu Asp Asp Leu Tyr Glu Ile Met Tyr Ser Cys Trp
               820                 825                 830
   Ser Ala Asp Pro Leu Asp Arg Pro Thr Phe Ser Val Leu Arg Leu Gln
           835                 840                 845
   Leu Glu Lys Leu Ser Glu Ser Leu Pro Asp Ala Gln Asp Lys Glu Ser
       850                 855                 860
   Ile Ile Tyr Ile Asn Thr Gln Leu Leu Glu Ser Cys Glu Gly Leu Ala
   865                 870                 875                 880
   Asn Arg Ser Ser Leu Ala Gly Leu Asp Met Asn Ile Asp Pro Asp Ser
                   885                 890                 895
   Ile Ile Ala Ser Cys Thr Ala Gly Ala Ala Val Ser Val Val Met Ala
               900                 905                 910
   Glu Val His Glu Asn Asn Leu His Glu Glu Arg Tyr Ile Leu Asn Gly
           915                 920                 925
   Gly Asn Glu Glu Trp Glu Asp Val Ala Ser Thr Pro Phe Ala Thr Val
       930                 935                 940
   Thr Ala Gly Lys Asp Gly Val Leu Pro Glu Asp Arg Leu Thr Lys Asn
   945                 950                 955                 960
   Gly Ile Ser Trp Ser His His Ser Thr Leu Pro Leu Gly Ser Pro Ser
                   965                 970                 975
   Pro Asp Glu Leu Leu Phe Ala Asp Asp Ser Ser Gly Asp Ser Glu Val
               980                 985                 990
   Leu Met
<210> 7
   <211> 3037
   <212> DNA
   <213> Gallus gallus
<220>
   <221> CDS
   <222> (26)..(2950)
<400> 7
   gaattccgct cggtgcgggc acggg atg ggc ggc ggg cgc tgc gcg ctg ctc      52
                               Met Gly Gly Gly Arg Cys Ala Leu Leu
                               1               5
   tgc gcg ttg ctc tgc gct ctg ccc ctc ccg cgc tgc ggg gcc gct gag     100
   Cys Ala Leu Leu Cys Ala Leu Pro Leu Pro Arg Cys Gly Ala Ala Glu
   10                  15                  20                  25
   ttt ggt ggt gct ggt gga cat gga ggc ctg agg tcc cta tct gca gga     148
   Phe Gly Gly Ala Gly Gly His Gly Gly Leu Arg Ser Leu Ser Ala Gly
                       30                  35                  40
   gcc ccg ctg gca agg ccc ctg tgg gca aag cac cac cgc ccc aaa cga     196
   Ala Pro Leu Ala Arg Pro Leu Trp Ala Lys His His Arg Pro Lys Arg
                   45                  50                  55
   ggc ctc acc agt ggc cgc tgg cct ccc cag ggc gca aag cct tct gcc     244
   Gly Leu Thr Ser Gly Arg Trp Pro Pro Gln Gly Ala Lys Pro Ser Ala
               60                  65                  70
   acc tcc gtg gga cag ctg aaa ttt aac ccc aca gtg gga cac gtt gtg     292
   Thr Ser Val Gly Gln Leu Lys Phe Asn Pro Thr Val Gly His Val Val
           75                  80                  85
   ata aat gag ctc aaa gat gtc aca ttt aac tgc tcc atc aaa gta cct     340
   Ile Asn Glu Leu Lys Asp Val Thr Phe Asn Cys Ser Ile Lys Val Pro
   90                  95                  100                 105
   cag ctg cta gtc cgg cca gac tcc cct ggc att tcc ctg tgg aag gat     388
   Gln Leu Leu Val Arg Pro Asp Ser Pro Gly Ile Ser Leu Trp Lys Asp
                       110                 115                 120
   ggc agg gag ctg cac acg ctg gac cgc atc gcc acc agc cac ttt gag     436
   Gly Arg Glu Leu His Thr Leu Asp Arg Ile Ala Thr Ser His Phe Glu
                   125                 130                 135
   atc ctt gat gag gag gag gta gcc atg acc tct aca ttc agc atc cgt     484
   Ile Leu Asp Glu Glu Glu Val Ala Met Thr Ser Thr Phe Ser Ile Arg
               140                 145                 150
   gct gct cag cgc tcg gat aac ggc tcc tac gtc tgc aaa ctc aac atc     532
   Ala Ala Gln Arg Ser Asp Asn Gly Ser Tyr Val Cys Lys Leu Asn Ile
           155                 160                 165
   tct ggc att gag att gca tct gat ccc atc ttg gta cag ctg gaa ggg     580
   Ser Gly Ile Glu Ile Ala Ser Asp Pro Ile Leu Val Gln Leu Glu Gly
   170                 175                 180                 185
   ctc cca cac ttc att caa cag cct gag aag ctg aat gtc acc agg aac     628
   Leu Pro His Phe Ile Gln Gln Pro Glu Lys Leu Asn Val Thr Arg Asn
                       190                 195                 200
   agc ccc ttc aac ctc acg tgc caa gct gtg ggc cca cca gag cct gtg     676
   Ser Pro Phe Asn Leu Thr Cys Gln Ala Val Gly Pro Pro Glu Pro Val
                   205                 210                 215
   gaa atc tac tgg ttt cgt aac aat gtc caa ctc aac atg aag ccc tac     724
   Glu Ile Tyr Trp Phe Arg Asn Asn Val Gln Leu Asn Met Lys Pro Tyr
               220                 225                 230
   atc tcc cca tca gtt ctg act gtc cca ggt ctc aat gaa aca gca ctg     772
   Ile Ser Pro Ser Val Leu Thr Val Pro Gly Leu Asn Glu Thr Ala Leu
           235                 240                 245
   ttc agt tgt gag gct cac aac agc aaa ggg ctg act gct tcc aac ccc     820
   Phe Ser Cys Glu Ala His Asn Ser Lys Gly Leu Thr Ala Ser Asn Pro
   250                 255                 260                 265
   ggg cag gtc aac gtg aaa gga ata cca tct gca cca aaa gct gtg cat     868
   Gly Gln Val Asn Val Lys Gly Ile Pro Ser Ala Pro Lys Ala Val His
                       270                 275                 280
   gtc ctg aag aga atg gcc cac agc att gtg atc tcc tgg gtg cca ggc     916
   Val Leu Lys Arg Met Ala His Ser Ile Val Ile Ser Trp Val Pro Gly
                   285                 290                 295
   ttc gat gcg ttc tct gcc ttg aac agc tgc agt gtg cag gtc aag gaa     964
   Phe Asp Ala Phe Ser Ala Leu Asn Ser Cys Ser Val Gln Val Lys Glu
           300                 305                 310
   gcc gtt cca caa agc aat gtc tca ctt ctg ctc ttt aac acg tcg gtg      1012
   Ala Val Pro Gln Ser Asn Val Ser Leu Leu Leu Phe Asn Thr Ser Val
       315                 320                 325
   cct ccc cat gtg tat cgc atc cag cag ctg tgg ccc atg gca gac tat      1060
   Pro Pro His Val Tyr Arg Ile Gln Gln Leu Trp Pro Met Ala Asp Tyr
   330                 335                 340                 345
   aac atc agt gtt tcc tgc aag aat gaa gtc ggt tgg tcg gca ttt agc      1108
   Asn Ile Ser Val Ser Cys Lys Asn Glu Val Gly Trp Ser Ala Phe Ser
                   350                 355                 360
   ccc tgg ata aca gcc agt acc acg gaa gga gct cca act acc cag cca      1156
   Pro Trp Ile Thr Ala Ser Thr Thr Glu Gly Ala Pro Thr Thr Gln Pro
               365                 370                 375
   ctg aat gtc aca gtg tca ctc aac gaa tcc agc tcc ttc ctg gaa atc      1204
   Leu Asn Val Thr Val Ser Leu Asn Glu Ser Ser Ser Phe Leu Glu Ile
           380                 385                 390
   cga tgg gtg aag cca ccc ctt gag agg aca cac ggg gag ctg cag gga      1252
   Arg Trp Val Lys Pro Pro Leu Glu Arg Thr His Gly Glu Leu Gln Gly
       395                 400                 405
   tat cat atc tgg cac acg tgg cag gac tcc aag ggg ctg cag aac atc      1300
   Tyr His Ile Trp His Thr Trp Gln Asp Ser Lys Gly Leu Gln Asn Ile
   410                 415                 420                 425
   tcc ttg gaa gcc cag cct aat gcc aca gtg gcc atc ctg cct gtg gtg      1348
   Ser Leu Glu Ala Gln Pro Asn Ala Thr Val Ala Ile Leu Pro Val Val
                   430                 435                 440
   gcc acc aat gcc acg tgc tca gtg cgt gtg gct gct gtc acc aag gga      1396
   Ala Thr Asn Ala Thr Cys Ser Val Arg Val Ala Ala Val Thr Lys Gly
               445                 450                 455
   ggc gtg ggg ccc ttc agc agc cca gtg gag gtc ttt gtt cct gcc agt      1444
   Gly Val Gly Pro Phe Ser Ser Pro Val Glu Val Phe Val Pro Ala Ser
           460                 465                 470
   ggg cta ata acc tca tct ccc tct tcg aca cca gca tct ggg aac aca      1492
   Gly Leu Ile Thr Ser Ser Pro Ser Ser Thr Pro Ala Ser Gly Asn Thr
       475                 480                 485
   gac tcc ttt ata gta gca ctg ggc ttc gtc tgt ggt acg gtt gct gtt      1540
   Asp Ser Phe Ile Val Ala Leu Gly Phe Val Cys Gly Thr Val Ala Val
   490                 495                 500                 505
   ggg ctg atc ctc tgc ttg tct gtg gtc atc cag aaa aga tgc atg gaa      1588
   Gly Leu Ile Leu Cys Leu Ser Val Val Ile Gln Lys Arg Cys Met Glu
                   510                 515                 520
   aca aag tat ggg aat gcc ttc agc aga aat gat tca gag ctg gtg gta      1636
   Thr Lys Tyr Gly Asn Ala Phe Ser Arg Asn Asp Ser Glu Leu Val Val
               525                 530                 535
   aac tac aca gcc aag aag tcc tac tgc cgg aga gcc gtc gaa ctg aca      1684
   Asn Tyr Thr Ala Lys Lys Ser Tyr Cys Arg Arg Ala Val Glu Leu Thr
           540                 545                 550
   ttg ggt agc ctg gga gtc agc agc gag ctc cag cag aag ctg cag gac      1732
   Leu Gly Ser Leu Gly Val Ser Ser Glu Leu Gln Gln Lys Leu Gln Asp
       555                 560                 565
   gtt gtc att gac aga aat gcc ctc agc ctg ggg aag gtc ctg gga gag      1780
   Val Val Ile Asp Arg Asn Ala Leu Ser Leu Gly Lys Val Leu Gly Glu
   570                 575                 580                 585
   ggg gag ttc ggg tca gtg atg gag gga cgt ctc agc cag cca gaa ggc      1828
   Gly Glu Phe Gly Ser Val Met Glu Gly Arg Leu Ser Gln Pro Glu Gly
                   590                 595                 600
   acc cca cag aag gtg gct gtc aag acc atg aag ttg gat aac ttt tcc      1876
   Thr Pro Gln Lys Val Ala Val Lys Thr Met Lys Leu Asp Asn Phe Ser
               605                 610                 615
   cat aga gag ata gaa gaa ttc ctc agt gaa gca gca tgc atg aag gac      1924
   His Arg Glu Ile Glu Glu Phe Leu Ser Glu Ala Ala Cys Met Lys Asp
           620                 625                 630
   ttt gac cac ccc aat gtc atc aag ctc cta ggt gtg tgc atc gag ctg      1972
   Phe Asp His Pro Asn Val Ile Lys Leu Leu Gly Val Cys Ile Glu Leu
       635                 640                 645
   agc tct cag cag atc ccc aag ccc atg gtg gtt ctc cca ttc atg aaa      2020
   Ser Ser Gln Gln Ile Pro Lys Pro Met Val Val Leu Pro Phe Met Lys
   650                 655                 660                 665
   tat ggt gac ctg cac agc ttc ctg ctt cgc tcc cgg ctg gag atg gcc      2068
   Tyr Gly Asp Leu His Ser Phe Leu Leu Arg Ser Arg Leu Glu Met Ala
                   670                 675                 680
   ccc cag ttc gtg ccc ctg cag atg ctg ctg aag ttc atg gtg gat att      2116
   Pro Gln Phe Val Pro Leu Gln Met Leu Leu Lys Phe Met Val Asp Ile
               685                 690                 695
   gcc ctg gga atg gag tac ctg agc agt cgg cag ttt ctt cac agg gat      2164
   Ala Leu Gly Met Glu Tyr Leu Ser Ser Arg Gln Phe Leu His Arg Asp
           700                 705                 710
   ttg gcg gct cgg aac tgc atg tta cgg gat gac atg acg gtg tgt gtg      2212
   Leu Ala Ala Arg Asn Cys Met Leu Arg Asp Asp Met Thr Val Cys Val
       715                 720                 725
   gca gac ttt ggg ctg tcc aag aag atc tac agc ggc gat tac tac cgt      2260
   Ala Asp Phe Gly Leu Ser Lys Lys Ile Tyr Ser Gly Asp Tyr Tyr Arg
   730                 735                 740                 745
   cag ggc cga ata gca aaa atg cca gtg aag tgg att gcg ata gag tcc      2308
   Gln Gly Arg Ile Ala Lys Met Pro Val Lys Trp Ile Ala Ile Glu Ser
                   750                 755                 760
   ctg gct gac cgt gtc tac acc acc aag agt gat gtg tgg gca ttt ggc      2356
   Leu Ala Asp Arg Val Tyr Thr Thr Lys Ser Asp Val Trp Ala Phe Gly
               765                 770                 775
   gtt acc atg tgg gag ata gcg acc aga ggg atg act ccg tac cca ggg      2404
   Val Thr Met Trp Glu Ile Ala Thr Arg Gly Met Thr Pro Tyr Pro Gly
           780                 785                 790
   gtg cag aac cac gag att tat gag tat cta ttc cac ggg cag cgg ctc      2452
   Val Gln Asn His Glu Ile Tyr Glu Tyr Leu Phe His Gly Gln Arg Leu
       795                 800                 805
   aaa aag cct gag aac tgc tta gat gaa ctg tac gat atc atg tcc tcc      2500
   Lys Lys Pro Glu Asn Cys Leu Asp Glu Leu Tyr Asp Ile Met Ser Ser
   810                 815                 820                 825
   tgc tgg agg gct gaa cct gct gac cga ccg acg ttc tca cag ctg aaa      2548
   Cys Trp Arg Ala Glu Pro Ala Asp Arg Pro Thr Phe Ser Gln Leu Lys
                   830                 835                 840
   gtt cat ctg gag aag ctt ttg gaa agc ctt cct gcc ccg agg ggg tcc      2596
   Val His Leu Glu Lys Leu Leu Glu Ser Leu Pro Ala Pro Arg Gly Ser
               845                 850                 855
   aag gac gtc atc tac gtc aac acc agc ctg cca gag gag agc ccc gac      2644
   Lys Asp Val Ile Tyr Val Asn Thr Ser Leu Pro Glu Glu Ser Pro Asp
           860                 865                 870
   tcc acc cag gat ttg ggc ttg gat tcg gtc atc ccc caa gcg gac tct      2692
   Ser Thr Gln Asp Leu Gly Leu Asp Ser Val Ile Pro Gln Ala Asp Ser
       875                 880                 885
   gac ttg gac ccc ggg gac att gct gag ccc tgc tgc tcc cac acg aag      2740
   Asp Leu Asp Pro Gly Asp Ile Ala Glu Pro Cys Cys Ser His Thr Lys
   890                 895                 900                 905
   gca gcg ctg gtg gca gtg gat atc cac gat ggg ggc tcg agg tac gtc      2788
   Ala Ala Leu Val Ala Val Asp Ile His Asp Gly Gly Ser Arg Tyr Val
                   910                 915                 920
   ctt gaa agt gag ggc agc ccc aca gag gat gct tac gtc cca ctg ctg      2836
   Leu Glu Ser Glu Gly Ser Pro Thr Glu Asp Ala Tyr Val Pro Leu Leu
               925                 930                 935
   ccc cac gag ggc tcg gcg tgg acc gag gcc agc acc ttg cct gtt ggc      2884
   Pro His Glu Gly Ser Ala Trp Thr Glu Ala Ser Thr Leu Pro Val Gly
           940                 945                 950
   agc tcg ctt gca gct cag ctg cca tgt gct gat ggc tgc ctg gag gac      2932
   Ser Ser Leu Ala Ala Gln Leu Pro Cys Ala Asp Gly Cys Leu Glu Asp
       955                 960                 965
   tcc gaa gcg ctg ctg tga atggagcctg cgcaaagcag ggtgtgaatc             2980
   Ser Glu Ala Leu Leu
   970
   aggacagaga gattttattt taataaacag tcttatttct tcttgcatta ttttcat 3037
<210> 8
   <211> 974
   <212> PRT
   <213> Gallus gallus
<400> 8
   Met Gly Gly Gly Arg Cys Ala Leu Leu Cys Ala Leu Leu Cys Ala Leu
   1 5 10 15
   Pro Leu Pro Arg Cys Gly Ala Ala Glu Phe Gly Gly Ala Gly Gly His
   20 25 30
   Gly Gly Leu Arg Ser Leu Ser Ala Gly Ala Pro Leu Ala Arg Pro Leu
   35 40 45
   Trp Ala Lys His His Arg Pro Lys Arg Gly Leu Thr Ser Gly Arg Trp
   50 55 60
   Pro Pro Gln Gly Ala Lys Pro Ser Ala Thr Ser Val Gly Gln Leu Lys
   65 70 75 80
   Phe Asn Pro Thr Val Gly His Val Val Ile Asn Glu Leu Lys Asp Val
   85 90 95
   Thr Phe Asn Cys Ser Ile Lys Val Pro Gln Leu Leu Val Arg Pro Asp
   100 105 110
   Ser Pro Gly Ile Ser Leu Trp Lys Asp Gly Arg Glu Leu His Thr Leu
   115 120 125
   Asp Arg Ile Ala Thr Ser His Phe Glu Ile Leu Asp Glu Glu Glu Val
   130 135 140
   Ala Met Thr Ser Thr Phe Ser Ile Arg Ala Ala Gln Arg Ser Asp Asn
   145 150 155 160
   Gly Ser Tyr Val Cys Lys Leu Asn Ile Ser Gly Ile Glu Ile Ala Ser
   165 170 175
   Asp Pro Ile Leu Val Gln Leu Glu Gly Leu Pro His Phe Ile Gln Gln
   180 185 190
   Pro Glu Lys Leu Asn Val Thr Arg Asn Ser Pro Phe Asn Leu Thr Cys
   195 200 205
   Gln Ala Val Gly Pro Pro Glu Pro Val Glu Ile Tyr Trp Phe Arg Asn
   210 215 220
   Asn Val Gln Leu Asn Met Lys Pro Tyr Ile Ser Pro Ser Val Leu Thr
   225 230 235 240
   Val Pro Gly Leu Asn Glu Thr Ala Leu Phe Ser Cys Glu Ala His Asn
   245 250 255
   Ser Lys Gly Leu Thr Ala Ser Asn Pro Gly Gln Val Asn Val Lys Gly
   260 265 270
   Ile Pro Ser Ala Pro Lys Ala Val His Val Leu Lys Arg Met Ala His
   275 280 285
   Ser Ile Val Ile Ser Trp Val Pro Gly Phe Asp Ala Phe Ser Ala Leu
   290 295 300
   Asn Ser Cys Ser Val Gln Val Lys Glu Ala Val Pro Gln Ser Asn Val
   305 310 315 320
   Ser Leu Leu Leu Phe Asn Thr Ser Val Pro Pro His Val Tyr Arg Ile
   325 330 335
   Gln Gln Leu Trp Pro Met Ala Asp Tyr Asn Ile Ser Val Ser Cys Lys
   340 345 350
   Asn Glu Val Gly Trp Ser Ala Phe Ser Pro Trp Ile Thr Ala Ser Thr
   355 360 365
   Thr Glu Gly Ala Pro Thr Thr Gln Pro Leu Asn Val Thr Val Ser Leu
   370 375 380
   Asn Glu Ser Ser Ser Phe Leu Glu Ile Arg Trp Val Lys Pro Pro Leu
   385 390 395 400
   Glu Arg Thr His Gly Glu Leu Gln Gly Tyr His Ile Trp His Thr Trp
   405 410 415
   Gln Asp Ser Lys Gly Leu Gln Asn Ile Ser Leu Glu Ala Gln Pro Asn
   420 425 430
   Ala Thr Val Ala Ile Leu Pro Val Val Ala Thr Asn Ala Thr Cys Ser
           435                     440                     445
   Val Arg Val Ala Ala Val Thr Lys Gly Gly Val Gly Pro Phe Ser Ser
       450                     455                     460
   Pro Val Glu Val Phe Val Pro Ala Ser Gly Leu Ile Thr Ser Ser Pro
   465                     470                     475                     480
   Ser Ser Thr Pro Ala Ser Gly Asn Thr Asp Ser Phe Ile Val Ala Leu
                   485                     490                     495
   Gly Phe Val Cys Gly Thr Val Ala Val Gly Leu Ile Leu Cys Leu Ser
               500                     505                     510
   Val Val Ile Gln Lys Arg Cys Met Glu Thr Lys Tyr Gly Asn Ala Phe
           515                     520                     525
   Ser Arg Asn Asp Ser Glu Leu Val Val Asn Tyr Thr Ala Lys Lys Ser
       530                     535                     540
   Tyr Cys Arg Arg Ala Val Glu Leu Thr Leu Gly Ser Leu Gly Val Ser
   545                     550                     555                     560
   Ser Glu Leu Gln Gln Lys Leu Gln Asp Val Val Ile Asp Arg Asn Ala
                   565                     570                     575
   Leu Ser Leu Gly Lys Val Leu Gly Glu Gly Glu Phe Gly Ser Val Met
               580                     585                     590
   Glu Gly Arg Leu Ser Gln Pro Glu Gly Thr Pro Gln Lys Val Ala Val
           595                     600                     605
   Lys Thr Met Lys Leu Asp Asn Phe Ser His Arg Glu Ile Glu Glu Phe
       610                     615                     620
   Leu Ser Glu Ala Ala Cys Met Lys Asp Phe Asp His Pro Asn Val Ile
   625                     630                     635                     640
   Lys Leu Leu Gly Val Cys Ile Glu Leu Ser Ser Gln Gln Ile Pro Lys
                   645                     650                     655
   Pro Met Val Val Leu Pro Phe Met Lys Tyr Gly Asp Leu His Ser Phe
               660                     665                     670
   Leu Leu Arg Ser Arg Leu Glu Met Ala Pro Gln Phe Val Pro Leu Gln
           675                     680                     685
   Met Leu Leu Lys Phe Met Val Asp Ile Ala Leu Gly Met Glu Tyr Leu
       690                     695                     700
   Ser Ser Arg Gln Phe Leu His Arg Asp Leu Ala Ala Arg Asn Cys Met
   705                     710                     715                     720
   Leu Arg Asp Asp Met Thr Val Cys Val Ala Asp Phe Gly Leu Ser Lys
                   725                     730                     735
   Lys Ile Tyr Ser Gly Asp Tyr Tyr Arg Gln Gly Arg Ile Ala Lys Met
               740                     745                     750
   Pro Val Lys Trp Ile Ala Ile Glu Ser Leu Ala Asp Arg Val Tyr Thr
           755                     760                     765
   Thr Lys Ser Asp Val Trp Ala Phe Gly Val Thr Met Trp Glu Ile Ala
       770                     775                     780
   Thr Arg Gly Met Thr Pro Tyr Pro Gly Val Gln Asn His Glu Ile Tyr
   785                     790                     795                     800
   Glu Tyr Leu Phe His Gly Gln Arg Leu Lys Lys Pro Glu Asn Cys Leu
                   805                     810                     815
   Asp Glu Leu Tyr Asp Ile Met Ser Ser Cys Trp Arg Ala Glu Pro Ala
               820                     825                     830
   Asp Arg Pro Thr Phe Ser Gln Leu Lys Val His Leu Glu Lys Leu Leu
           835                     840                     845
   Glu Ser Leu Pro Ala Pro Arg Gly Ser Lys Asp Val Ile Tyr Val Asn
       850                     855                     860
   Thr Ser Leu Pro Glu Glu Ser Pro Asp Ser Thr Gln Asp Leu Gly Leu
   865                     870                     875                     880
   Asp Ser Val Ile Pro Gln Ala Asp Ser Asp Leu Asp Pro Gly Asp Ile
                   885                     890                     895
   Ala Glu Pro Cys Cys Ser His Thr Lys Ala Ala Leu Val Ala Val Asp
               900                     905                     910
   Ile His Asp Gly Gly Ser Arg Tyr Val Leu Glu Ser Glu Gly Ser Pro
           915                     920                     925
   Thr Glu Asp Ala Tyr Val Pro Leu Leu Pro His Glu Gly Ser Ala Trp
   930                 935                 940
   Thr Glu Ala Ser Thr Leu Pro Val Gly Ser Ser Leu Ala Ala Gln Leu
   945                 950                 955                 960
   Pro Cys Ala Asp Gly Cys Leu Glu Asp Ser Glu Ala Leu Leu
                   965                 970

## Description

### Field Of The Invention

The present invention relates to the diagnosis of disorders associated with the Mer transmembrane receptor tyrosine kinase.

### Background Of The Invention

Tyrosine kinases play an important role in normal cellular growth and differentiation. Deregulation of tyrosine kinase activity can result in cellular transformation leading to the development of human cancer. Mer is a transmembrane receptor tyrosine kinase that is likely the human homologue of the chicken retroviral gene, v-eyk, which causes many types of cancer in chicken. The human Mer gene and the mouse Mer gene and cDNA have been sequenced and characterized, and the expression of Mer has been profiled in cell lines and tissues (Graham et al., Cell Growth and Differentiation, 1994, 5:647-657; Graham et al., Oncogene, 1995, 10:2349-2359; and U.S. Patent No. 5,585,269). The Mer receptor tyrosine kinase, initially cloned from a human B lymphoblastoid cell line, is expressed in a spectrum of-hematopoietic, epithelial, and mesenchymal cell lines. Interestingly, while the RNA transcript of Mer is detected in numerous T and B lymphoblastic cell lines, Mer RNA is not found in normal human thymocytes, lymphocytes or in PMA/PHA stimulated lymphocytes. Mer is composed of two immunoglobulin domains and two fibronectin III domains in the extracellular portion, and a tyrosine kinase domain in the intracellular portion (Graham *et al*., (1994), *supra* and Graham *et al*., (1995), *supra*). Human Mer is known to be transforming and anti-apoptotic, and Mer overexpression has been linked to a number of different human cancers including subsets of B and T cell leukemia, lymphoma, pituitary adenoma, gastric cancer, and rhabdomyosarcoma.

Mer is related to two other receptor tyrosine kinases, Axl and Tyro-3. Mer, Axl, and Tyro-3 are all expressed in a spectrum of hematopoeitic, epithelial, and mesenchymal cell lines. Each protein has been shown to have the capability to transform cells *in vitro.* The overexpression of Mer leads to the transformation of NIH 3T3 cells and Ba/F3 pro lymphocytes (Ling et al., Mol. Cell Bio. 15:6582-6592 (1995) and Georgescu et al., Mol. Cell Bio. 19:1171-1181 (1999)). Axl, originally identified as a protein encoded by a transforming gene from primary human myeloid leukemia cells, is overexpressed in a number of different tumor cell types and transforms NIH3T3 fibroblasts (O'Bryan et al., Mol. Cell Bio. 11:5016-5031 (1991)). Tyro-3 is expressed at elevated levels in mammary tumors (Taylor et al., J. Biol. Chem., 270:6872-6880 (1995)) and its overexpression causes transformed growth of fibroblasts (Lai et al., Oncogene 9:2567-2578 (1995)).

Within hematopoietic cell lines, the Mer receptor tyrosine kinase is normally expressed in monocytes/macrophages, dendritic cells, megakaryocytes, and platelets. Mer RNA transcript or protein is not detected in lymphocytes or thymocytes. However, in acute lymphoblastic leukemia cell lines and patient samples, Mer RNA transcript and protein is present (Graham *et al*., (1994), *supra*; Graham *et al*., (1995), *supra*; and U.S. Patent 5,585,269).

Mer, Axl, and Tyro-3 are all activated by the ligand Gas6. Gas6 is structurally similar to Protein S, a cofactor for anticoagulant Protein C, and shares 48% protein identity with Protein S. Gas6 plays a role in coagulation (Angelillo-Scherrer et al., Nature Medicine 7:215-21 (2002)). Gas6 antibodies may be used to protect wild type mice against fatal thromboembolism (Angelillo-Scherrer *et al*., (2002)). Mice with an inactivated Gas6 and/or Mer gene have platelet dysfunction that prevents venous and arterial thrombosis. These knockout mice are protected against fatal collagen/epinephrine induced thromboembolism and inhibited ferric chloride-induced thrombosis *in vivo.* Gas6 amplifies platelet aggregation and secretion response of platelets to known agonists (Chen et al., Aterioscler. Thromb. Vasc. Biol. 24:1118-1123 (2004)). The platelet dysfunction caused by Gas6 is thought to be mediated through the Mer, Axl, or Tyro-3. Thus, the Mer receptor tyrosine kinase may play a significant role in the development of hemostasis and human cancer.

Various types of thrombosis and the complications associated with thrombosis represent a major cause of morbidity and death in the world. Malignant cellular growth or tumors (cancer) are also a leading cause of death worldwide. The development of effective therapy for cardiovascular and neoplastic disease is the subject of a large body of research. Although a variety of innovative approaches to treat and prevent such diseases have been proposed, these diseases continue to have a high rate of mortality and may be difficult to treat or relatively unresponsive to conventional therapies. Therefore, there is a continued need in the art for new therapies that can effectively target and prevent or treat these diseases.

### Summary Of The Invention

The present invention relates to a method of diagnosing leukemia or lymphoma. The method includes detecting aberrant glycoforms of Mer transmembrane receptor tyrosine kinase (Mer) in lymphocytes from an individual, wherein the expression of aberrant glycoforms of the Mer transmembrane receptor tyrosine kinase by lymphocytes in the individual is indicative of leukemia or lymphoma. The individual is preferably a mammal, and more preferably, a human.

In one aspect, the method comprises detecting an aberrant glycoform of Mer selected from: a Mer glycoform having a molecular weight between 170 kD and 190 kD, a Mer glycoform having a molecular weight between 135 kD and 144 kD, and a Mer glycoform having a molecular weight between 190 kD and 195kD. In one aspect, the leukemia is a lymphoblastic leukemia, and wherein the method includes the detection of at least one Mer glycoform having a molecular weight of between 170 kD and 190 kD, or having a molecular weight between 135 kD and 140 kD. In another aspect, a Mer glycoform having a molecular weight of between 170 kD and

190 kD is detected, and the method further comprises detecting the amount of the Mer glycoform expressed by the cells, wherein expression of Mer and lack of CD3 expression by the cells, indicates a poor prognosis for the individual. In one aspect, the leukemia is a myelogenous leukemia or lymphoma, and the method includes the detection of a Mer glycoform having a molecular weight of between 190 kD and 195 kD.

In this method of the invention, the step of detection can include contacting the sample with an antibody or antigen binding fragment thereof that selectively binds to said Mer transmembrane receptor tyrosine kinase glycoform.

We describe a method for the production of a monoclonal antibody that selectively binds to a specific glycoform of the Mer transmembrane receptor tyrosine kinase comprising: (a) immunizing a non-human mammal with a glycosylated Mer transmembrane receptor tyrosine kinase such that antibody-producing cells are produced, wherein the glycosylated Mer transmembrane receptor tyrosine kinase is at least an extracellular portion of a full-length Mer transmembrane receptor tyrosine kinase, wherein the full-length Mer transmembrane receptor tyrosine kinase has a molecular weight of less than about 195 kD; (b) removing and immortalizing said antibody producing cells; (c) selecting and cloning the immortalized antibody producing cells producing the desired antibody; and (d) isolating the antibodies produced by the selected, cloned immortalized antibody producing cells. In one aspect, the Mer transmembrane receptor tyrosine kinase is selected from the group consisting of: a Mer glycoform having a molecular weight between about 165 kD and about 170 kD, a Mer glycoform having a molecular weight between about 170 kD and about 190 kD, a Mer glycoform having a molecular weight between about 135 kD and about 140 kD, and a Mer glycoform having a molecular weight between about 190 kD and about 195kD. In another aspect, the Mer transmembrane receptor tyrosine kinase is expressed by a cell type selected from the group consisting of: platelets, lymphoblastic leukemia cells, and myelogenous leukemia cells. Included in this embodiment is any antibody produced by the method, including a monoclonal antibody.

We describe an isolated antibody that selectively binds to a glycosylated Mer transmembrane receptor tyrosine kinase protein, wherein the antibody detects any glycosylation form of the Mer protein.

We describe an isolated antibody that selectively binds to a Mer transmembrane receptor tyrosine kinase glycoform (Mer glycoform) selected from: a Mer glycoform having a molecular weight between about 195 kD and about 210 kD, a Mer glycoform having a molecular weight between about 165 kD and about 170 kD, a Mer glycoform having a molecular weight between about 170 kD and about 190 kD, a Mer glycoform having a molecular weight between about 135 kD and about 140 kD, and a Mer glycoform having a molecular weight between about 190 kD and about 195kD.

Any of the above-identified antibodies selectively bind to all of the Mer glycoforms selected from the group consisting of: a Mer glycoform having a molecular weight between about 195 kD and about 210 kD, a Mer glycoform having a molecular weight between about 165 kD and about 170 kD, a Mer glycoform having a molecular weight between about 170 kD and about 190 kD, a Mer glycoform having a molecular weight between about 135 kD and about 140 kD, and a Mer glycoform having a molecular weight between about 190 kD and about 195kD. The antibody may selectively bind to one of said Mer glycoforms and not to the other Mer glycoforms.

We describe an isolated antibody that selectively binds to a Mer transmembrane receptor tyrosine kinase glycoform (Mer glycoform), wherein the Mer glycoform has a molecular weight of less than about 195 kD. In one aspect, the Mer glycoform has a molecular weight of between about 170 kD and about 190 kD or less than about 160 kD. The Mer glycoform may have a molecular weight of between about 170 kD and about 190 kD or between about 135 kD and about 140 kD. The Mer glycoform may have a molecular weight of between about 190 kD and about 195kD. The antibody may selectively bind to a Mer glycoform expressed by leukemia or lymphoma cells. The antibody may selectively bind to a Mer glycoform expressed by lymphoblastic leukemia cells. The antibody may selectively bind to a Mer glycoform expressed by myelogenous leukemia cells.

We describe a composition comprising any of the above-identified antibodies. The composition may further comprise a pharmaceutically acceptable carrier.

The invention includes the use of any of the above-identified antibodies in a composition for diagnosing leukemia or lymphoma.

Yet another embodiment of the invention includes the use of any of the above-identified antibodies in a pharmaceutical formulation for treating a cancer in an individual.

We describe an antibody that selectively binds to the Mer glycoform and inhibits the activity of the Mer glycoform for treating cancer in an individual positive for surface expression of a Mer glycoform, comprising administering the antibody to the individual an antibody that selectively binds to the Mer glycoform and inhibits the activity of the Mer glycoform. The cancer may be a leukemia or lymphoma. The Mer glycoform may be selected from the group consisting of: a Mer glycoform having a molecular weight between about 170 kD and about 190 kD, a Mer glycoform having a molecular weight between about 135 kD and about 140 kD, and a Mer glycoform having a molecular weight between about 190 kD and about 195kD. The antibody may be any of the above-described antibodies.

We describe an antibody that selectively binds to and inhibits the activity of the Mer glycoform having a molecular weight of between about 165 kD and about 170 kD for treating or preventing a clotting disorder in an individual, comprising administrating to the individual a therapeutically effective amount of the antibody.

We describe a method of treating or preventing a clotting disorder in an individual, comprising administrating to the individual an effective amount of a soluble glycoform of a Mer transmembrane receptor tyrosine kinase, wherein the soluble Mer glycoform is glyosylated in a manner similar to the glycoform of Mer present on platelets. The soluble Mer glycoform may have a molecular weight of between about 165 kD and about 170 kD.

In the above-identified embodiment, the clotting disorder can include, but is not limited to, thrombophilia.

We describe an isolated soluble Mer transmembrane receptor tyrosine kinase (Mer) protein, wherein the soluble Mer protein is glycosylated, wherein the soluble Mer protein binds to a Mer ligand, and wherein the soluble Mer protein is a soluble portion of a full-length Mer glycoform or a homologue thereof having a molecular weight of less than about 195 kD. In one description, the full-length Mer glycoform is a Mer glycoform expressed by platelets. In another description, the full-length Mer glycoform is a Mer glycoform having a molecular weight of from about 165 kD to about 170 kD. In another description, the full-length Mer glycoform has a molecular weight of between about 170 kD and about 190 kD or less than about 160 kD. In yet another description, the full-length Mer glycoform is a Mer glycoform expressed by a leukemia or lymphoma cell. In yet another description, the full-length Mer glycoform is a Mer glycoform expressed by a lymphoblastic leukemia cell. In this description, the full-length Mer glycoform can have a molecular weight of between about 170 kD and 190 kD or between about 135 kD and about 140 kD. In another description, the full-length Mer glycoform is a Mer glycoform expressed by a myelogenous leukemia cell. In this description, the full-length Mer glycoform can have a molecular weight of between about 190 kD and about 195 kD. In yet another description, the full-length Mer glycoform comprises an amino acid sequence that is at least about 90% identical to SEQ ID NO:2. In another description, the soluble portion is an extracellular portion of the full-length Mer glycoform that binds to the Mer ligand. The Mer ligands include, but are not limited to, Gas6 and Protein S. In yet another description, the soluble Mer transmembrane receptor tyrosine kinase binds to Protein S and not to Gas6. In another description, the soluble Mer transmembrane receptor tyrosine kinase binds to Protein S with a higher affinity than to Gas6.

We describe a fusion protein, comprising any of the above-described isolated soluble Mer transmembrane receptor tyrosine kinases, linked to a heterologous protein. In one description, the heterologous protein is an Fc fragment of an immunoglobulin protein.

We describe the use of any of the above-identified isolated soluble Mer transmembrane receptor tyrosine kinases or fusion proteins in a pharmaceutical formulation.

We describe the use of certain of the above-identified isolated soluble Mer transmembrane receptor tyrosine kinases or fusion proteins in a pharmaceutical formulation for treating a cancer.

We describe the use of certain of the above-identified isolated soluble Mer transmembrane receptor tyrosine kinases or fusion proteins in a pharmaceutical formulation for treating a clotting disorder.

We describe a method of treating cancer in an individual positive for surface expression of a Mer glycoform in cancer cells, comprising administrating to the individual a soluble form of the Mer glycoform or a fusion protein comprising the soluble form of the Mer glycoform, including the above identified soluble Mer transmembrane receptor tyrosine kinases.

We describe a method of treating or preventing a clotting disorder in an individual comprising administering to the individual a soluble form of a Mer glycoform having a molecular weight of between about 165 kD and about 170 kD, or a fusion protein comprising the soluble form of the Mer glycoform.

We describe a method of treating or preventing a clotting disorder in an individual comprising administering to the individual an agent which modulates the cleavage of the extracellular domain of the Mer transmembrane receptor tyrosine kinase. In one aspect, the agent inhibits cleavage of the extracellular domain of the Mer transmembrane receptor tyrosine kinase, and can include, but is not limited to, a TACE inhibitor. In another aspect, the agent cleaves the extracellular domain of the Mer transmembrane receptor tyrosine kinase, and can include, but is not limited to, a TACE-like metalloprotease.

In the above-described methods related to clotting disorders, the disorder may be thrombophilia.

We describe a method of screening for compounds that regulate blood clotting comprising: (a) contacting a putative regulatory compound with cells expressing a Mer transmembrane receptor tyrosine kinase; and (b) detecting compounds that cleave an extracellular fragment of the Mer transmembrane receptor kinase into the medium as compared to cells not in contact with said compound. In one aspect, the cells are platelets. The compound may be a cleavage agent.

We describe a method for screening for compounds that modulate the activity of a specific glycoform of Mer transmembrane receptor tyrosine kinase comprising: (a) contacting a putative regulatory compound with a Mer transmembrane receptor tyrosine kinase, wherein the Mer transmembrane receptor tyrosine kinase is a glycoform of Mer selected from the group consisting of: a Mer glycoform having a molecular weight between about 195 kD and about 210 kD and a Mer glycoform having a molecular weight of less than about 195 kD; and (b) detecting compounds that selectively bind to the Mer glycoform. In one description, the Mer glycoform is selected from: a Mer glycoform having a molecular weight between about 195 kD and about 210 kD, a Mer glycoform having a molecular weight between about 165 kD and about 170 kD, a Mer glycoform having a molecular weight between about 170 kD and about 190 kD, a Mer glycoform having a molecular weight between about 135 kD and about 140 kD, and a Mer glycoform having a molecular weight between about 190 kD and about 195kD. In one description, the Mer glycoform has a molecular weight of between about 170 kD and about 195 kD or less than about 160 kD. In another description, the Mer glycoform is a Mer glycoform expressed by lymphoblastic leukemia cells. In this description, the Mer glycoform can have a molecular weight of between about 170 kD and about 190 kD, or a molecular weight of between about 135 kD and about 140 kD. In another description, the Mer glycoform is a Mer glycoform expressed by myelogenous leukemia cells. In this description, the Mer glycoform can have a molecular weight of between about 190 kD and about 195 kD.

In any of the above screening methods, the step of detecting may comprise a step of detecting compounds that bind to one Mer glycoform and not to another Mer glycoform. The Mer glycoform may be expressed by a cell. In yet another description, the Mer glycoform is a soluble Mer glycoform. In yet another description, the method further comprises detecting whether the compound inhibits the binding of a Mer ligand to Mer expressed by a cell. For example, a Mer ligand can include, but is not limited to, Gas6 and Protein S. In another description, the method further includes detecting compounds that inhibit the binding of Protein S to Mer expressed by a cell and do not inhibit the binding of Gas6 to Mer expressed by a cell. In one description, the method further comprises detecting whether the compound inhibits the activity of Mer expressed by a cell. In another aspect, the compound is selected from the group consisting of: a small molecule, a nucleic acid, a protein, a peptide and an antibody. In another description, the compound is a soluble Mer protein. In yet another description, such a soluble Mer protein is a different glycoform than the Mer transmembrane receptor tyrosine kinase of step (a).

### Brief Description Of The Drawings

Fig. 1 is a Western Blot illustrating that the monoclonal antibody 311 directed against human Mer recognizes a 205 kD protein in the monocytic cell line U937 and a 185 kD protein in the Jurkat leukemia cell line.
Fig. 2 is a Western blot showing that the human Mer 185 kD protein in leukemia cell lines is distinct from Mer in other hematopoietic lineages.
Fig. 3 is a Western blot confirming through deglycosylation that the Jurkat leukemia cell line expresses unique Mer proteins.
Fig. 4 is a Western blot illustrating detection of two different glycosylation forms of Mer, 185kD and 140kD, in leukemia cell lines and T cell acute lymphoblastic leukemia (ALL) patient samples.
Figs. 5A-5C are Western blots showing that mutations/truncations of Mer protein exist in some leukemia patient samples.
Fig. 6 is a Western blot illustrating that both Gas6 and Protein S are ligands for Mer.
Figs. 7A-7D are a Western blots showing that soluble Mer is shed into the medium of cultured cells.
Figs. 8A-8D are flow cytometry (Figs. 8A-8B) and Western blots (Figs. 8C-8D) demonstrating that LPS and PMA induce cleavage (shedding) of the Mer extracellular domain.
Fig. 9 is a Western blot showing that soluble Mer is shed into the medium by cultured mouse macrophage and spleen cells and is present in mouse blood.
Fig. 10 is a Western blot illustrating that soluble Mer is present in human blood.
Fig. 11 is a Western blot showing that a specific metalloprotease inhibitor (TAPI) blocks production of soluble Mer.
Figs. 12A and 12B are a schematic drawing (Fig. 12A) and a Western blot (Fig. 12B) indicating that soluble Mer (Mer/Fc) binds to Gas6.
Figs. 13A and 13B are Western blots illustrating that soluble Mer (Mer/Fc) inhibits Gas6 signaling.
Fig. 14 is a Western blot demonstrating that soluble Mer (Mer/Fc) is glycosylated differently in mammalian and insect cells.
Fig. 15 is a graph illustrating that Mer expression is associated with the lack of surface CD3 on lymphoblasts.
Figs. 16A-16D are platelet aggregometer traces showing that sMer (Mer/Fc) inhibits platelet aggregation.
Fig. 17 is a graph illustrating that sMer (Mer/Fc) protects against fatal thromboembolism.
Fig. 18 is a Western blot demonstrating that Mer activation leads to the activation of downstream pro-survival pathways AKT and ERK 1/2.
Figs. 19A-19F are digital images of Mer transgenic mice and histological tissues demonstrating the presence of lymphoblastic leukemia/lymphoma and flow cytometry confirming that the Mer positive tumors are T cell in origin.

### Detailed Description Of The Invention

The present invention generally relates to the present inventors' discovery that unique glycoforms of Mer, also known as c-Mer, Mertk, or Mer receptor tyrosine kinase, are expressed by different cell types. In particular, the present inventors have discovered that Mer expressed by monocytes and macrophages can be distinguished from Mer expressed by platelets on the basis of the glycosylation of the extracellular domain of the receptor, and furthermore, that additional unique Mer glycoforms are expressed by different types of tumor cells. For example, in addition to the ectopic expression of Mer in certain cancers, including leukemia and lymphoma, the Mer extracellular domain in cancer cells (e.g., leukemia and lymphoma cells), is glycosylated in a manner that is different from the glycosylation present on Mer found in normal platelets and monocytes/macrophages. Moreover, different types of leukemia cells express different Mer glycoforms. The discovery of the presence of multiple unique Mer glycoforms that are differentially associated with cell type has significant diagnostic and therapeutic potential. For example, a specific glycoform of Mer can be used as a marker to identify a high risk leukemia or lymphoma patient that should be classified differently from other leukemia or lymphoma patients. In addition, therapeutic strategies can be designed to target a particular Mer glycoform and thus, a particular cell type or tumor type.

In addition to the unique Mer glycoforms present in various cell types as described herein, the present inventors have also detected variants (mutations or Mer protein alterations) in leukemia patient samples (*e.g*., resulting from mutations, deletions, or alternative splicing of the *Mer* gene). Such Mer mutants or splice variants (collectively referred to as Mer variants) can also be used to design additional diagnostic and therapeutic agents for use in the various methods described herein.

Therefore, in addition to the use of Mer as a diagnostic or prognostic marker, the unique Mer glycoforms and Mer variants discovered by the present inventors are valuable therapeutic targets for drug discovery and/or for *in vivo* treatments related to cancer or thrombosis. For example, small molecule inhibitors or drugs targeting glycoform-specific Mer proteins, or proteins directly downstream of Mer expressed by specific cell types, can be engineered and used in treatment regimens for such diseases. The Mer glycoforms can further be used to develop novel agents, such as specifically glycosylated soluble Mer (sMer) proteins, fusion proteins and chimeric proteins, that can be used in diagnostic or therapeutic methods. For example, the present invention encompasses the production and use of Mer glycoforms that are competitive inhibitors of Mer proteins that are endogenously expressed by particular cell types.

The present invention also relates to novel antibodies or antigen-binding fragments thereof that are capable of recognizing different glycoforms and protein variants of Mer protein, such as the aberrant glycoforms expressed by leukemia cells and protein variants within this glycoform. These antibodies are also useful as diagnostic and/or therapeutic reagents. Other embodiments of the present invention will be apparent to those of skill in the art from the description provided herein.

### Mer Glycoforms

Accordingly, one embodiment of the present invention relates to the recognition of unique, cell type-specific glycoforms of Mer, and the use of these glycoforms to design novel diagnostic and therapeutic agents (*e.g.,* antibodies, soluble Mer proteins, etc.) that relate to these glycoforms, as well as methods for using these agents and methods that take advantage of the discovery of the various Mer glycoforms. Specifically, the present inventors have discovered that several different glycosylation forms of the Mer protein are expressed by cells in a cell-type-dependent manner. Such glycosylation forms (also referred to as "Mer glycoforms") include, but are not limited to: a fully glycosylated 195-210 kD Mer protein that is expressed by monocytic cells (*e.g.,* monocytes and macrophages), which can be detected as any value between about 195 kD and about 210 kD, inclusive, such as between about 205 kD and about 210 kD, or about 205 kD, and several less-glycosylated Mer proteins (Mer proteins having less glycosylation than the monocytic cell types) that are expressed by other cell types. Glycoforms of Mer that are less than the fully glycosylated form expressed by monocytic cell types include, but are not limited to: (1) a glycoform expressed by platelets, which is from about 165 kD to about 170 kD; (2) two distinct glycoforms expressed by Mer lymphoblastic leukemias, including a glycoform found on acute lymphoblastic leukemia (ALL) cell lines that is between approximately 170 kD and approximately 190 kD (including from about 170 kD to about 180 kD or about 185 kD) and/or from about 135 kD to about 140 kD; and (3) a novel Mer glycoform specific to chronic myelogenous leukemia detected at from about 190 kD to about 195kD. Therefore, the present inventors have found that Mer glycoforms present in lymphoblastic leukemia or myelogenous leukemia, for example, differ from each other and from the forms of Mer found in monocytic cells and platelets.

The present invention makes use of Mer glycoforms that are normally (naturally, endogenously, constitutively) expressed by certain hematopoietic cells (*e.g.*, monocytic cells or platelets) or by other cell types that are not transformed (not neoplastically transformed, cancerous, or displaying aberrant or abnormal growth), as well as "aberrantly glycosylated" Mer glycoforms that are expressed by cells that are displaying aberrant or abnormal growth (cancerous, neoplastically transformed) or that are becoming or are predisposed to becoming cancerous. According to the present invention, a "Mer glycoform" is a Mer receptor tyrosine kinase that is described or characterized by the level of glycosylation of the extracellular domain of the receptor, which reflects the molecular weight of the Mer protein, as determined by any suitable method known in the art (*e.g.*, Western blot, other electrophoresis methods, chromatography, analytical ultracentrifugation, etc.). The glycosylation status of a Mer glycoform can be represented herein as a range of molecular weights (described above), which is reflective of potential differences in the determination of molecular weight, depending on the detection method used, standards used, and normal variation from assay to assay. A Mer glycoform can also be described more particularly by the available glycosylation sites on the protein and the number of such sites that are glycosylated, but will generally be referred to herein by the resulting molecular weight of the post-translationally modified protein. The terms "aberrantly glycosylated Mer", "aberrantly glycosylated Mer glycoform" or "aberrant Mer glycoforms", described in the present invention and defined herein refer to novel and unique Mer glycoforms that are expressed by cells that ectopically (abnormally) express Mer, such as cells displaying aberrant growth characteristics (*e.g.* cancer cells or neoplastically transformed cells, such as leukemia and lymphoma cells) or cells that are in the process of developing aberrant growth characteristics (*e.g.*, precancerous cells). The term "aberrant Mer glycoform" is not used to describe Mer that is expressed by hematopoietic cell lineages that normally express Mer (*e.g.,* monocytic cells and platelets). Aberrant Mer glycoforms include all glycoforms having molecular weights less than about 160 kD, and those forms between about 170 kD and about 190 kD. According to the present invention, the term "about" used in connection with the Mer glycoforms refers to a value that is plus or minus 2.5 kD.

### Mer Antibodies

One embodiment of the present invention relates to the development of novel anti-Mer antibodies, and particularly, novel anti-human Mer antibodies, and even more particularly, novel anti-human Mer monoclonal antibodies (mAb), any of which can be used in a variety of diagnostic and/or therapeutic methods as described below, as well as for the further study of the Mer expression, and particularly, ectopic Mer expression, in various cell types (*e.g.*, leukemia cells, lymphoblasts). Also included in this embodiment are antigen-binding fragments of such antibodies. An exemplary monoclonal antibody of the present invention can detect, by any method (*e.g.*, Western blot), the spectrum of Mer glycosylation states (Mer glycoforms) existing in normal human tissue and in human disease, or alternatively, selectively binds to a particular Mer glycoform and not to other Mer glycoforms. Antibodies of the present invention are useful for diagnostic applications in human cancer, thrombosis, and autoimmune disease. In the case of cancer, for example, since lymphoblastic leukemia and lymphoblastic lymphoma are considered to be different clinical manifestations of the same disease process, and are treated with similar chemotherapeutic regimes, this invention has a variety of diagnostic, prognostic, and therapeutic uses (*e.g.*, in leukemias and non-Hodgkin's lymphomas). In addition, antibodies of the present invention are useful in therapeutic applications directed to similar conditions. Antibodies of the present invention are also useful as valuable research tools and for purification of Mer glycoforms of the invention.

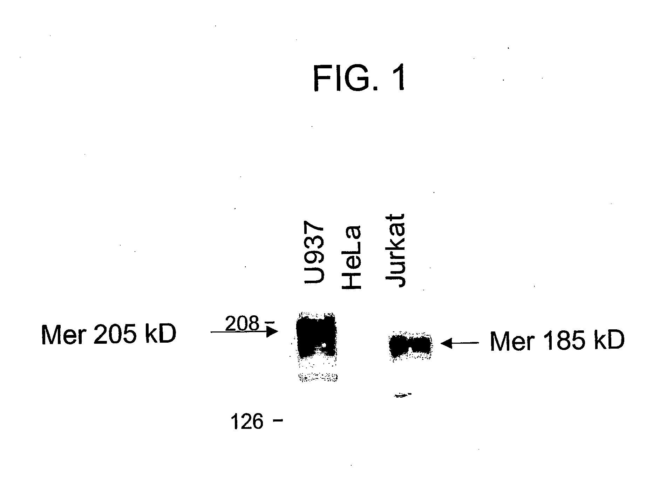

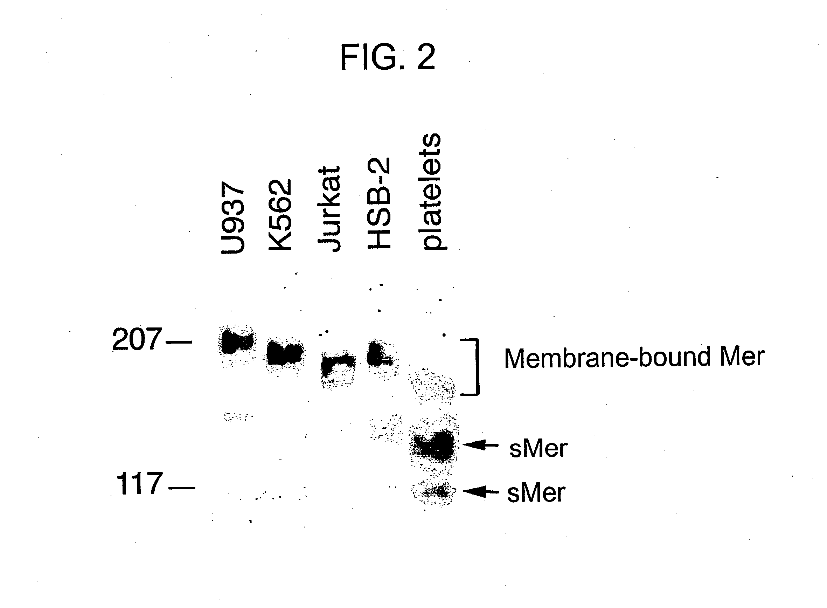

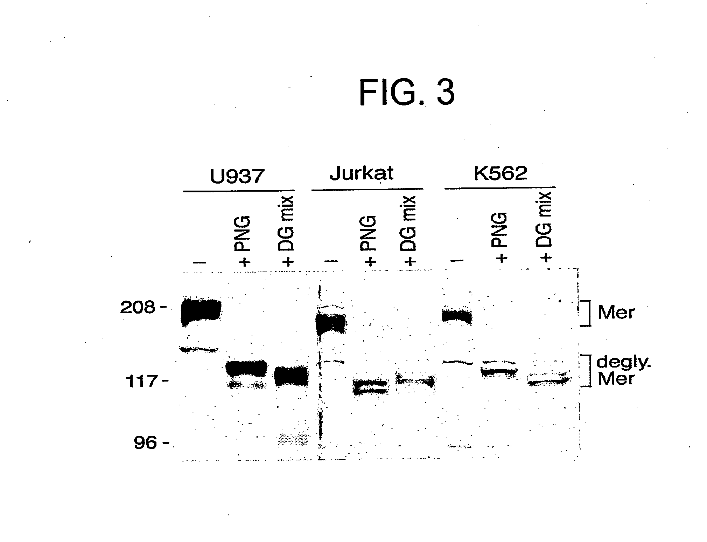

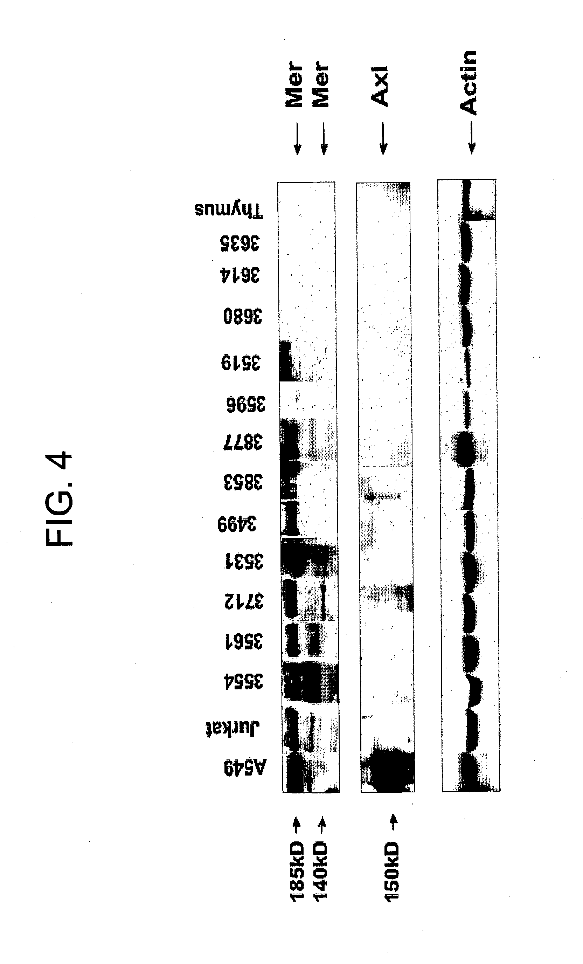

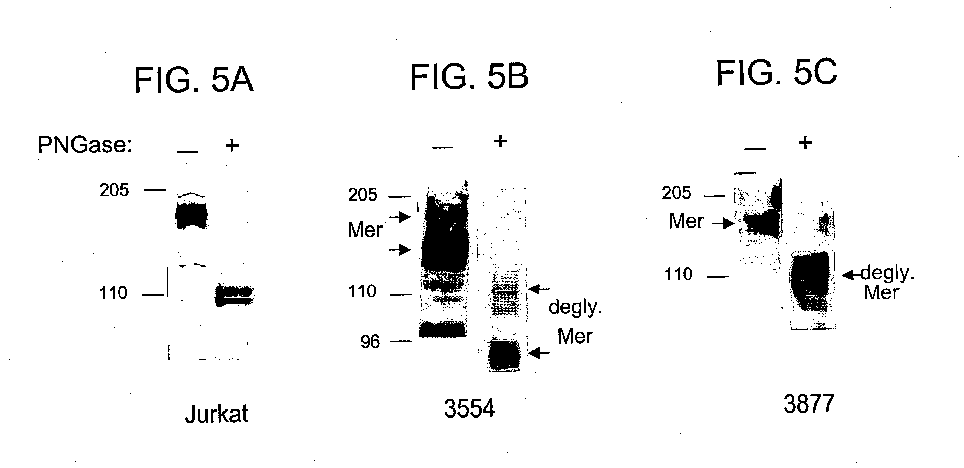

Accordingly, one embodiment of the present invention relates to anti-human Mer monoclonal antibodies that can detect the spectrum of Mer glycosylation states existing in human disease (*i.e.*, both normal and aberrant Mer glycoforms) (Figure 1), thereby allowing one to distinguish among the Mer glycoforms. To the best of the present inventors' knowledge, this is the first-described Mer antibody with such a specificity. For example, the anti-human Mer antibody referred to as the "311" antibody selectively recognizes multiple Mer glycoforms, including unique 170-190 kD forms of the protein in leukemia cell lines that are not present in other hematopoietic lineages (Figure 2). The antibodies of the present invention are also capable of distinguishing among protein variants of Mer that do not differ in the glycosylation of the protein. For example, as described in the Examples, Western blots of samples treated with glycosidases to remove all N-linked and most O-linked carbohydrates from the glycoproteins revealed different Mer glycosylation patterns for leukemia cell lines when probed with Mer monoclonal antibody 311 (Figure 3), further revealing the existence of distinct Mer protein variants within the unique Mer glycoform expressed by leukemia cells. Detection of the 170-190 kD glycoforms in T cell acute lymphoblastic leukemia (ALL) patient samples with the 311 antibody showed two different Mer glycoforms (170-190 kD and 135-145 kD) (Figure 4). Furthermore, mutations or deletions resulting in potential differences at the glycosylation sites (in addition to glycosylation variation) can account for the differences in the size of the Mer protein as reflected in gel electrophoresis (Figure 5). The discovery (through the utilization of the Mer 311 mAb as described above) that the Mer extracellular domain in leukemia cells that are resistant to common therapy is glycosylated in a manner different from the glycosylation present in platelets and monocytes/macrophages has diagnostic and therapeutic potential. The 311 monoclonal antibody is considered to be a prototypic antibody, and similar monoclonal antibodies to Mer (antibodies having the same or substantially similar specificity) are expected to show similar results. These similar monoclonal antibodies can be used to detect the presence of distinct Mer glycoforms in different cells types, such as in leukemia and lymphoma, by a variety of techniques, such as flow cytometry and Western blot, and can also be used to detect Mer-positive solid tumors, including lymphoma, by techniques such as immunohistochemistry (IHC) and Western blot.

Preferably, an antibody encompassed by the present invention includes any antibody that selectively binds to a conserved binding surface or epitope of a Mer protein, and preferably, to a conserved binding surface or epitope in the extracellular domain of the Mer protein (defined below). In one embodiment, an antibody of the present invention is capable of recognizing a spectrum of Mer glycoforms including a fully glycosylated Mer protein (an about 195 kD to about 210 kD Mer protein) as well as one or more other Mer glycoforms that are less than about 195 kD, and including aberrant Mer glycoforms as described above. Other Mer glycoforms can particularly include: a Mer glycoform that is from about 165 kD to about 170 kD; a Mer glycoform that is approximately 170 kD to about 190 kD (and more typically from about 170 kD to about 180 kD or more typically, about 185 kD), a Mer glycoform that is from about 135 kD to about 140 kD; and/or a Mer glycoform that is from about 190 kD to about 195kD.

According to the present invention, an "epitope" of a given protein or peptide or other molecule is generally defined, with regard to antibodies, as a part of or a site on a larger molecule to which an antibody or antigen-binding fragment thereof will bind, and against which an antibody will be produced. The term epitope can be used interchangeably with the term "antigenic determinant", "antibody binding site", or "conserved binding surface" of a given protein or antigen. More specifically, an epitope can be defined by both the amino acid residues involved in antibody binding and also by their conformation in three dimensional space (e.g., a conformational epitope or the conserved binding surface). An epitope can be included in peptides as small as about 4-6 amino acid residues, or can be included in larger segments of a protein, and need not be comprised of contiguous amino acid residues when referring to a three dimensional structure of an epitope, particularly with regard to an antibody-binding epitope. Antibody-binding epitopes are frequently conformational epitopes rather than a sequential epitope (i.e., linear epitope), or in other words, an epitope defined by amino acid residues arrayed in three dimensions on the surface of a protein or polypeptide to which an antibody binds. As mentioned above, the conformational epitope is not comprised of a contiguous sequence of amino acid residues, but instead, the residues are perhaps widely separated in the primary protein sequence, and are brought together to form a binding surface by the way the protein folds in its native conformation in three dimensions.

As used herein, the term "selectively binds to" refers to the specific binding of one protein to another (e.g., an antibody, fragment thereof, or binding partner to an antigen), wherein the level of binding, as measured by any standard assay (e.g., an immunoassay), is statistically significantly higher than the background control for the assay. For example, when performing an immunoassay, controls typically include a reaction well/tube that contain antibody or antigen binding fragment alone (i.e., in the absence of antigen), wherein an amount of reactivity (e.g., non-specific binding to the well) by the antibody or antigen binding fragment thereof in the absence of the antigen is considered to be background. Binding can be measured using a variety of methods standard in the art, including, but not limited to: Western blot, immunoblot, enzyme-linked immunosorbant assay (ELISA), radioimmunoassay (RIA), immunoprecipitation, surface plasmon resonance, chemiluminescence, fluorescent polarization, phosphorescence, immunohistochemical analysis, matrix-assisted laser desorption/ionization time-of-flight (MALDI-TOF) mass spectrometry, microcytometry, microarray, microscopy, fluorescence activated cell sorting (FACS), and flow cytometry.

One embodiment of the present invention includes an antibody or antigen binding fragment thereof that is a competitive inhibitor of the binding of a Mer ligand (*e.g.*, Gas6 or Protein S) to a Mer glycoform that is expressed by a particular cell or cell type. In another embodiment, the present invention includes an antibody or antigen binding fragment thereof that is a competitive inhibitor of the binding of another anti-Mer antibody described herein (*e.g.*, the 311 antibody). According to the present invention, a competitive inhibitor is an inhibitor (*e.g.*, another antibody or antigen binding fragment or polypeptide) that binds to Mer that is expressed by a cell, and inhibits or blocks the binding of a natural Mer ligand (*e.g.*, Gas6 or Protein S) to the Mer that is expressed by the cell. The antibody competitive inhibitor can also be defined by its ability to bind to Mer expressed by the cell at the same or similar epitope as another anti-Mer antibody described herein (*e.g.*, mAb 311) such that binding of the anti-Mer antibody described herein is inhibited. A competitive inhibitor may bind to the target (*e.g.*, Mer) with a greater affinity for the target than the Mer ligand or the other anti-Mer antibody. Other types of competitive inhibitors related to soluble Mer are described below. A competitive inhibitor can be used in a manner similar to that described herein for the anti-Mer antibody. Competition assays can be performed using standard techniques in the art (e.g., competitive ELISA or other binding assays). For example, competitive inhibitors can be detected and quantitated by their ability to inhibit the binding of Mer to another, labeled anti-Mer antibody.

Isolated antibodies of the present invention can include serum containing such antibodies, or antibodies that have been purified to varying degrees. Whole antibodies of the present invention can be polyclonal or monoclonal. Alternatively, functional equivalents of whole antibodies, such as antigen binding fragments in which one or more antibody domains are truncated or absent (*e.g.*, Fv, Fab, Fab', or F(ab)₂ fragments), as well as genetically-engineered antibodies or antigen binding fragments thereof, including single chain antibodies, humanized antibodies (discussed below), fully human antibodies, antibodies that can bind to more than one epitope (*e.g.*, bi-specific antibodies), or antibodies that can bind to one or more different antigens (*e.g.*, bi- or multi-specific antibodies), may also be employed in the invention.

Limited digestion of an immunoglobulin with a protease may produce two fragments. An antigen binding fragment is referred to as an Fab, an Fab', or an F(ab')₂ fragment. A fragment lacking the ability to bind to antigen is referred to as an Fc fragment. An Fab fragment comprises one arm of an immunoglobulin molecule containing a L chain (V_{L} + C_{L} domains) paired with the V_{H} region and a portion of the C_{H} region (CH1 domain). An Fab' fragment corresponds to an Fab fragment with part of the hinge region attached to the CH1 domain. An F(ab')₂ fragment corresponds to two Fab' fragments that are normally covalently linked to each other through a di-sulfide bond, typically in the hinge regions.

The C_{H} domain defines the isotype of an immunoglobulin and confers different functional characteristics depending upon the isotype. For example, µ constant regions enable the formation of pentameric aggregates of IgM molecules and α constant regions enable the formation of dimers.

Other functional aspects of an immunoglobulin molecule include the valency of an immunoglobulin molecule, the affinity of an immunoglobulin molecule, and the avidity of an immunoglobulin molecule. As used herein, affinity refers to the strength with which an immunoglobulin molecule binds to an antigen at a single site on an immunoglobulin molecule (*i.e.*, a monovalent Fab fragment binding to a monovalent antigen). Affinity differs from avidity which refers to the sum total of the strength with which an immunoglobulin binds to an antigen. Immunoglobulin binding affinity can be measured using techniques standard in the art, such as competitive binding techniques, equilibrium dialysis or BIAcore methods. As used herein, valency refers to the number of different antigen binding sites per immunoglobulin molecule (*i.e*., the number of antigen binding sites per antibody molecule of antigen binding fragment). For example, a monovalent immunoglobulin molecule can only bind to one antigen at one time, whereas a bivalent immunoglobulin molecule can bind to two or more antigens at one time, and so forth.

In one embodiment, the antibody is a bi- or multi-specific antibody. A bi-specific (or multi-specific) antibody is capable of binding two (or more) antigens, as with a divalent (or multivalent) antibody, but in this case, the antigens are different antigens (*i.e.*, the antibody exhibits dual or greater specificity). For example, an antibody that selectively binds to Mer can be constructed as a bi-specific antibody, wherein the second antigen binding specificity is for a desired target, such as another cell surface marker on a target cell.

Antibodies of the present invention can include, but are not limited to, neutralizing antibodies, catalytic antibodies and blocking (binding) antibodies. According to the present invention, a neutralizing antibody is an antibody that reacts with an infectious agent (usually a virus) and destroys or inhibits its infectivity and virulence. A catalytic antibody is an antibody selected for its ability to catalyze a chemical reaction by binding to and stabilizing the transition-state intermediate. A blocking antibody is an antibody that binds to an antigen and blocks another antibody or agent from later binding to that antigen.

In one embodiment, antibodies of the present invention include humanized antibodies. Humanized antibodies are molecules having an antigen binding site derived from an immunoglobulin from a non-human species, the remaining immunoglobulin-derived parts of the molecule being derived from a human immunoglobulin. The antigen binding site may comprise either complete variable regions fused onto human constant domains or only the complementarity determining regions (CDRs) grafted onto appropriate human framework regions in the variable domains. Humanized antibodies can be produced, for example, by modeling the antibody variable domains, and producing the antibodies using genetic engineering techniques, such as CDR grafting (described below). A description various techniques for the production of humanized antibodies is found, for example, in Morrison et al. (1984) Proc. Natl. Acacl. Sci. USA 81:6851-55; Whittle et al. (1987) Prot. Eng. 1:499-505; Co et al. (1990) J. Immunol. 148:1149-1154; Co et al. (1992) Proc. Natl. Acad. Sci. USA 88:2869-2873; Carter et al. (1992) Proc. Natl. Acad. Sci. 89:4285-4289; Routledge et al. (1991) Eur. J. Immunol. 21:2717-2725 and PCT Patent Publication Nos. WO 91/09967; WO 91/09968 and WO 92/113831.

In one embodiment, antibodies of the present invention include fully human antibodies. Fully human antibodies are fully human in nature. One method to produce such antibodies having a particular binding specificity includes obtaining human antibodies from immune donors (e.g., using EBV transformation of B-cells or by PCR cloning and phage display). In addition, and more typically, synthetic phage libraries have been created which use randomized combinations of synthetic human antibody V-regions. By selection on antigen, "fully human antibodies: can be made in which it is assumed the V-regions are very human like in nature. Phage display libraries are described in more detail below. Finally, fully human antibodies can be produced from transgenic mice. Specifically, transgenic mice have been created which have a repertoire of human immunoglobulin germline gene segments. Therefore, when immunized, these mice produce human like antibodies. All of these methods are known in the art.

Genetically engineered antibodies of the invention include those produced by standard recombinant DNA techniques involving the manipulation and re-expression of DNA encoding antibody variable and/or constant regions. Particular examples include, chimeric antibodies, where the V_{H} and/or V_{L} domains of the antibody come from a different source as compared to the remainder of the antibody, and CDR grafted antibodies (and antigen binding fragments thereof), in which at least one CDR sequence and optionally at least one variable region framework amino acid is (are) derived from one source and the remaining portions of the variable and the constant regions (as appropriate) are derived from a different source. Construction of chimeric and CDR-grafted antibodies are described, for example, in European Patent Applications: EP-A 0194276, EP-A 0239400, EP-A 0451216 and EP-A 0460617.

In one embodiment, chimeric antibodies are produced according to the present invention comprising antibody variable domains that bind to Mer and fused to these domains, a protein that serves as a second targeting moiety. For example, the targeting moiety can include a protein that is associated with the cell or tissue to be targeted or with a particular system in the animal.

In an additional embodiment, the present invention provides a method for the production of a monoclonal antibody that specifically binds to specific glycoforms of the Mer transmembrane receptor tyrosine kinase. Such an antibody can include any of the antibodies described herein, including, but not limited to, blocking or binding antibodies, neutralizing antibodies and catalytic antibodies. The method includes the steps of: (a) immunizing an animal with a specific Mer transmembrane receptor tyrosine kinase (*i.e.*, a specific Mer glycoform) such that antibody producing cells are produced in the animal; (b) removing and immortalizing the antibody producing cells; (c) selecting and cloning the immortalized antibody producing cells producing the desired antibody; and (d) isolating the antibodies produced by the selected, cloned immortalized antibody producing cells. In one embodiment, the Mer transmembrane receptor tyrosine kinase used to produce the antibody is a soluble Mer (*i.e.*, a fragment of Mer that comprises at least the portion of the extracellular domain of Mer that binds to its natural (cognate) ligands, such as Gas6 or Protein S.) Soluble Mer or extracellular domains of Mer are described in detail below. In a further embodiment, the invention provides for the monoclonal antibody produced by the above method.

Generally, in the production of an antibody, a suitable experimental animal, such as, for example, but not limited to, a rabbit, a sheep, a hamster, a guinea pig, a mouse, a rat, or a chicken, is exposed to an antigen against which an antibody is desired. Typically, an animal is immunized with an effective amount of antigen that is injected into the animal. An effective amount of antigen refers to an amount needed to induce antibody production by the animal. The animal's immune system is then allowed to respond over a pre-determined period of time. The immunization process can be repeated until the immune system is found to be producing antibodies to the antigen. In order to obtain polyclonal antibodies specific for the antigen, serum is collected from the animal that contains the desired antibodies (or in the case of a chicken, antibody can be collected from the eggs). Such serum is useful as a reagent. Polyclonal antibodies can be further purified from the serum (or eggs) by, for example, treating the serum with ammonium sulfate.

Monoclonal antibodies may be produced according to the methodology of Kohler and Milstein (Nature 256:495-497, 1975), or using the human B-cell hybridoma method, Kozbor, J., Immunol, 133:3001 (1984); Brodeur et al., Monoclonal Antibody Production Techniques and Applications, pp.51-63 (Marcel Dekker, Inc., New York, 1987). For example, B lymphocytes are recovered from the spleen (or any suitable tissue) of an immunized animal and then fused with myeloma cells to obtain a population of hybridoma cells capable of continual growth in suitable culture medium. Hybridomas producing the desired antibody are selected by testing the ability of the antibody produced by the hybridoma to bind to the desired antigen. The hybridomas may be cloned and the antibodies may be produced by and then isolated from the hybridomas. A preferred method to produce antibodies of the present invention includes (a) administering to an animal an effective amount of a protein or peptide (e.g., a Mer protein or peptide including extracellular domains thereof) to produce the antibodies and (b) recovering the antibodies. As used herein, the term "monoclonal antibody" includes chimeric, humanized, and human forms of a monoclonal antibody. Monoclonal antibodies are often synthesized in the laboratory in pure form by a single clone (population) of cells. These antibodies can be made in large quantities and have a specific affinity for certain target antigens which can be found on the surface of cells. Monoclonal antibodies directed toward aberrant Mer glycoforms or a soluble form of the extracellular Mer receptor tyrosine kinase may be most easily produced using any of the well known methods that provides for the production of antibody molecules by continuous cell lines in culture.

In another method, antibodies of the present invention are produced recombinantly. For example, once a cell line, for example a hybridoma, expressing an antibody according to the invention has been obtained, it is possible to clone therefrom the cDNA and to identify the variable region genes encoding the desired antibody, including the sequences encoding the CDRs. From here, antibodies and antigen binding fragments according to the invention may be obtained by preparing one or more replicable expression vectors containing at least the DNA sequence encoding the variable domain of the antibody heavy or light chain and optionally other DNA sequences encoding remaining portions of the heavy and/or light chains as desired, and transforming/transfecting an appropriate host cell, in which production of the antibody will occur. Suitable expression hosts include bacteria, (for example, an *E. coli* strain), fungi, (in particular yeasts, *e.g*. members of the genera *Pichia*, *Saccharomyces*, or *Kluyveromyces,*) and mammalian cell lines, *e.g*. a non-producing myeloma cell line, such as a mouse NSO line, or CHO cells. In order to obtain efficient transcription and translation, the DNA sequence in each vector should include appropriate regulatory sequences, particularly a promoter and leader sequence operably linked to the variable domain sequence. Particular methods for producing antibodies in this way are generally well known and routinely used. For example, basic molecular biology procedures are described by Maniatis et al. (Molecular Cloning, Cold Spring Harbor Laboratory, New York, 1989); DNA sequencing can be performed as described in Sanger et al. (PNAS 74, 5463, (1977)) and the Amersham International plc sequencing handbook; and site directed mutagenesis can be carried out according to the method of Kramer et al. (Nucl. Acids Res. 12, 9441, (1984)) and the Anglian Biotechnology Ltd. handbook. Additionally, there are numerous publications, including patent specifications, detailing techniques suitable for the preparation of antibodies by manipulation of DNA, creation of expression vectors and transformation of appropriate cells, for example as reviewed by Mountain A and Adair, J R in Biotechnology and Genetic Engineering Reviews (ed. Tombs, M P, 10, Chapter 1, 1992, Intercept, Andover, UK) and in the aforementioned European Patent Applications.

Alternative methods, employing, for example, phage display technology (see for example, U.S. Patent No. 5,969,108, U.S. Patent No. 5,565,332, U.S. Patent No. 5,871,907, U.S. Patent No. 5,858,657, U.S. Patent No. 5,223,409; Fuchs et al. Bio/Technology, 9:1370-1372 (1991); or Griffiths et al. EMBO J., 12:725-734 (1993)) or the selected lymphocyte antibody method of US Patent No. 5,627,052 may also be used for the production of antibodies and/or antigen fragments of the invention, as will be readily apparent to the skilled individual. For example, a monoclonal antibody to an aberrantly glycosylated Mer polypeptide can be identified and isolated by screening a recombinant combinatorial immunoglobulin library (*e.g.*, an antibody phage display library) with the polypeptide to thereby isolate immunoglobulin library members that bind the polypeptide. Kits for generating and screening phage display libraries are well known and commercially available.

The Mer 311 monoclonal antibody, or other monoclonal antibodies produced with the polypeptides described above, can be used to isolate a Mer polypeptide including any Mer glycoform that is specifically recognized by the antibody, by standard techniques (such as affinity chromatography or immunoprecipitation). An antibody specific to (that selectively binds to) a Mer polypeptide or another peptide of the present invention can be used to detect Mer (*e.g.*, in a cellular lysate, cell supernatant, or tissue sample) to evaluate the abundance of, pattern of expression of, glycosylation of, and variants of Mer. Antibodies can be used diagnostically to monitor protein levels in tissue as part of a clinical testing procedure, *e.g*., to determine the efficacy of a given treatment regimen or to select appropriate patients for a Mer-specific therapy. Furthermore, the presently claimed glycoforms, isoforms, or mutated Mer proteins may have increased tyrosine kinase activity that plays a role in oncogenesis. Thus in one aspect, the monoclonal antibodies of the invention, which recognize and bind or inactivate native, aberrant glycoforms of, or altered Mer protein in leukemia and lymphoma cells, may be used to treat Mer positive cancer patients. These embodiments of the invention are described in detail below.

Coupling the antibody to a detectable substance can facilitate detection. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### Mer Proteins, Soluble Mer Protein and Mer Protein Variants

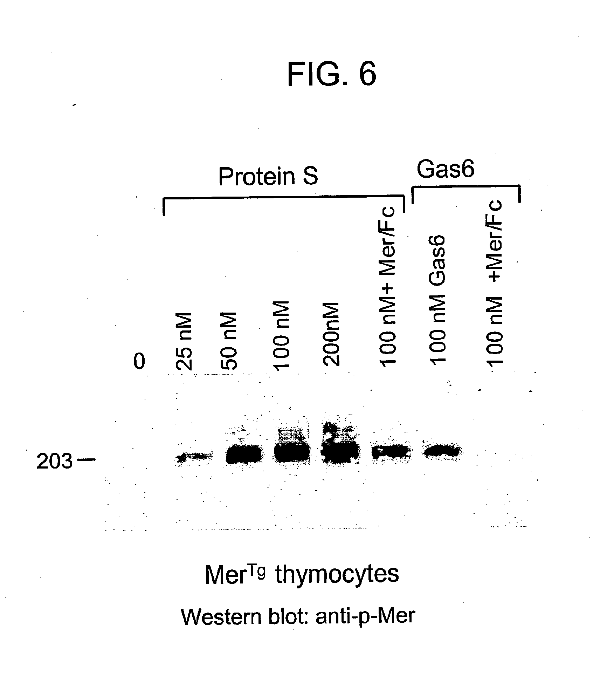

Another embodiment of the invention concerns the discovery that the Mer extracellular domain can be cleaved, and that this soluble portion of the Mer receptor tyrosine kinase is useful in inhibiting activation of the Mer receptor tyrosine kinase. The invention further contemplates novel, soluble forms of the Mer receptor tyrosine kinase present in human and mouse plasma that play a significant role in disease states or conditions where the Mer receptor tyrosine kinase is activated, such as cell survival signaling and cell proliferation in the case of Mer positive cancers, and in anticoagulation. While the inventors do not wish to be limited to their present theory as to how the invention operates, it is believed that the anticoagulation aspects of this invention function through the sMer interaction with its ligands including anticoagulant Protein S (Figure 6) and Gas6. The novel, soluble Mer (sMer) proteins include specific glycoforms of sMer, particularly including the Mer glycoforms that have been specifically described herein. As used herein, the term "soluble form" of Mer, "sMer" or "soluble Mer" refers to a Mer receptor tyrosine kinase that is cleaved near the transmembrane receptor or that includes any portion of the extracellular domain of Mer (described below) that retains the ability to bind to a Mer ligand (*e.g.*, Gas6 or Protein S). The exact cleavage site is not critical. Alternatively, the soluble form can be generated by differential splicing, by recombinant means, or by post-translational proteolytic cleavage, as described in additional detail below. A "soluble Mer glycoform" or "sMer glycoform" refers to a soluble form of Mer as described above that is additionally characterized by its particular glycosylation pattern or level of glycosylation.

As discussed above, the nucleic acid sequence (genomic or mRNA) and amino acid sequence for Mer from several different species are known in the art (*e.g.*, see U.S. Patent No. 5,585,269). The nucleic acid sequence for human Mer (mRNA) is represented herein by SEQ ID NO: 1. SEQ ID NO:1 encodes human Mer protein, represented herein by SEQ ID NO:2. The nucleic acid sequence for murine Mer and the amino acid sequence of the protein encoded thereby are represented by SEQ ID NO:3 and SEQ ID NO:4, respectively. The nucleic acid sequence for rat Mer and the amino acid sequence of the protein encoded thereby are represented by SEQ ID NO:5 and SEQ ID NO:6, respectively. The nucleic acid sequence for chicken Mer and the amino acid sequence of the protein encoded thereby are represented by SEQ ID NO:7 and SEQ ID NO:8, respectively. The extracellular domain of human Mer (SEQ ID NO:2) spans amino acid positions from about 1 to about 473. The corresponding domain in other species can be readily determined by aligning the sequences. However, as discussed above, one may produce a soluble Mer that is cleaved at a site other than position 484 of SEQ ID NO:2 (or the corresponding position in Mer from other species). For example, soluble Mer proteins of the invention can include any smaller portions (fragments) of the extracellular domain of Mer that retain the ability to bind to a Mer ligand (*e.g.,* Gas6 or Protein S).

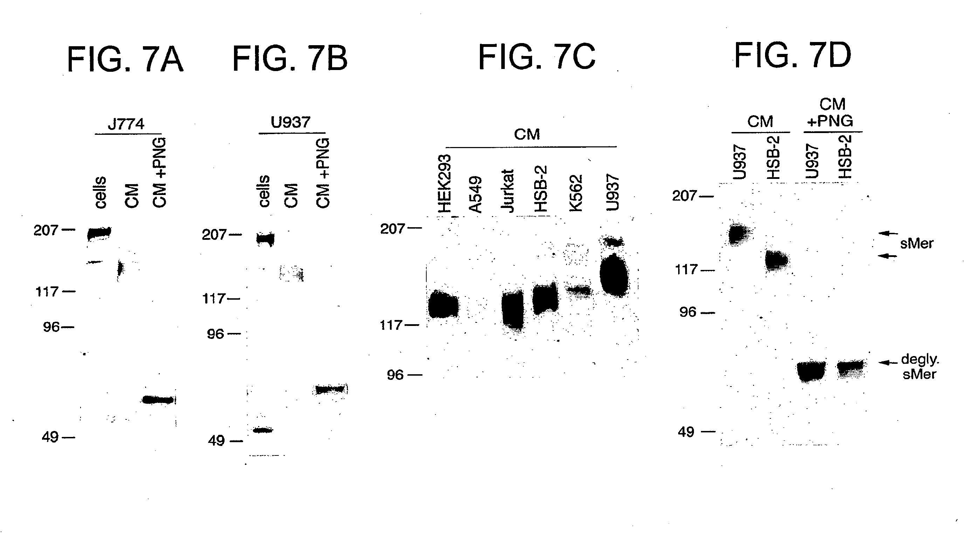

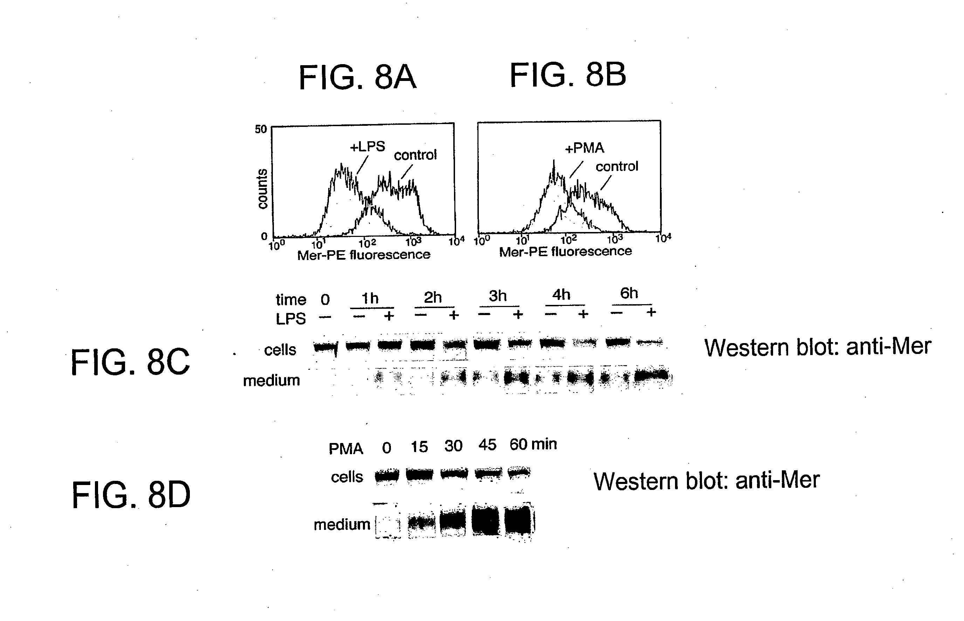

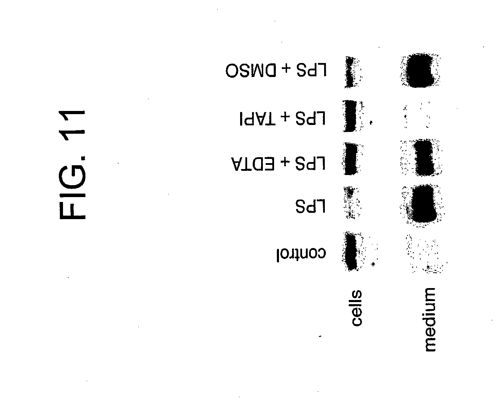

In one embodiment of the invention, the 165-170 kD Mer protein in platelets and the 195-210 kD Mer protein in monocytes is post-translationally processed by cleavage of the extracellular domain via a Tumor Necrosis Factor-α (TNF) converting enzyme (TACE)-like metalloprotease. The cleavage results in a soluble extracellular domain protein (approximately 150kD) (Figures 7, 8, 9 and 10) and a membrane-bound kinase domain. The proteolytic cleavage of Mer can be enhanced by lipopolysaccharide (LPS) and Phorbol 12-myristate B-acetate (PMA) (Figure 8) and can be specifically inhibited by a TNF-α Protease Inhibitor (TAPI), a TACE inhibitor (Figure 11). In an alternate embodiment of the invention, a soluble Mer protein is produced as a result of mRNA splicing. Sequence analysis of some full-length Mer samples has led the present inventors to discover the existence of a novel exon encoding a stop codon. This novel RNA splice form consists of exons 1 through 7 in human Mer gene juxtaposed to a novel exon 7A. The resulting truncated, soluble Mer contains 381 amino acids from exons 1 through 7 and 12 amino acids from exon 7A prior to the inframe stop codon.

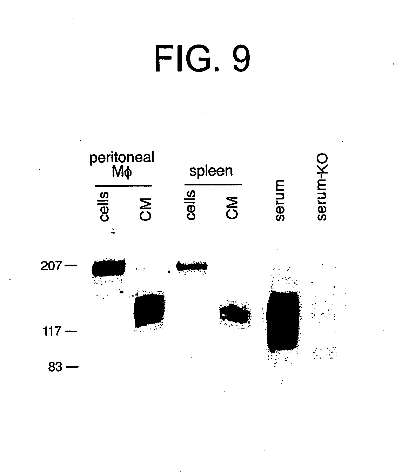

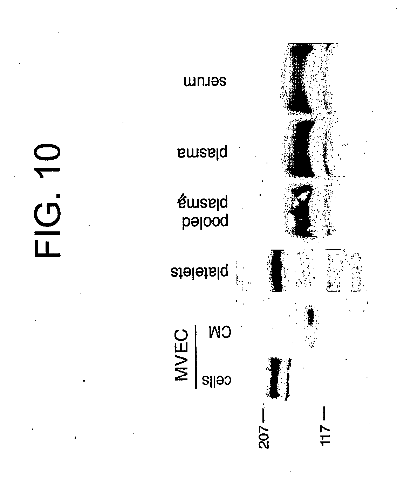

Significant amounts of the soluble Mer protein are present in human and mouse serum (Figures 9 and 10). As described in additional detail below, the soluble form can be produced by recombinant methods. Such a recombinant form could be created with alternative forms of glycosylation or indeed, with no glycosylation at all. In a preferred embodiment of the present invention, the soluble Mer protein is produced as a specific glycosylation form that is useful in a diagnostic or therapeutic method of the present invention. The glycosylation forms include any of the forms described herein, including: a fully glycosylated 195-210 kD Mer glycoform; a glycoform having a molecular weight of from about 165 kD to about 170 kD; a glycoform having a molecular weight of from about 170 kD to about 190 kD; a glycoform having a molecular weight of from about 135 kD to about 140 kD, and a glycoform having a molecular weight of from about 190 kD to about 195kD.

In one embodiment of the present invention, the discovery of different Mer glycoforms by the present inventors is used to selectively and advantageously produce soluble Mer glycoforms that are therapeutically useful because they are competitive inhibitors of a Mer glycoform that is expressed by a particular cell type. For example, a Mer protein having a particular level or pattern of glycosylation may have a higher binding affinity for a Mer ligand (*e.g.,* Gas6 or Protein S) than the endogenous Mer receptor that is expressed by a particular cell type. Such a Mer glycoform can be produced as a soluble Mer protein and then used to inhibit the binding of a Mer ligand to its endogenous Mer receptor, for example, in a therapeutic treatment of a clotting disorder or cancer. Such a soluble Mer glycoform can be targeted to the cell type of interest, if desired.

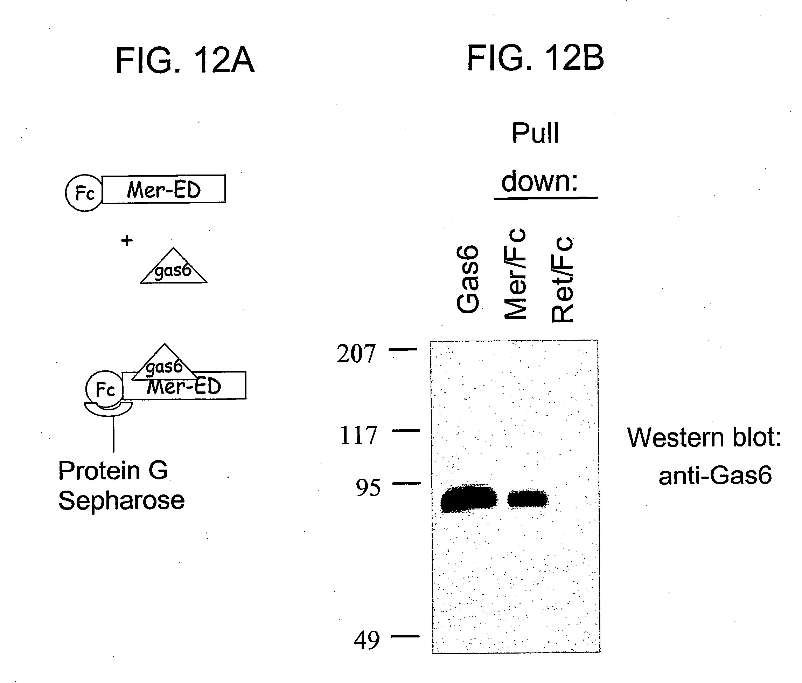

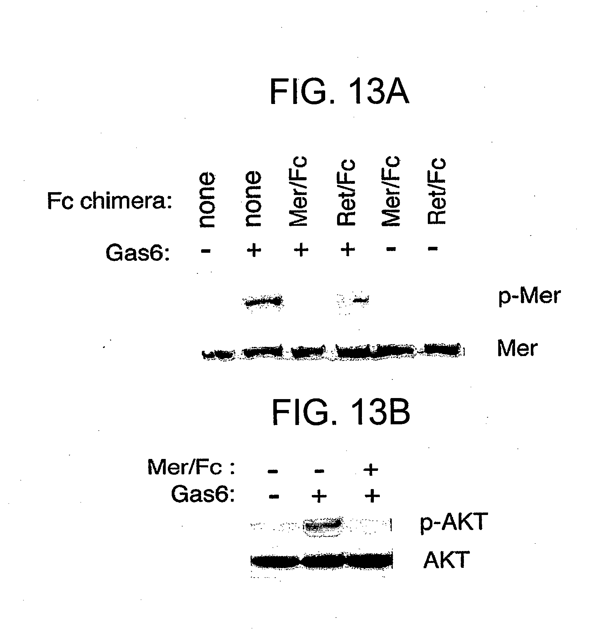

The present inventors have shown that soluble Mer protein directly binds the Mer Gas6 (Figure 12) and Protein S, thereby inhibiting stimulation of full-length Mer (Figures 6 and 13). Gas6 and Protein S are also ligands for Axl and Tyro-3. The cleavage of Mer therefore represents a mechanism of directly regulating (including upregulating or downregulating) the numerous functions of the Mer, Axl and Tyro-3 ligands, including promoting platelet adhesion and clot stability, stimulating cell proliferation, inducing cell adhesion and chemotaxis, and preventing apoptosis. Finally, the cleavage of Mer or the use of its soluble form, and specifically, cleavage of specific Mer glycoforms or the use of soluble forms of the Mer glycoforms described herein, represents a mechanism to indirectly modulate (regulate, modify) the activities of the Mer, Axl and Tyro-3 tyrosine kinases by modulating the functions of Protein S, Gas6 and other Mer ligands.

Accordingly, embodiments of the present invention also pertain to isolated polypeptides described herein, and specifically various Mer glycoforms, and particularly aberrant Mer glycoforms and/or soluble forms of the extracellular Mer receptor tyrosine kinase, including those expressed by nucleic acids encoding a Mer variant (described below).

As used herein, reference to an isolated protein or polypeptide in the present invention, including an isolated Mer protein, includes full-length proteins, fusion proteins, or any fragment or other homologue (variant) of such a protein. The amino acid sequence for Mer from human, mouse, rat and chicken are described herein as exemplary Mer proteins (see above). Reference to a Mer protein can include, but is not limited to, purified Mer protein, recombinantly produced Mer protein, membrane bound Mer protein, Mer protein complexed with lipids, soluble Mer protein, any Mer glycoform, and isolated Mer protein associated with other proteins. More specifically, an isolated protein, such as a Mer protein, according to the present invention, is a protein (including a polypeptide or peptide) that has been removed from its natural milieu (i.e., that has been subject to human manipulation) and can include purified proteins, partially purified proteins, recombinantly produced proteins, and synthetically produced proteins, for example. As such, "isolated" does not reflect the extent to which the protein has been purified. The term "polypeptide" refers to a polymer of amino acids, and not to a specific length; thus, peptides, oligopeptides and proteins are included within the definition of a polypeptide. As used herein, a polypeptide is said to be "purified" when it is substantially free of cellular material when it is isolated from recombinant and non-recombinant cells, or free of chemical precursors or other chemicals when it is chemically synthesized. A polypeptide, however, can be joined to another polypeptide with which it is not normally associated in a cell (*e.g.*, in a "fusion protein") and still be "isolated" or "purified."

In addition, and by way of example, a "human Mer protein" refers to a Mer protein (generally including a homologue of a naturally occurring Mer protein) from a human (*Homo sapiens*) or to a Mer protein that has been otherwise produced from the knowledge of the structure (e.g., sequence) and perhaps the function of a naturally occurring Mer protein from *Homo sapiens.* In other words, a human Mer protein includes any Mer protein that has substantially similar structure and function of a naturally occurring Mer protein from *Homo sapiens* or that is a biologically active (i.e., has biological activity) homologue of a naturally occurring Mer protein from *Homo sapiens* as described in detail herein. As such, a human Mer protein can include purified, partially purified, recombinant, mutated/modified and synthetic proteins. According to the present invention, the terms "modification" and "mutation" can be used interchangeably, particularly with regard to the modifications/mutations to the amino acid sequence of Mer (or nucleic acid sequences) described herein. An isolated protein useful as an antagonist or agonist according to the present invention can be isolated from its natural source, produced recombinantly or produced synthetically.

The polypeptides of the invention also encompass fragment and sequence variants, generally referred to herein as homologues. As used herein, the term "homologue" is used to refer to a protein or peptide which differs from a naturally occurring protein or peptide (i.e., the "prototype" or "wild-type" protein) by minor modifications to the naturally occurring protein or peptide, but which maintains the basic protein and side chain structure of the naturally occurring form. Such changes include, but are not limited to: changes in one or a few amino acid side chains; changes one or a few amino acids, including deletions (e.g., a truncated version of the protein or peptide) insertions and/or substitutions; changes in stereochemistry of one or a few atoms; and/or minor derivatizations, including but not limited to: methylation, glycosylation, phosphorylation, acetylation, myristoylation, prenylation, palmitation, amidation and/or addition of glycosylphosphatidyl inositol. A homologue can have either enhanced, decreased, or substantially similar properties as compared to the naturally occurring protein or peptide. A homologue can include an agonist of a protein or an antagonist of a protein.

Variants or homologues include a substantially homologous polypeptide encoded by the same genetic locus in an organism, *i.e*., an allelic variant, as well as other splicing variants. A naturally occurring allelic variant of a nucleic acid encoding a protein is a gene that occurs at essentially the same locus (or loci) in the genome as the gene which encodes such protein, but which, due to natural variations caused by, for example, mutation or recombination, has a similar but not identical sequence. Allelic variants typically encode proteins having similar activity to that of the protein encoded by the gene to which they are being compared. One class of allelic variants can encode the same protein but have different nucleic acid sequences due to the degeneracy of the genetic code. Allelic variants can also comprise alterations in the 5' or 3' untranslated regions of the gene (e.g., in regulatory control regions). Allelic variants are well known to those skilled in the art.

The terms variant or homologue may also encompass polypeptides derived from other genetic loci in an organism, but having substantial homology to any of the previously defined aberrant Mer glycoforms or a soluble form of the extracellular Mer receptor tyrosine kinase, or polymorphic variants thereof. Variants also include polypeptides substantially homologous or identical to these polypeptides but derived from another organism. Variants also include polypeptides that are substantially homologous or identical to these polypeptides that are produced by chemical synthesis.

Variants also include polypeptides that are substantially homologous or identical to these polypeptides that are produced by recombinant methods. As used herein, two polypeptides (or a region of the polypeptides) are substantially homologous or identical when the amino acid sequences are at least about 45-55%, typically at least about 70-75%, more typically at least about 80-85%, and most typically greater than about 90% or more homologous or identical. In one embodiment, a Mer homologue comprises, consists essentially of, or consists of, an amino acid sequence that is at least about 45%, or at least about 50%, or at least about 55%, or at least about 60%, or at least about 65%, or at least about 70%, or at least about 75%, or at least about 80%, or at least about 85%, or at least about 90%, or at least about 95% identical, or at least about 95% identical, or at least about 96% identical, or at least about 97% identical, or at least about 98% identical, or at least about 99% identical (or any percent identity between 45% and 99%, in whole integer increments), to a naturally occurring Mer amino acid sequence. A homologue of Mer differs from a reference (*e.g.,* wild-type) Mer and therefore is less than 100% identical to the reference Mer at the amino acid level. Wild-type Mer sequences include, but are not limited to, SEQ ID NO:2, SEQ ID NO:4, SEQ ID NO:6 and SEQ ID NO:8.

As used herein, unless otherwise specified, reference to a percent (%) identity refers to an evaluation of homology which is performed using: (1) a BLAST 2.0 Basic BLAST homology search using blastp for amino acid searches and blastn for nucleic acid searches with standard default parameters, wherein the query sequence is filtered for low complexity regions by default (described in Altschul, S.F., Madden, T.L., Schaaffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402, incorporated herein by reference in its entirety); (2) a BLAST 2 alignment (using the parameters described below); (3) and/or PSI-BLAST with the standard default parameters (Position-Specific Iterated BLAST. It is noted that due to some differences in the standard parameters between BLAST 2.0 Basic BLAST and BLAST 2, two specific sequences might be recognized as having significant homology using the BLAST 2 program, whereas a search performed in BLAST 2.0 Basic BLAST using one of the sequences as the query sequence may not identify the second sequence in the top matches. In addition, PSI-BLAST provides an automated, easy-to-use version of a "profile" search, which is a sensitive way to look for sequence homologues. The program first performs a gapped BLAST database search. The PSI-BLAST program uses the information from any significant alignments returned to construct a position-specific score matrix, which replaces the query sequence for the next round of database searching. Therefore, it is to be understood that percent identity can be determined by using any one of these programs.

Two specific sequences can be aligned to one another using BLAST 2 sequence as described in Tatusova and Madden, (1999), "Blast 2 sequences - a new tool for comparing protein and nucleotide sequences", FEMS Microbiol Lett. 174:247-250, incorporated herein by reference in its entirety. BLAST 2 sequence alignment is performed in blastp or blastn using the BLAST 2.0 algorithm to perform a Gapped BLAST search (BLAST 2.0) between the two sequences allowing for the introduction of gaps (deletions and insertions) in the resulting alignment. For purposes of clarity herein, a BLAST 2 sequence alignment is performed using the standard default parameters as follows.

For blastn, using 0 BLOSUM62 matrix:
Reward for match = 1
Penalty for mismatch = -2
Open gap (5) and extension gap (2) penalties
gap x_dropoff (50) expect (10) word size (11) filter (on)

For blastp, using 0 BLOSUM62 matrix:
Open gap (11) and extension gap (1) penalties
gap x_dropoff(50) expect (10) word size (3) filter (on).

Embodiments of the invention also encompass polypeptides having a lower degree of identity but having sufficient similarity so as to perform one or more of the same functions performed by a polypeptide of the invention. A variant polypeptide can differ in amino acid sequence by one or more substitutions, deletions, insertions, inversions, fusions, and truncations or a combination of any of these. Further, variant polypeptides can be fully functional or can lack function in one or more activities.

Embodiments of the invention also include polypeptide fragments of the polypeptides of the invention. The invention also encompasses fragments of the variants of the polypeptides described herein. As used herein, a fragment comprises at least 6 contiguous amino acids and includes any fragment of a full-length Mer protein described herein, including the entire extracellular domain of Mer or any portion thereof that retains the ability to bind to a Mer ligand. Useful fragments include those that retain one or more of the biological activities of the polypeptide (*e.g.,* ligand binding and/or signal transduction capability) as well as fragments that can be used as an immunogen to generate polypeptide-specific antibodies. Fragments can be discrete (not fused to other amino acids or polypeptides) or can be within a larger polypeptide. Further, several fragments can be comprised within a single larger polypeptide. Therefore, fragments can include any size fragment between about 6 amino acids and 998 amino acids, including any fragment in between, in whole integer increments (*e.g.,* 7, 8, 9...67, 68, 69...278, 279, 280...amino acids).

Embodiments of the invention thus provide chimeric or fusion polypeptides. These comprise a polypeptide of the invention operatively linked to a heterologous protein or polypeptide having an amino acid sequence not substantially homologous to the polypeptide. "Operatively linked" indicates that the polypeptide and the heterologous protein (also called a fusion segment or fusion partner) are fused in-frame. The heterologous protein can be fused to the N-terminus or C-terminus of the polypeptide. A chimeric or fusion polypeptide can be produced by standard recombinant DNA techniques well known in the art. Preferred heterologous proteins according to the present invention include, but are not limited to, any proteins or peptides that can: enhance a protein's stability; provide other desirable biological activity; and/or assist with the purification of a protein (e.g., by affinity chromatography), or provide another protein function (*e.g.*, as in a chimeric protein). A suitable heterologous protein can be a domain of any size that has the desired function (*e.g.*, imparts increased stability, solubility, action or biological activity; simplifies purification of a protein; or provides the additional protein function). In one embodiment, a suitable heterologous protein with which a chimeric or fusion protein can be produced is an antibody fragment and particularly, the Fc portion of an immunoglobulin protein. Any fusion or chimera partner that enhances the stability or half-life of Mer *in vivo,* for example, is contemplated for use in the present invention.

As used herein, the phrase "Mer agonist" refers to any compound that is characterized by the ability to agonize (e.g., stimulate, induce, increase, enhance, or mimic) the biological activity of a naturally occurring Mer as described herein, and includes any Mer homologue, binding protein (e.g., an antibody), agent that interacts with Mer or mimics Mer, or any suitable product of drug/compound/peptide design or selection which is characterized by its ability to agonize (e.g., stimulate, induce, increase, enhance) the biological activity of a naturally occurring Mer protein in a manner similar to the natural agonist, Mer.

Similarly, the phrase, "Mer antagonist" refers to any compound which inhibits (e.g., antagonizes, reduces, decreases, blocks, reverses, or alters) the effect of an Mer agonist as described above. More particularly, a Mer antagonist is capable of acting in a manner relative to Mer activity, such that the biological activity of the natural agonist Mer, is decreased in a manner that is antagonistic (e.g., against, a reversal of, contrary to) to the natural action of Mer. Such antagonists can include, but are not limited to, a protein (*e.g.*, soluble Mer), peptide, or nucleic acid (including ribozymes, RNAi, aptamers, and antisense), antibodies and antigen binding fragments thereof, or product of drug/compound/peptide design or selection that provides the antagonistic effect.

Homologues of Mer, including peptide and non-peptide agonists and antagonists of Mer (analogues), can be products of drug design or selection and can be produced using various methods known in the art. Such homologues can be referred to as mimetics. A mimetic refers to any peptide or non-peptide compound that is able to mimic the biological action of a naturally occurring peptide, often because the mimetic has a basic structure that mimics the basic structure of the naturally occurring peptide and/or has the salient biological properties of the naturally occurring peptide. Mimetics can include, but are not limited to: peptides that have substantial modifications from the prototype such as no side chain similarity with the naturally occurring peptide (such modifications, for example, may decrease its susceptibility to degradation); anti-idiotypic and/or catalytic antibodies, or fragments thereof; non-proteinaceous portions of an isolated protein (e.g., carbohydrate structures); or synthetic or natural organic molecules, including nucleic acids and drugs identified through combinatorial chemistry, for example. Such mimetics can be designed, selected and/or otherwise identified using a variety of methods known in the art. Various methods of drug design, useful to design or select mimetics or other therapeutic compounds useful in the present invention are disclosed in Maulik et al., 1997, Molecular Biotechnology: Therapeutic Applications and Strategies, Wiley-Liss, Inc., which is incorporated herein by reference in its entirety.

Homologues can be produced using techniques known in the art for the production of proteins including, but not limited to, direct modifications to the isolated, naturally occurring protein, direct protein synthesis, or modifications to the nucleic acid sequence encoding the protein using, for example, classic or recombinant DNA techniques to effect random or targeted mutagenesis. For smaller peptides, chemical synthesis methods may be preferred. For example, such methods include well known chemical procedures, such as solution or solid-phase peptide synthesis, or semi-synthesis in solution beginning with protein fragments coupled through conventional solution methods. Such methods are well known in the art and may be found in general texts and articles in the area such as: Merrifield, 1997, Methods Enzymol. 289:3-13; Wade et al., 1993, Australas Biotechnol. 3(6):332-336; Wong et al., 1991, Experientia 47(11-12):1123-1129; Carey et al., 1991, Ciba Found Symp. 158:187-203; Plaue et al., 1990, Biologicals 18(3):147-157; Bodanszky, 1985, Int. J. Pept. Protein Res. 25(5):449-474; or H. Dugas and C. Penney, BIOORGANIC CHEMISTRY, (1981) at pages 54-92, all of which are incorporated herein by reference in their entirety. For example, peptides may be synthesized by solid-phase methodology utilizing a commercially available peptide synthesizer and synthesis cycles supplied by the manufacturer. One skilled in the art recognizes that the solid phase synthesis could also be accomplished using the FMOC strategy and a TFA/scavenger cleavage mixture.

The polypeptides of the invention can be purified to homogeneity. It is understood, however, that preparations in which the polypeptide is not purified to homogeneity are useful. The critical feature is that the preparation allows for the desired function of the polypeptide, even in the presence of considerable amounts of other components. Indeed, in some of the assay formats contemplated by the invention, the natural biological milieu may contain important other proteins that affect the normal activity of Mer and the soluble forms of Mer. Thus, the invention encompasses various degrees of purity. In one embodiment, the language "substantially free of cellular material" includes preparations of the polypeptide having less than about 30% (by dry weight) other proteins (*i.e.*, contaminating protein), less than about 20% other proteins, less than about 10% other proteins, or less than about 5% other proteins.

In one aspect of the present invention, the soluble form of the Mer receptor tyrosine kinase is a product of TACE-like metalloprotease cleavage near the transmembrane domain of the full-length Mer receptor that removes the intracellular kinase of Mer as well as much, if not all of the transmembrane domain of the protein.

In a preferred aspect of the present invention, Mer proteins, and especially soluble Mer proteins, are produced as a specific glycoform of Mer, and in one aspect, as a specific glycoform described herein, including a glycoform that is associated with a particular cell type or a glycoform that is selected to compete with a natural Mer receptor for binding to a Mer ligand. Glycosylation is a post-translational modification. Glycosylation of Mer proteins of the invention can be achieved by any suitable method. For example, a Mer glycoform corresponding to a particular cell type can be produced by recombinantly expressing the Mer protein (including soluble Mer proteins) in a host cell of the cell type that naturally produces the specified Mer glycoform. Alternatively, Mer homologues can be produced (*e.g.,* using recombinant technology) that have altered (mutated, modified) glycosylation sites, such that the desired glycosylation pattern and/or level is achieved. Glycosylation of a protein can also be achieved by glycosylating an isolated protein *in vitro,* after its production and isolation or secretion from a cell.

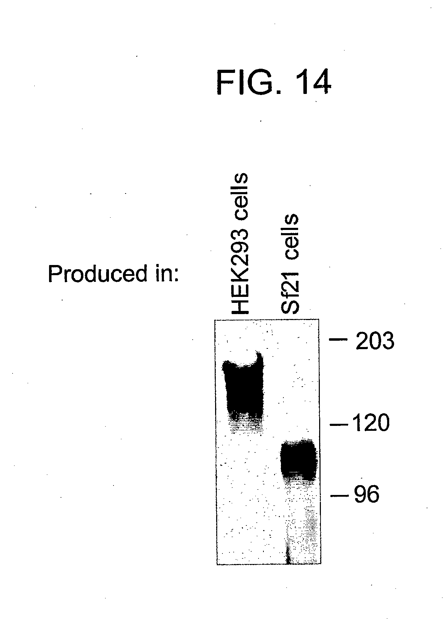

For example, preferred Mer proteins include Mer proteins that are wild-type Mer proteins that have been post-translationally glycosylated by a particular cell-type to provide a specific Mer glycoform. Preferred Mer proteins also include Mer protein variants (homologues) with glycosylation sites that differ from the wild-type protein such that Mer proteins that are less than or more than fully glycosylated as compared to a wild-type protein produced by monocytic cells are produced. Desirable Mer glycoforms to produce according to the invention, as discussed above, include, but are not limited to, Mer glycoforms having a molecular weight (due to altered glycosylation), of from about 195 to 210 kD, from about 165 kD to about 170 kD, less than about 160 kD, or between about 170 kD to about 195 kD, such as those glycoforms found in leukemia and lymphoma cells. Particularly desirable glycoforms to produce by modifying glycosylation sites in the protein include, but are not limited to, (1) a glycoform from about 165 kD to about 170 kD; (2) a glycoform from about 170 kD to about 190 kD; (3) a glycoform from about 135 kD to about 140 kD; and (4) a glycoform from about 190 kD to about 195kD. As discussed above, such glycoforms could be the result of mutations in the nucleic acid molecule resulting in reduced or altered glycosylation sites on the Mer receptor tyrosine kinase, mutations in other cellular proteins which result in faulty post-translational processing, mutations which produce truncated or variant extracellular domains, or the cell-type in which the Mer protein is expressed and post-translationally modified. For example, aberrantly glycosylated forms of the Mer receptor tyrosine kinase can be produced by expressing the protein in any of a variety of mammalian cell types known to produce varying glycosylation patterns, including the Jurkat human leukemia, U937 human monocyte, K562 human chronic myelogenous, and the HEK293 human kidney cell lines (Figure 14). For example, using the HEK293 cell line, a specific sMer glycoform has been produced (Figure 7C).

According to the present invention, an isolated Mer protein, including a biologically active homologue or fragment thereof, has at least one characteristic of biological activity of activity a wild-type, or naturally occurring Mer protein (which can vary depending on whether the homologue or fragment is an agonist, antagonist, or mimic of Mer, and the isoform of Mer). Biological activity of Mer and methods of determining the same have been described previously herein.

In one embodiment, a particularly preferred Mer protein is a Mer protein variant and/or a Mer glycoform that preferentially binds to one Mer ligand as compared to another Mer ligand. For example, known Mer ligands include Gas6 and Protein S. In one aspect of the invention, the Mer protein, preferably a soluble Mer protein, is either a variant of Mer or a glycoform of Mer, or both, that binds to Protein S with a statistically significantly higher binding affinity than the binding of the Mer protein to Gas6. In another aspect, the Mer protein, preferably a soluble Mer protein, is either a variant of Mer or a glycoform of Mer, or both, that binds to Gas6 with a statistically significantly higher binding affinity than the binding of the Mer protein to Protein S.

A particularly preferred Mer protein of the invention includes any soluble Mer protein and preferably any soluble form of any of the Mer glycoforms described herein, and most preferably, any soluble form of any of the aberrant glycosylated Mer proteins described herein, or any soluble form of a Mer protein that is selectively glycosylated, such as to provide a competitive inhibitor that preferentially binds to Mer ligand as compared to an endogenous Mer cellular receptor. In one embodiment of the present invention, a preferred soluble Mer protein is a Mer chimeric protein consisting of the Mer extracellular domain (*e.g.,* positions 1 to about 473 of SEQ ID NO:2), or a smaller portions of this extracellular domain that retain the ability to bind to at least one Mer ligand, fused to the Fc region of human IgG and expressed in different mammalian cell lines, such as HEK293, to yield specific glycoslyation forms of soluble Mer (Figure 14). Other glycoforms of soluble Mer can be made in a similar manner by expressing the Mer-Fc construct in other mammalian cell lines since cell types glycosylate Mer in a distinct manner (Figure 7C). Without being bound by theory, the present inventors believe that the different glycoforms of soluble Mer are believed to have different binding affinities to different Mer ligands, including Protein S and Gas6. The Mer extracellular domain can include any extracellular fragment of Mer that binds to a Mer ligand. Such a soluble Mer protein is glycosylated to form a fully glycosylated sMer, or any of the less-glycosylated Mer proteins as described herein.

### Nucleic Acid Molecules Encoding sMer and Mer Variants

Another embodiment of the invention relates to an isolated nucleic acid molecule, or complement thereof, encoding a Mer protein (full-length wild-type) or any homologue thereof (including variants and fragments), and can include Mer proteins in which the resulting amino acid sequence of the encoded Mer protein is altered (e.g., modified by substitution, deletion and/or insertion) to add or eliminate those amino acids that form glycosylation sites in the encoded protein, such that aberrantly glycosylated Mer glycoforms can be produced. In particular, one aspect of the present invention relates to nucleic acid molecules encoding Mer proteins with glycosylation sites that differ from the wild-type protein, so that Mer glycoforms including, but not limited to, Mer glycoforms having a molecular weight (due to altered glycosylation) of less than about 160 kD or between about 170 kD to about 195 kD, such as those glycoforms found in leukemia and lymphoma cells. Particularly desirable glycoforms to produce by modifying glycosylation sites in the protein include, but are not limited to, (1) a glycoform from about 165 kD to about 170 kD; (2) a glycoform from about 170 kD to about 190 kD; (3) a glycoform from about 135 kD to about 140 kD; and (4) a glycoform from about 190 kD to about 195kD. Such glycoforms produced by nucleic acid molecules of the invention could be the result of mutations in the nucleic acid molecule resulting in reduced or altered glycosylation sites on the Mer receptor tyrosine kinase, including mutations which produce truncated or variant extracellular domains. Such mutations resulting in aberrant glycoforms can be introduced into the molecule using standard molecular biology techniques such as site directed mutagenesis. Site directed mutagenesis could be targeted against 13 potential NH₂ linked glycoslyation sites in the Mer extracellular domain, which are identified by the amino acid sequence: NXS/T.

The present invention also relates to an isolated nucleic acid molecule, or complement thereof, encoding a soluble form of the extracellular Mer receptor tyrosine kinase as described previously. This sMer can also be engineered to have glycosylation patterns identical to the aberrant forms using techniques as described above. As discussed above, it is one aspect of the present invention to provide particular glycoforms of the sMer that have different binding affinities for Mer ligands, including Protein S and Gas6 and indeed, without being bound by theory, the present inventors believe that different glycoforms of Mer have different ligand binding affinities. Assays for measuring binding affinities are well-known in the art. In one embodiment, a BIAcore machine can be used to determine the binding constant of a complex between the target protein (*e.g*., a Mer glycoform) and a natural ligand. For example, the Mer glycoform can be immobilized on a substrate. A natural or synthetic ligand is contacted with the substrate to form a complex. The dissociation constant for the complex can be determined by monitoring changes in the refractive index with respect to time as buffer is passed over the chip (O'Shannessy et al. Anal. Biochem. 212:457-468 (1993); Schuster et al., Nature 365:343-347 (1993)). Contacting a second compound (*e.g*., a different ligand or a different soluble Mer glycoform) at various concentrations at the same time as the first ligand and monitoring the response function (*e.g*., the change in the refractive index with respect to time) allows the complex dissociation constant to be determined in the presence of the second compound and indicates whether the second compound is an inhibitor of the complex. Other suitable assays for measuring the binding of a soluble receptor to a ligand include, but are not limited to, Western blot, immunoblot, enzyme-linked immunosorbant assay (ELISA), radioimmunoassay (RIA), immunoprecipitation, surface plasmon resonance, chemiluminescence, fluorescent polarization, phosphorescence, immunohistochemical analysis, matrix-assisted laser desorption/ionization time-of-flight (MALDI-TOF) mass spectrometry, microcytometry, microarray, microscopy, fluorescence activated cell sorting (FACS), and flow cytometry.

The isolated nucleic acid molecules of the present invention can be RNA, for example, mRNA, or DNA, such as cDNA and genomic DNA. DNA molecules can be double-stranded or single-stranded; single stranded RNA or DNA can be either the coding, or sense, strand or the non-coding, or antisense, strand. The nucleic acid molecule can include all or a portion of the coding sequence of the gene and can further comprise additional non-coding sequences such as introns and non-coding 3' and 5' sequences (including regulatory sequences, for example). The nucleic acid can also comprise the sequences that would code for the aberrantly glycosylated glycoforms. Additionally, the nucleic acid molecule can be fused to a marker sequence, for example, a sequence that encodes a polypeptide to assist in isolation or purification of the polypeptide.

An "isolated" nucleic acid molecule, as used herein, is one that is separated from nucleic acids that normally flank the gene or nucleotide sequence (as in genomic sequences) and/or has been completely or partially purified from other transcribed sequences (*e.g*., as in an RNA library). For example, an isolated nucleic acid of the invention may be substantially isolated with respect to the complex cellular milieu in which it naturally occurs, or culture medium when produced by recombinant techniques, or chemical precursors or other chemicals when chemically synthesized. In some instances, the isolated material will form part of a composition (for example, a crude extract containing other substances), buffer system or reagent mix. In other circumstances, the material may be purified to essential homogeneity, for example as determined by PAGE or column chromatography such as HPLC.

The nucleic acid molecule can be fused to other coding or regulatory sequences and still be considered isolated. Thus, recombinant DNA contained in a vector is included in the definition of "isolated" as used herein. Also, isolated nucleic acid molecules include recombinant DNA molecules in heterologous host cells, as well as partially or substantially purified DNA molecules in solution. "Isolated" nucleic acid molecules also encompass *in vivo* and *in vitro* RNA transcripts of the DNA molecules of the present invention. An isolated nucleic acid molecule or nucleotide sequence can include a nucleic acid molecule or nucleotide sequence that is synthesized chemically or by recombinant means. Therefore, recombinant DNA contained in a vector is included in the definition of "isolated" as used herein. Also, isolated nucleotide sequences include partially or substantially purified DNA molecules in solution. *In vivo* and *in vitro* RNA transcripts of the DNA molecules of the present invention are also encompassed by "isolated" nucleotide sequences. Such isolated nucleotide sequences are useful in the manufacture of the encoded polypeptide, as probes for isolating homologous sequences (e.g., from other mammalian species), for gene mapping (*e.g*., by *in situ* hybridization with chromosomes), or for detecting expression of the gene in tissue (e.g., human tissue), such as by Northern blot analysis.

The present invention also pertains to variant nucleic acid molecules that are not necessarily found in nature but which encode novel aberrant Mer glycoforms (*e.g*., by altering one or more glycosylation sites on the encoded protein) and/or a soluble form of the extracellular Mer receptor tyrosine kinase. Thus, for example, DNA molecules which comprise a sequence that is different from the naturally-occurring nucleotide sequence but which codes for aberrant Mer glycoforms or a soluble form of the extracellular Mer receptor tyrosine kinase polypeptide of the present invention are also the subject of this invention. The invention also encompasses nucleotide sequences encoding portions (fragments), or encoding variant polypeptides such as analogues or derivatives of novel Mer glycoforms or a soluble form of the Mer receptor tyrosine kinase. Such variants can be naturally occurring, such as in the case of allelic variation or single nucleotide polymorphisms, or non-naturally-occurring, such as those induced by various mutagens and mutagenic processes. Intended variations include, but are not limited to, addition, deletion and substitution of one or more nucleotides that can result in conservative or non-conservative amino acid changes, including additions and deletions.

Other alterations of the nucleic acid molecules of the invention can include, for example, labeling, methylation, internucleotide modifications such as uncharged linkages (*e.g*., methyl phosphonates, phosphotriesters, phosphoamidates, carbamates), charged linkages (*e.g*., phosphorothioates, phosphorodithioates), pendent moieties *(e.g.,* polypeptides), intercalators (*e.g*., acridine, psoralen), chelators, alkylators, and modified linkages (*e.g*., alpha anomeric nucleic acids). Also included are synthetic molecules that mimic nucleic acid molecules in the ability to bind to designated sequences via hydrogen bonding and other chemical interactions. Such molecules include, for example, those in which peptide linkages substitute for phosphate linkages in the backbone of the molecule.

The invention also pertains to nucleic acid molecules that hybridize under high stringency hybridization conditions, such as for selective hybridization, to a nucleotide sequence described herein (*e.g*., nucleic acid molecules which specifically hybridize to a nucleotide sequence encoding polypeptides described herein, and, optionally, have an activity of the polypeptide). In one embodiment, the invention includes variants described herein which hybridize under high stringency hybridization conditions (*e.g*., for selective hybridization) to a nucleotide sequence encoding novel Mer glycoforms (*e.g*., where one or more glycosylation sites on the protein is disrupted), including novel aberrant Mer glycoforms described herein, and/or a soluble form of the extracellular Mer receptor tyrosine kinase or the complements thereof.

The present invention also provides isolated nucleic acid molecules that contain a fragment or portion that hybridizes under highly stringent conditions to a nucleic acid encoding an aberrant Mer glycoform or a soluble form of the extracellular Mer receptor tyrosine kinase or the complements thereof. "Stringency conditions" for hybridization is a term of art which refers to the incubation and wash conditions, e.g., conditions of temperature and buffer concentration, which permit hybridization of a particular nucleic acid to a second nucleic acid; the first nucleic acid may be perfectly (i.e., 100%) complementary to the second, or the first and second may share some degree of complementarity which is less than perfect (e.g., 70%, 75%, 85%, 95%). For example, certain high stringency conditions can be used which distinguish perfectly complementary nucleic acids from those of less complementarity. "High stringency conditions", "moderate stringency conditions" and "low stringency conditions" for nucleic acid hybridizations are explained on pages 2.10.1-2.10.16 and pages 6.3.1-6.3.6 in Current Protocols in Molecular Biology (Ausubel, F. M. et al., "Current Protocols in Molecular Biology", John Wiley & Sons, (1998), the entire teachings of which are incorporated by reference herein). Typically, conditions are used such that sequences at least about 60%, at least about 70%, at least about 80%, at least about 90% or at least about 95% or more identical to each other remain hybridized to one another. By varying hybridization conditions from a level of stringency at which no hybridization occurs to a level at which hybridization is first observed, conditions which will allow a given sequence to hybridize (*e.g*., selectively) with the most similar sequences in the sample can be determined.

More particularly, moderate stringency hybridization and washing conditions, as referred to herein, refer to conditions which permit isolation of nucleic acid molecules having at least about 70% nucleic acid sequence identity with the nucleic acid molecule being used to probe in the hybridization reaction (i.e., conditions permitting about 30% or less mismatch of nucleotides). High stringency hybridization and washing conditions, as referred to herein, refer to conditions which permit isolation of nucleic acid molecules having at least about 80% nucleic acid sequence identity with the nucleic acid molecule being used to probe in the hybridization reaction (i.e., conditions permitting about 20% or less mismatch of nucleotides). Very high stringency hybridization and washing conditions, as referred to herein, refer to conditions which permit isolation of nucleic acid molecules having at least about 90% nucleic acid sequence identity with the nucleic acid molecule being used to probe in the hybridization reaction (i.e., conditions permitting about 10% or less mismatch of nucleotides). As discussed above, one of skill in the art can use the formulae in Meinkoth et al., *ibid.* to calculate the appropriate hybridization and wash conditions to achieve these particular levels of nucleotide mismatch. Such conditions will vary, depending on whether DNA:RNA or DNA:DNA hybrids are being formed. Calculated melting temperatures for DNA:DNA hybrids are 10°C less than for DNA:RNA hybrids. In particular embodiments, stringent hybridization conditions for DNA:DNA hybrids include hybridization at an ionic strength of 6X SSC (0.9 M Na⁺) at a temperature of between about 20°C and about 35°C (lower stringency), more preferably, between about 28°C and about 40°C (more stringent), and even more preferably, between about 35°C and about 45°C (even more stringent), with appropriate wash conditions. In particular embodiments, stringent hybridization conditions for DNA:RNA hybrids include hybridization at an ionic strength of 6X SSC (0.9 M Na⁺) at a temperature of between about 30°C and about 45°C, more preferably, between about 38°C and about 50°C, and even more preferably, between about 45°C and about 55°C, with similarly stringent wash conditions. These values are based on calculations of a melting temperature for molecules larger than about 100 nucleotides, 0% formamide and a G + C content of about 40%. Alternatively, Tₘ can be calculated empirically as set forth in Sambrook et al., *supra,* pages 9.31 to 9.62. In general, the wash conditions should be as stringent as possible, and should be appropriate for the chosen hybridization conditions. For example, hybridization conditions can include a combination of salt and temperature conditions that are approximately 20-25°C below the calculated Tₘ of a particular hybrid, and wash conditions typically include a combination of salt and temperature conditions that are approximately 12-20°C below the calculated Tₘ of the particular hybrid. One example of hybridization conditions suitable for use with DNA:DNA hybrids includes a 2-24 hour hybridization in 6X SSC (50% formamide) at about 42°C, followed by washing steps that include one or more washes at room temperature in about 2X SSC, followed by additional washes at higher temperatures and lower ionic strength (e.g., at least one wash as about 37°C in about 0.1X-0.5X SSC, followed by at least one wash at about 68°C in about 0.1X-0.5X SSC).

In a related aspect of the invention, the nucleic acid fragments of the invention are used as probes or primers in assays such as those described herein. "Probes" or "primers" are oligonucleotides that hybridize in a base-specific manner to a complementary strand of nucleic acid molecules. By "base specific manner" is meant that the two sequences must have a degree of nucleotide complementarity sufficient for the primer or probe to hybridize. Accordingly, the primer or probe sequence is not required to be perfectly complementary to the sequence of the template. Non-complementary bases or modified bases can be interspersed into the primer or probe, provided that base substitutions do not substantially inhibit hybridization. The nucleic acid template may also include "non-specific priming sequences" or "nonspecific sequences" to which the primer or probe has varying degrees of complementarity. Such probes and primers include polypeptide nucleic acids, as described in Nielsen et al., Science, 254, 1497-1500 (1991). Typically, a probe or primer comprises a region of nucleotide sequence that hybridizes to at least about 15, typically about 20-25, and more typically about 40, 50, 75, 100, 150, 200, or more, consecutive nucleotides of a nucleic acid molecule comprising a nucleotide sequence encoding a Mer protein, including an aberrant Mer glycoform (*e.g*., a Mer glycoform in which glycosylation sites have been modified) or a soluble form of the extracellular Mer receptor tyrosine kinase or the complements thereof.

The nucleic acid molecules of the invention such as those described above can be identified and isolated using standard molecular biology techniques and the sequence information provided herein. For example, nucleic acid molecules can be amplified and isolated by the polymerase chain reaction using synthetic oligonucleotide primers designed based on a nucleotide sequence encoding an aberrant Mer glycoform or a soluble form of the extracellular Mer receptor tyrosine kinase or the complements thereof. See generally PCR Technology: Principles and Applications for DNA Amplification (ed. H. A. Erlich, Freeman Press, NY, N.Y., 1992); PCR Protocols. A Guide to Methods and Applications (Eds. Innis, et al., Academic Press, San Diego, Calif., 1990); Mattila et al., Nucleic Acids Res., 19:4967 (1991); Eckert et al., PCR Methods and Applications, 1:17 (1991); PCR (eds. McPherson et al., IRL Press, Oxford); and U.S. Patent No. 4,683,202. The nucleic acid molecules can be amplified using cDNA, mRNA or genomic DNA as a template, cloned into an appropriate vector and characterized by DNA sequence analysis. Soluble forms of Mer consisting essentially of the extracellular form of Mer and lacking the kinase region of the molecules can be discerned from an inspection of U.S. Patent No. 5,585,689 taken with the instant disclosure.

Other suitable amplification methods include the ligase chain reaction (LCR) (see Wu and Wallace, Genomics, 4:560 (1989), Landegren et al., Science, 241:1077 (1988)), transcription amplification (Kwoh et al., Pro. Natl. Acad. Sci. USA, 86:1173 (1989)), and self-sustained sequence replication (Guatelli et al., Proc. Nat. Acad. Sci. USA, 87:1874 (1990)) and nucleic acid based sequence amplification (NASBA).

The amplified DNA can be labeled (*e.g*., with radiolabel or other reporter molecule) and used as a probe for screening a cDNA library derived from human cells, mRNA in zap express, ZIPLOX or other suitable vector. Corresponding clones can be isolated, DNA can obtained following *in vivo* excision, and the cloned insert can be sequenced in either or both orientations by art recognized methods to identify the correct reading frame encoding a polypeptide of the appropriate molecular weight. For example, the direct analysis of the nucleotide sequence of nucleic acid molecules of the present invention can be accomplished using well-known methods that are commercially available. See, for example, Sambrook et al., Molecular Cloning, A Laboratory Manual (2nd Ed., CSHP, New York 1989); Zyskind et al., Recombinant DNA Laboratory Manual, (Acad. Press, 1988). Using these or similar methods, the polypeptide and the DNA encoding the polypeptide can be isolated, sequenced and further characterized.

In general, the isolated nucleic acid sequences of the invention can be used as molecular weight markers on Southern gels, and as chromosome markers that are labeled to map related gene positions. The nucleic acid sequences can also be used to compare with endogenous DNA sequences in patients to identify genetic disorders and as probes, such as to hybridize and discover related DNA sequences or to subtract out known sequences from a sample. The nucleic acid sequences can further be used to derive primers for genetic fingerprinting, to raise anti-polypeptide antibodies using DNA immunization techniques, and as an antigen to raise anti-DNA antibodies or elicit immune responses. Additionally, and preferably, the nucleotide sequences of the invention can be used to identify and express recombinant polypeptides for analysis, for characterization, for diagnostic use, for therapeutic use, or as markers for tissues in which the corresponding polypeptide is expressed, either constitutively, during tissue differentiation, or in diseased states. The nucleic acid sequences can additionally be used as reagents in the screening and/or diagnostic assays described herein, and can also be included as components of kits (e.g., reagent kits) for use in the screening and/or diagnostic assays described herein.

Such nucleic acid sequences can be incorporated into host cells and expression vectors that are well known in the art. According to the present invention, a recombinant nucleic acid molecule includes at least one isolated nucleic acid molecule of the present invention that is linked to a heterologous nucleic acid sequence. Such a heterologous nucleic acid sequence is typically a recombinant nucleic acid vector (e.g., a recombinant vector) which is suitable for cloning, sequencing, and/or otherwise manipulating the nucleic acid molecule, such as by expressing and/or delivering the nucleic acid molecule into a host cell to form a recombinant cell. Such a vector contains heterologous nucleic acid sequences, that is nucleic acid sequences that are not naturally found adjacent to nucleic acid molecules of the present invention, although the vector can also contain regulatory nucleic acid sequences (e.g., promoters, untranslated regions) which are naturally found adjacent to nucleic acid molecules of the present invention. The vector can be either RNA or DNA, either prokaryotic or eukaryotic, and typically is a virus or a plasmid. The vector can be maintained as an extrachromosomal element (e.g., a plasmid) or it can be integrated into the chromosome. The entire vector can remain in place within a host cell, or under certain conditions, the plasmid DNA can be deleted, leaving behind the nucleic acid molecule of the present invention. The integrated nucleic acid molecule can be under chromosomal promoter control, under native or plasmid promoter control, or under a combination of several promoter controls. Single or multiple copies of the nucleic acid molecule can be integrated into the chromosome. As used herein, the phrase "recombinant nucleic acid molecule" is used primarily to refer to a recombinant vector into which has been ligated the nucleic acid sequence to be cloned, manipulated, transformed into the host cell (i.e., the insert).

The nucleic acid sequence encoding the protein to be produced is inserted into the vector in a manner that operatively links the nucleic acid sequence to regulatory sequences in the vector (e.g., expression control sequences) which enable the transcription and translation of the nucleic acid sequence when the recombinant molecule is introduced into a host cell. According to the present invention, the phrase "operatively linked" refers to linking a nucleic acid molecule to an expression control sequence (e.g., a transcription control sequence and/or a translation control sequence) in a manner such that the molecule can be expressed when transfected (i.e., transformed, transduced, transfected, conjugated or conduced) into a host cell. Transcription control sequences are sequences that control the initiation, elongation, or termination of transcription. Particularly important transcription control sequences are those that control transcription initiation, such as promoter, enhancer, operator and repressor sequences. Suitable transcription control sequences include any transcription control sequence that can function in a host cell into which the recombinant nucleic acid molecule is to be introduced.

Recombinant molecules of the present invention, which can be either DNA or RNA, can also contain additional regulatory sequences, such as translation regulatory sequences, origins of replication, and other regulatory sequences that are compatible with the recombinant cell. In one embodiment, a recombinant molecule of the present invention, including those which are integrated into the host cell chromosome, also contains secretory signals (i.e., signal segment nucleic acid sequences) to enable an expressed protein to be secreted from the cell that produces the protein. Suitable signal segments include a signal segment that is naturally associated with a protein of the present invention or any heterologous signal segment capable of directing the secretion of a protein according to the present invention.

One or more recombinant molecules of the present invention can be used to produce an encoded product of the present invention. In one embodiment, an encoded product is produced by expressing a nucleic acid molecule as described herein under conditions effective to produce the protein. A preferred method to produce an encoded protein is by transfecting a host cell with one or more recombinant molecules to form a recombinant cell. Suitable host cells to transfect include, but are not limited to, any bacterial, fungal (e.g., yeast), insect, plant or animal cell that can be transfected. Host cells can be either untransfected cells or cells that are already transfected with at least one nucleic acid molecule.

According to the present invention, the term "transfection" is used to refer to any method by which an exogenous nucleic acid molecule (i.e., a recombinant nucleic acid molecule) can be inserted into the cell. The term "transformation" can be used interchangeably with the term "transfection" when such term is used to refer to the introduction of nucleic acid molecules into microbial cells, such as bacteria and yeast. In microbial systems, the term "transformation" is used to describe an inherited change due to the acquisition of exogenous nucleic acids by the microorganism and is essentially synonymous with the term "transfection". However, in animal cells, transformation has acquired a second meaning which can refer to changes in the growth properties of cells in culture after they become cancerous, for example. Therefore, to avoid confusion, the term "transfection" is preferably used with regard to the introduction of exogenous nucleic acids into animal cells, and the term "transfection" will be used herein to generally encompass both transfection of animal cells and transformation of microbial cells, to the extent that the terms pertain to the introduction of exogenous nucleic acids into a cell. Therefore, transfection techniques include, but are not limited to, transformation, electroporation, microinjection, lipofection, adsorption, infection and protoplast fusion.

### Compositions

Some embodiments of the present invention include a composition or formulation for diagnostic, screening or therapeutic purposes. Such compositions or formulations can include any antibodies against Mer glycoforms as described herein, any Mer polypeptides (*e.g*., soluble forms of Mer and/or any Mer glycoforms described herein), or any nucleic acid molecules encoding Mer and particularly, any of the soluble Mer proteins or Mer glycoforms of the invention). In one aspect, the agents described above can be formulated with a phannaceutically acceptable carrier. The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Common suitable pharmaceutical carriers are described in "Remington's Pharmaceutical Sciences" by E.W. Martin.

According to the invention, the pharmaceutical composition of the invention can be introduced parenterally, transmucosally, *e.g*., orally (per os), nasally or transdermally. Parental routes include intravenous, intra-arteriole, intramuscular, intradermal, subcutaneous, intraperitoneal, intraventricular and intracranial administration. Preferably, administration is directly into the cerebrospinal fluid, *e.g*., by a spinal tap.

In another embodiment, the therapeutic compound can be delivered in a vesicle, in particular a liposome (*see* Langer, Science 249:1527-1533 (1990); Treat et al., in Liposomes in the Therapy of Infectious Disease and Cancer, Lopez-Berestein and Fidler (eds.), Liss: New York, pp. 353-365 (1989). To reduce its systemic side effects, this may be a preferred method for introducing the compound.

In yet another embodiment, the therapeutic compound can be delivered in a controlled release system. For example, a polypeptide may be administered using intravenous infusion with a continuous pump, in a polymer matrix such as polylactic/glutamic acid (PLGA), a pellet containing a mixture of cholesterol and the anti-amyloid peptide antibody compound (U.S. Pat. No. 5,554,601) implanted subcutaneously, an implantable osmotic pump, a transdermal patch, liposomes, or other modes of administration.

The pharmaceutical compositions of the invention may further comprise a therapeutically effective amount of the monoclonal antibodies of the invention, or the soluble extracellular form of Mer and/or Mer glycoform, preferably in respective proportions such as to provide a synergistic effect in the said prevention or treatment. A therapeutically effective amount of an pharmaceutical composition of the invention relates generally to the amount needed to achieve a therapeutic objective.

### Methods of the Invention

The present invention also includes a variety of diagnostic, prognostic and therapeutic methods that particularly make use of the Mer glycoforms discovered by the present inventors, as well as the agents disclosed herein, including antibodies of the present invention, Mer glycoforms, soluble Mer proteins (particularly soluble Mer glycoforms), and nucleic acid molecules encoding soluble Mer proteins and Mer proteins having modified glycosylation sites.

### Diagnostic Methods of the Invention

Accordingly, one embodiment of the present invention provides a method of diagnosing cancers, such as leukemias or lymphomas in an individual, comprising detecting specific glycoforms of the Mer transmembrane receptor tyrosine kinase in a patient sample, wherein the presence of a specific glycoforms of the Mer transmembrane receptor tyrosine kinase that is associated with a particular cancer is indicative of the cancer, or wherein the presence of certain specific glycoforms are prognostic markers for the tractability of various therapeutic methods. Diagnostic assays of the present invention are designed to assess aberrant Mer glycoforms. In one embodiment, the assays are used in the context of a biological sample (*e.g*., blood, serum, cells, tissue) to thereby determine whether an individual is afflicted with a cancer, and in a preferred embodiment, leukemia or lymphoma, and the severity of the disease.

The method of the invention includes the step of detecting an aberrant glycoform(s) of Mer transmembrane receptor tyrosine kinase in an individual, wherein the presence of the aberrant glycoform(s) of the Mer transmembrane receptor tyrosine kinase is indicative of the presence of a cancer cell that expresses the aberrant glycoform of Mer in the individual.

According to the present invention, the phrase "tumorigenicity" refers primarily to the tumor status of a cell or cells (i.e., the extent of neoplastic transformation of a cell, the malignancy of a cell, or the propensity for a cell to form a tumor and/or have characteristics of a tumor), which is a change of a cell or population of cells from a normal to malignant state. Tumorigenicity indicates that tumor cells are present in a sample, and/or that the transformation of cells from normal to tumor cells is in progress, as may be confirmed by any standard of measurement of tumor development. The change typically involves cellular proliferation at a rate which is more rapid than the growth observed for normal cells under the same conditions, and which is typically characterized by one or more of the following traits: continued growth even after the instigating factor (e.g., carcinogen, virus) is no longer present; a lack of structural organization and/or coordination with normal tissue, and typically, a formation of a mass of tissue, or tumor. A tumor, therefore, is most generally described as a proliferation of cells (e.g., a neoplasia, a growth, a polyp) resulting from neoplastic growth and is most typically a malignant tumor. In the case of a neoplastic transformation, a neoplasia is malignant or is predisposed to become malignant. Malignant tumors are typically characterized as being anaplastic (primitive cellular growth characterized by a lack of differentiation), invasive (moves into and destroys surrounding tissues) and/or metastatic (spreads to other parts of the body). As used herein, reference to a "potential for neoplastic transformation", "potential for tumorigenicity" or a "potential for tumor cell growth" refers to an expectation or likelihood that, at some point in the future, a cell or population of cells will display characteristics of neoplastic transformation, including rapid cellular proliferation characterized by anaplastic, invasive and/or metastatic growth. In the present invention, the expectation or likelihood of tumorigenicity or neoplastic transformation and particularly malignant tumor cell growth (i.e., a positive diagnosis of tumorigenicity) is determined based on a detection of aberrant expression of a specific Mer glycoform(s) in a cell.

This method of the present invention has several different uses. First, the method can be used to diagnose the presence or absence of tumor cells of a particular type, in a subject. The subject can be an individual who is suspected of having a tumor, or an individual who is presumed to be healthy, but who is undergoing a routine or diagnostic screening for the presence of a tumor (cancer). The subject can also be an individual who has previously been diagnosed with cancer and treated, and who is now under surveillance for recurring tumor growth. The terms "diagnose", "diagnosis", "diagnosing" and variants thereof refer to the identification of a disease or condition on the basis of its signs and symptoms. As used herein, a "positive diagnosis" indicates that the disease or condition, or a potential for developing the disease or condition, has been identified. In contrast, a "negative diagnosis" indicates that the disease or condition, or a potential for developing the disease or condition, has not been identified. Therefore, in the present invention, a positive diagnosis (i.e., a positive assessment) of tumor growth or tumorigenicity (i.e., malignant or inappropriate cell growth or neoplastic transformation), or the potential therefore, means that the indicators (e.g., signs, symptoms) of tumor presence and/or growth according to the present invention (i.e., expression of a particular Mer glycoform) has been identified in the sample obtained from the subject. Such a subject can then be prescribed treatment to reduce or eliminate the tumor growth. Similarly, a negative diagnosis (i.e., a negative assessment) for tumor growth or a potential therefore or the absence of tumor cells means that the indicators of tumor growth or tumor presence or a likelihood of developing tumors as described herein (i.e., no detection of Mer or a particular Mer glycoform) have not been identified in the sample obtained from the subject. In this instance, the subject is typically not prescribed any treatment, but may be reevaluated at one or more timepoints in the future to again assess tumor growth.

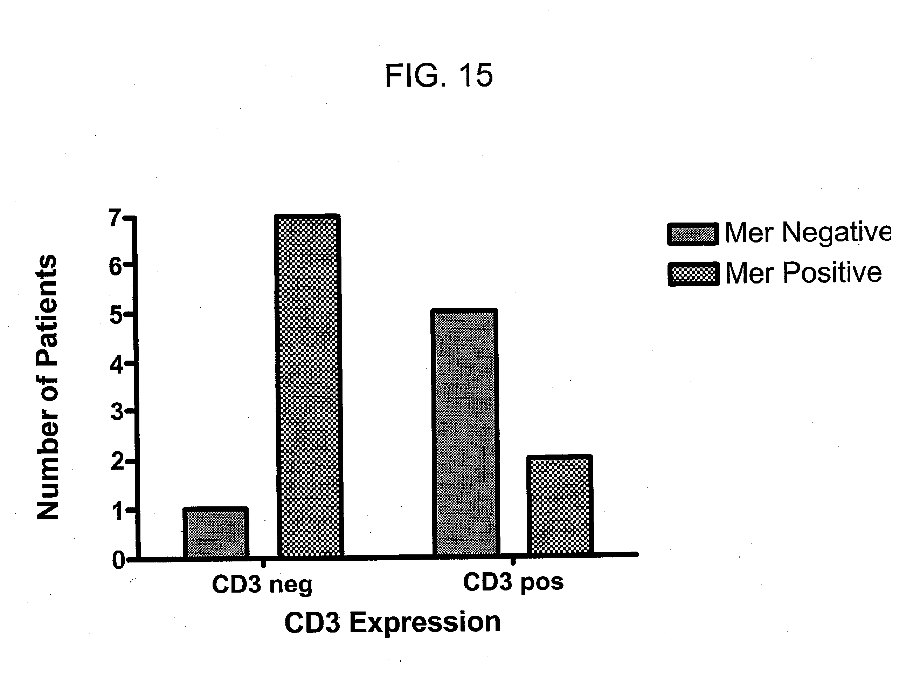

In another embodiment of the invention, Mer glycoform expression has prognostic significance for cancer patients, and particularly, for leukemia patients. For example, the present inventors have discovered that patients diagnosed with T cell acute lymphoblastic leukemia (ALL) having high levels of Mer expression also had lymphoblasts which were negative for CD3 expression, suggesting that the leukemia arose from an immature stage of thymocyte differentiation (Figure 15). CD3 negative *(i.e.,* immature stage) T cell leukemias have a decreased event free survival, unless chemotherapy is intensified. The association of Mer with the CD3 negative T cell ALL subset suggests a prognostic significance for Mer expression in T cell ALL. Therefore, in addition to detecting Mer glycoforms, the type and level of Mer glycoform expression can be used to determine a prognosis for certain cancer patients.

Preferred cancers to diagnose using the methods of the present invention include any cancers wherein tumor cells have been correlated with expression of Mer and specifically, an aberrant glycoform of Mer. Preferred cancers to diagnose using the method of the invention include, but are not limited to, leukemia and lymphoma, and more particularly, lymphoblastic leukemias, including acute lymphoblastic leukemia (ALL), and myelogenous leukemia. In particular, detection of a Mer glycoform having a molecular weight of between about 170 kD and about 190 kD and/or from about 135 kD to about 140 kD indicates a positive diagnosis of lymphoblastic leukemia. Detection of a Mer glycoform having a molecular weight of between about 190 kD to about 195kD indicates a positive diagnosis of myelogenous leukemia. Furthermore, detection of high levels of ectoptic Mer RNA transcript in lymphoblasts by PCR or quantitative PCR or detection of a Mer glycoform in lymphoblasts having a molecular weight of between about 170 kD and about 190 kD by Western blot or flow cytometry in combination with lack of surface CD3, indicates a positive diagnosis for lymphoblastic leukemia that has a decreased event free survival, unless chemotherapy is intensified.

The method of the present invention includes detecting Mer glycoform expression in a test sample from a subject. According to the present invention, the term "test sample" can be used generally to refer to a sample of any type which contains cells or products that have been secreted from cells to be evaluated by the present method, including but not limited to, a sample of isolated cells, a tissue sample and/or a bodily fluid sample. According to the present invention, a sample of isolated cells is a specimen of cells, typically in suspension or separated from connective tissue which may have connected the cells within a tissue *in vivo,* which have been collected from an organ, tissue or fluid by any suitable method which results in the collection of a suitable number of cells for evaluation by the method of the present invention. A cell sample can also be processed to obtain a soluble product therefrom, such as a supernatant or lysate from the cell that might contain a soluble Mer protein. The cells in the cell sample are not necessarily of the same type, although purification methods can be used to enrich for the type of cells that are preferably evaluated. Cells can be obtained, for example, by scraping of a tissue, processing of a tissue sample to release individual cells, or isolation from a bodily fluid. A tissue sample, although similar to a sample of isolated cells, is defined herein as a section of an organ or tissue of the body which typically includes several cell types and/or cytoskeletal structure which holds the cells together. One of skill in the art will appreciate that the term "tissue sample" may be used, in some instances, interchangeably with a "cell sample", although it is preferably used to designate a more complex structure than a cell sample. A tissue sample can be obtained by a biopsy, for example, including by cutting, slicing, or a punch. A bodily fluid sample, like the tissue sample, contains the cells to be evaluated for Mer glycoform expression, and is a fluid obtained by any method suitable for the particular bodily fluid to be sampled. Bodily fluids suitable for sampling include, but are not limited to, blood, mucous, seminal fluid, saliva, breast milk, bile and urine. In general, the sample type (i.e., cell, tissue or bodily fluid) is selected based on the accessibility and structure of the organ or tissue to be evaluated for tumor cell growth and/or on what type of cancer is to be evaluated.

Once a sample is obtained from the subject, the sample is evaluated for detection of Mer glycoform expression in the cells of the sample. The phrase "Mer expression" (including as it applies to either form of Mer) can generally refer to Mer mRNA transcription or Mer protein translation. Detection of Mer transcription is useful to detect whether or not a given cell expresses Mer, but is not useful for detecting Mer glycoforms unless the Mer glycoforms are due to a mutation in the nucleic acid sequence encoding Mer that results in a modification of glycosylation sites in the expressed protein. However, detection of other variations in Mer may be combined with the detection of Mer glycoforms to enhance the diagnostic potential of the method of the present invention.

Accordingly, methods suitable for detecting Mer transcription include any suitable method for detecting and/or measuring mRNA levels from a cell or cell extract. Such methods include, but are not limited to: polymerase chain reaction (PCR), reverse transcriptase PCR (RT-PCR), *in situ* hybridization, Northern blot, sequence analysis, gene microarray analysis (gene chip analysis) and detection of a reporter gene. Such methods for detection of transcription levels are well known in the art, and many of such methods are described in detail in the attached examples, in Sambrook et al., Molecular Cloning: A Laboratory Manual, Cold Spring Harbor Labs Press, 1989 and/or in Glick et al., Molecular Biotechnology: Principles and Applications of Recombinant DNA, ASM Press, 1998; Sambrook et al., *ibid.,* and Glick et al., *ibid.* are incorporated by reference herein in their entireties.

Mer glycoform expression is more typically identified by detection of Mer translation (i.e., detection of Mer protein in a sample). Methods suitable for the detection of Mer protein include any suitable method for detecting and/or measuring proteins from a cell or cell extract. Such methods include, but are not limited to, immunoblot (e.g., Western blot), enzyme-linked immunosorbant assay (ELISA), radioimmunoassay (RIA), immunoprecipitation, immunohistochemistry and immunofluorescence. Particularly preferred methods for detection of proteins include any single-cell assay, including immunohistochemistry and immunofluorescence assays. Such methods are well known in the art. Furthermore, antibodies against Mer that are particularly useful for the detection of Mer glycoforms are provided by the present invention and can be used in these methods.

A positive diagnosis of the target Mer glycoform in the individual sample indicates that tumor cell growth (neoplastic transformation), has occurred, is occurring, or is statistically likely to occur in the cells or tissue from which the sample was obtained. A negative diagnosis of the target Mer glycoform in the individual sample *(i.e.,* the Mer glycoform was not detected) means that the indicators of tumor presence or a likelihood of developing tumors as described herein have not been identified in the sample obtained from the subject. In this instance, the subject is typically not prescribed any treatment, but may be reevaluated at one or more timepoints in the future to again assess tumor growth.

The nucleic acids, probes, primers, polypeptides (including soluble Mer and Mer glycoforms) and antibodies described herein can be used in methods of diagnosis of cancers, and particularly, leukemias and lymphomas, as well as in kits useful for diagnosis of such cancers, particularly leukemias and lymphomas. For example, the invention provides methods for identifying the presence of a polynucleotide that hybridizes to a nucleic acid of the invention *(i.e.,* nucleic acids encoding aberrant Mer glycoforms or the soluble extracellular Mer), as well as for identifying the presence of a polypeptide of the invention (e.g., aberrant Mer glycoforms or the soluble extracellular Mer), using agents that detect such Mer glycoforms or soluble Mer (*e.g*., antibodies or antigen binding fragments thereof).

In one embodiment, the presence (or absence) of a nucleic acid molecule of interest *(e.g.,* a nucleic acid that has significant homology with a nucleic acid of the invention) in a sample can be assessed by contacting the sample with a nucleic acid comprising a nucleic acid of the invention (*e.g*., a nucleic acid encoding an aberrant Mer glycoform or the soluble extracellular Mer) under stringent conditions as described above, and then assessing the sample for the presence (or absence) of hybridization. In a preferred embodiment, high stringency conditions are conditions appropriate for selective hybridization. In another embodiment, a sample containing the nucleic acid molecule of interest is contacted with a nucleic acid containing a contiguous nucleotide sequence (*e.g*., a primer or a probe as described above) that is at least partially complementary to a part of the nucleic acid molecule of interest), and the contacted sample is assessed for the presence or absence of hybridization. In a preferred embodiment, the nucleic acid containing a contiguous nucleotide sequence is completely complementary to a part of the nucleic acid molecule of interest. In any of these embodiments, all or a portion of the nucleic acid of interest can be subjected to amplification prior to performing the hybridization.

More particularly, in one method of diagnosing leukemia or lymphoma, hybridization methods, such as Southern analysis, Northern analysis, or *in situ* hybridizations, can be used. Such techniques of detection are well known in the art, *see, e.g*., Current Protocols in Molecular Biology, Ausubel, F. et al., eds., John Wiley & Sons, including all current supplements. For example, a biological sample from a test subject (a "test sample") of genomic DNA, RNA, or cDNA, is obtained from an individual suspected of having leukemia (the "test individual"). The test sample can be from any source which contains genomic DNA, such as a blood sample, sample of amniotic fluid, sample of cerebrospinal fluid, or tissue sample from skin, muscle, buccal or conjunctival mucosa, placenta, gastrointestinal tract or other organs. The DNA, RNA, or cDNA sample is then examined to determine whether nucleic acid encoding variant isoforms, including but not limited to Mer isoforms in the 170 kD to 195 kD range, is present. Such a variant will be detected by this method when the glycosylation of the Mer protein results from a modification in the glycosylation sites of the protein expressed by the nucleic acid sequence from the patient sample (*e.g*., the nucleic acid encoding Mer is a variant). The presence of the isoform or splicing variant(s) can be indicated by hybridization of the gene in the genomic DNA, RNA, or cDNA to a nucleic acid probe. A "nucleic acid probe", as used herein, can be a DNA probe or an RNA probe. The probe can be any of the nucleic acid molecules described above (*e.g*., the gene, a fragment, a vector comprising the gene, a probe or primer, etc.).

The sample is maintained under conditions that are sufficient to allow specific hybridization. "Specific hybridization", as used herein, indicates exact hybridization (*e.g*., with no mismatches). Specific hybridization can be performed under high stringency conditions or moderate stringency conditions, for example, as described above. In a particularly preferred embodiment, the hybridization conditions for specific hybridization are high stringency. Hybridization is then detected by standard methods well known in the art.

Sequence analysis can also be used to detect mutations in Mer causing size differences. A test sample of DNA or RNA is obtained from the test individual. PCR or other appropriate methods can be used to amplify the gene, and/or its flanking sequences, if desired. The sequence of Mer, or a fragment of the gene, or cDNA, or fragment of the cDNA, or mRNA, or fragment of the mRNA, is determined, using standard methods. The sequence of the gene, gene fragment, cDNA, cDNA fragment, mRNA, or mRNA fragment is compared with the known nucleic acid sequence of the gene.

When aberrant glycoforms are due to mutations in the Mer gene, oligonucleotides produced from the nucleic acids of the invention that detect such differences can also be used to detect the presence of aberrant Mer glycoforms, through the use of dot-blot hybridization. An "oligonucleotide" (also referred to herein as a "probe") is a nucleic acid of approximately 10-50 base pairs, preferably approximately 15-30 base pairs, that specifically hybridizes to the sequence coding for the aberrant Mer glycoform. An oligonucleotide probe that is specific for the aberrant Mer glycoform can be prepared, using standard methods (see Current Protocols in Molecular Biology, *supra*). To do this, a test sample of DNA is obtained from the individual. PCR can be used to amplify all or a fragment of Mer, and its flanking sequences. The DNA containing the amplified Mer (or fragment of the gene) is dot-blotted, using standard methods (see Current Protocols in Molecular Biology, *supra*), and the blot is contacted with the oligonucleotide probe. The presence of specific hybridization of the probe to the aberrant Mer glycoform is then detected. Specific hybridization of an oligonucleotide probe to DNA from the individual is suggestive of leukemia or lymphoma.

Other methods of nucleic acid analysis can be directed to the sequence coding for the aberrant Mer glycoform. Representative methods include direct manual sequencing (Church and Gilbert, Proc. Natl. Acad. Sci. USA 81:1991-1995 (1988); Sanger, F. et al., Proc. Natl. Acad. Sci. 74:5463-5467 (1977); Beavis et al. U.S. Pat. No. 5,288,644); automated fluorescent sequencing; single-stranded conformation polymorphism assays (SSCP); clamped denaturing gel electrophoresis (CDGE); denaturing gradient gel electrophoresis (DGGE) (Sheffield, V. C. et al., Proc. Natl. Acad. Sci. USA 86:232-236 (19891)), mobility shift analysis (Orita, M. et al., Proc. Natl. Acad. Sci. USA 86:2766-2770 (1989)), restriction enzyme analysis (Flavell et al., Cell 15:25 (1978); Geever, et al., Proc. Natl. Acad. Sci. USA 78:5081 (1981)); heteroduplex analysis; chemical mismatch cleavage (CMC) (Cotton et al., Proc. Natl. Acad. Sci. USA 85:4397-4401 (1985)); RNase protection assays (Myers, R. M. et al., Science 230:1242 (1985)).

In another embodiment, the presence (or absence) of a polypeptide of interest, such as a polypeptide of the invention or a fragment or variant thereof, in a sample can be assessed by contacting the sample with an antibody that specifically hybridizes to the polypeptide of interest (e.g., an antibody such as those described above), and then assessing the sample for the presence (or absence) of binding of the antibody to the polypeptide of interest For example, diagnosis of cancer by diagnosing an aberrant Mer glycoform, soluble Mer, or a Mer variant, as in leukemia or lymphoma, can be made by examining expression and/or composition of the Mer polypeptide, by a variety of methods, including enzyme linked immunosorbent assays (ELISAs), Western blots, immunoprecipitations and immunofluorescence. A test sample from an individual is assessed for the presence aberrant Mer glycoforms, or for the presence of a particular variant. In a preferred embodiment, any of the antibodies or antigen binding fragments thereof described herein can be used such methods of the invention.

In one embodiment, Western blotting analysis, *e.g*., using the 311 monoclonal antibody as described herein or other antibodies that identify all glycoforms of Mer or specifically identify the aberrant glycoform of Mer as described herein can be used to identify the presence in a test sample of any in the spectrum of Mer glycoforms or of soluble Mer in a sample. Other techniques to identify the Mer glycoforms will be known in the art and some are described elsewhere herein. The presence of a Mer glycoform from about 170 kD to about 190 kD (and particularly, 185 kD) or a Mer glycoform from about 135 kD to about 140 kD (and particularly 140 kD) is diagnostic of leukemia (acute lymphoblastic leukemia). The presence of the 190-195 kD Mer glycoform is diagnostic of myelogenous leukemia, or lymphoma.

Kits (e.g., reagent kits) useful in the methods of diagnosis comprise components useful in any of the methods described herein, including for example, soluble Mer proteins, including soluble Mer glycoforms described herein, cells expressing such Mer proteins and glycoforms, hybridization probes or primers as described herein (e.g., labeled probes or primers), reagents for detection of labeled molecules, restriction enzymes (e.g., for RFLP analysis), allele-specific oligonucleotides, antibodies and antigen-binding fragments thereof, means for amplification of nucleic acids, or means for analyzing nucleic acid or amino acid sequences, positive controls etc.

### Screening Assays of the Invention

In another embodiment, the invention provides methods and screening assays for identifying agents (*e.g*., fusion proteins, polypeptides, peptidomimetics, prodrugs, receptors, binding agents, antibodies, small molecules or other drugs, ribozymes, or nucleic acids) that alter (*e.g*., increase or decrease), mimic or regulate the activity of the sMer, or aberrant Mer glycoforms or which otherwise interact with these polypeptides described herein. For example, such agents can be agents which bind to polypeptides described herein; which have a stimulatory or inhibitory effect on, for example, activity of polypeptides of the invention; or which change (*e.g*., enhance or inhibit) the ability of the polypeptides of the invention to interact with binding agents (*e.g*., receptors or other binding agents). For example, the ability of a molecule to affect the binding of the extracellular domain of Mer to its ligands presents a screening assay for molecules that affect blood clotting or for molecules that can be used to treat a particular cancer. Similarly, the ability of a molecule to inhibit the proteolysis that releases Mer from its cellular anchor, as exemplified by TAPI (see Figure 11), presents a screening assay for molecules that affect blood clotting from an alternative direction. In another example, a screening assay using the sMer can identify molecules which bind the sMer and therefore subsequently modulate (inhibit, interfere with, enhance, or otherwise affect) the Mer tyrosine kinase and inhibit the activity of Mer in cancer. For example, such assays can be in the form of examining Mer phosphorylation or glycosylation (or lack thereof) (Figure 13), and the effect on this activation on cell survival and cell proliferation. This invention also contemplates an assay for identifying protease inhibitors, more preferably a metalloprotease inhibitor and most preferably TACE inhibitors.

In a preferred embodiment, such assays take advantage of the discovery by the present inventors of the differential expression of various glycoforms of Mer. In particular, methods of the present invention can make use of particular glycoforms of Mer, including any specific glycoforms described herein, to screen for compounds (proteins, peptides, antibodies, small molecules, etc.) that selectively bind to a particular Mer glycoform (*e.g*., to one glycoform preferentially over another). In addition, methods of the present invention can be used to screen for Mer glycoforms that competitively inhibit the binding of a Mer ligand to its endogenous Mer receptor and that can be used as therapeutic agent. The methods of the present invention can also be used to screen for Mer proteins (including specific Mer glycoforms) that selectively (preferentially) bind to one Mer ligand (*e.g*., Gas6 or Protein S) over another Mer ligand.

In one embodiment, several different Mer glycoforms (*e.g*., any combination or all of the glycoforms described herein) are contacted with Mer ligands or other putative regulatory compounds to identify differences in binding affinity or Mer activity (in the case of a cell-based assay) in response to the contact with the ligand or compound. In another embodiment, additional glycoforms of Mer other than those described herein (*e.g*., having any molecular weight, as a result of glycosylation, from between about 109 kD and 210 kD or larger (in the case of Mer variants where additional glycosylation sites are added to the protein as compared to the wild-type protein), are tested for binding to Mer ligands or putative regulatory compounds. Specifically, any Mer glycoform from 100 kd to 210 kD or larger, including any size between 100 kD and 210 kD or larger, in increments of 1 kD *(i.e.,* 100 kD, 101 kD, 102 kD,...145 kD, 146 kD,...201 kD, 202 kD,...210 kD, 211 kD,...etc.) can be tested to identify Mer glycoforms that have preferred affinity for Mer ligands or putative regulatory compounds. Mer glycoforms with differential affinity for Mer ligands, or that bind to Mer ligands with a greater affinity than a natural Mer glycoform expressed by a target cell type *(e.g.,* a platelet or a tumor cell) are particularly desirable, as these glycoforms can be produced as soluble Mer glycoforms for use in various therapeutic assays *(e.g.,* as competitive inhibitors of endogenous Mer to treat a condition such as a cancer or a clotting disorder).

This invention also contemplates an assay for identifying portions of extracellular domain of Mer functions in modulating Gas6 functionalities and where the Mer domain interacts with Gas6, as well as identifying small molecule mimetics that would also interact at the same Gas6 domains.

Another aspect of the invention pertains to assays for monitoring the influence of agents (e.g., drugs, compounds or other agents) on the gene expression or activity of polypeptides of the invention, as well as to assays for identifying agents that bind to or inhibit aberrant Mer glycoforms and the soluble Mer polypeptides of the invention.

Such a method includes the steps of: (a) contacting a Mer protein with a test compound; and (b) determining whether the test compound binds to the Mer protein. The method can optionally include an additional step of determining whether the test compound activates or inhibits the biological activity of a Mer receptor, by detecting, for example, signal transduction events that are associated with Mer tyrosine kinase activity. The assay can be cell-based, in the event where Mer activity is evaluated, or non-cell based, in the event where only binding is evaluated.

A cell suitable for use in the present method is any cell which expresses or can be induced to express, a detectable level of Mer, and particularly, a specific glycoform of Mer. A detectable level of Mer is a level which can be detected using any of the methods for Mer detection described herein. Since Mer is expressed by many mammalian cell types, a variety of cell types could be selected. Preferred cell types that endogenously express particular glycoforms of Mer are described herein. Alternatively, a cell suitable for use in the method is a cell which has been transfected with a recombinant nucleic acid molecule encoding Mer and operatively linked to a transcription control sequence so that Mer is expressed by the cell. The cell can be selected to post-translationally modify the Mer protein to produce a particular glycoform of Mer, or the nucleic acid molecule encoding the Mer protein can be modified so that glycosylation sites on the Mer protein are designed to produce a particular Mer glycoform. Methods and reagents for preparing recombinant cells are known in the art.

For non-cell-based assays, soluble Mer glycoforms can be produced using any method described herein and immobilized on a suitable substrate or mixed with a test compound in an assay container.

As used herein, the term "putative regulatory compound" refers to compounds having an unknown or previously unappreciated regulatory activity in a particular process. The above-described method for identifying a compound of the present invention includes a step of contacting a test compound with a Mer receptor and particularly, a specified Mer glycoform. When cells expressing Mer are used, test cells can be grown in liquid culture medium or grown on solid medium in which the liquid medium or the solid medium contains the compound to be tested. In addition, the liquid or solid medium contains components necessary for cell growth, such as assimilable carbon, nitrogen and micronutrients.

The above-described methods, in one aspect, involve contacting cells with the compound being tested for a sufficient time to allow for interaction of the putative regulatory compound with the Mer glycoform. The period of contact with the compound being tested can be varied depending on the result being measured, and can be determined by one of skill in the art. For example, for binding assays, a shorter time of contact with the compound being tested is typically suitable, than when activity is assessed. As used herein, the term "contact period" refers to the time period during which cells are in contact with the compound being tested. The term "incubation period" refers to the entire time during which cells are allowed to grow prior to evaluation, and can be inclusive of the contact period. Thus, the incubation period includes all of the contact period and may include a further time period during which the compound being tested is not present but during which growth is continuing (in the case of a cell based assay) prior to scoring. The incubation time for growth of cells can vary but is sufficient to allow for the upregulation or downregulation of Mer biological activity in a cell. It will be recognized that shorter incubation times are preferable because compounds can be more rapidly screened. A preferred incubation time is between about 1 hour to about 48 hours.

The conditions under which the cell or cell lysate of the present invention is contacted with a putative regulatory compound, such as by mixing, are any suitable culture or assay conditions and includes an effective medium in which the cell can be cultured or in which a soluble Mer or immobilized Mer can be evaluated in the presence and absence of a putative regulatory compound. Cells of the present invention can be cultured in a variety of containers including, but not limited to, tissue culture flasks, test tubes, microtiter dishes, and petri plates. Culturing is carried out at a temperature, pH and carbon dioxide content appropriate for the cell. Such culturing conditions are also within the skill in the art. Cells are contacted with a putative regulatory compound under conditions which take into account the number of cells per container contacted, the concentration of putative regulatory compound(s) administered to a cell, the incubation time of the putative regulatory compound with the cell, and the concentration of compound administered to a cell. Determination of effective protocols can be accomplished by those skilled in the art based on variables such as the size of the container, the volume of liquid in the container, conditions known to be suitable for the culture of the particular cell type used in the assay, and the chemical composition of the putative regulatory compound (i.e., size, charge etc.) being tested. A preferred amount of putative regulatory compound(s) comprises between about 1 nM to about 10 mM of putative regulatory compound(s) per well of a 96-well plate.

Mer proteins and nucleic acid molecules encoding Mer may be recombinantly expressed and utilized in non-cell based assays to identify compounds that bind to the protein or nucleic acid molecule, respectively. In non-cell based assays the recombinantly expressed Mer or nucleic acid encoding Mer is attached to a solid substrate such as a test tube, microtiter well or a column, by means well known to those in the art.

Compounds suitable for testing and use in the methods of the present invention include any known or available proteins, nucleic acid molecules, as well as products of drug design, including peptides, oligonucleotides, carbohydrates and/or synthetic organic molecules. Such an agent can be obtained, for example, from molecular diversity strategies (a combination of related strategies allowing the rapid construction of large, chemically diverse molecule libraries), libraries of natural or synthetic compounds, in particular from chemical or combinatorial libraries (i.e., libraries of compounds that differ in sequence or size but that have the same building blocks) or by rational drug design. See for example, Maulik et al., 1997, Molecular Biotechnology: Therapeutic Applications and Strategies, Wiley-Liss, Inc., which is incorporated herein by reference in its entirety. Candidate compounds initially identified by drug design methods can be screened for the ability to modulate the expression and/or biological activity of Mer using the methods described herein.

Compounds identified by the method described above can be used in a method to regulate cell growth or thrombosis, as described below and any such compounds are encompassed for use in the method described below.

### Therapeutic Methods of the Invention

The present invention also relates to methods of treatment (prophylactic and/or therapeutic) for Mer-positive cancers and clotting disorders, using the polypeptides (including sMer and particularly, specific glycoforms of Mer, and more particularly, specific glycoforms of sMer), nucleic acid molecules, and/or antibodies of the invention.

As used herein, "treatment" refers to clinical intervention in an attempt to alter the natural course of the individual or cell being treated, and may be performed either for prophylaxis or during the course of clinical pathology. Desirable effects include preventing occurrence or recurrence of disease, alleviation of symptoms, diminishment of any direct or indirect pathological consequences of the disease, preventing metastasis, lowering the rate of disease progression, amelioration or palliation of the disease state, and remission or improved prognosis. Accordingly, a therapeutic benefit is not necessarily a cure for a particular disease or condition, but rather, preferably encompasses a result which most typically includes alleviation of the disease or condition, elimination of the disease or condition, reduction of a symptom associated with the disease or condition, prevention or alleviation of a secondary disease or condition resulting from the occurrence of a primary disease or condition (e.g., metastatic tumor growth resulting from a primary cancer), and/or prevention of the disease or condition. A beneficial effect can easily be assessed by one of ordinary skill in the art and/or by a trained clinician who is treating the patient. The term, "disease" refers to any deviation from the normal health of a mammal and includes a state when disease symptoms are present, as well as conditions in which a deviation (e.g., infection, gene mutation, genetic defect, etc.) has occurred, but symptoms are not yet manifested.

According to the present invention, the methods and assays disclosed herein are suitable for use in or with regard to an individual that is a member of the Vertebrate class, Mammalia, including, without limitation, primates, livestock and domestic pets (e.g., a companion animal). Most typically, a patient will be a human patient. According to the present invention, the terms "patient", "individual" and "subject" can be used interchangeably, and do not necessarily refer to an animal or person who is ill or sick (*i.e.,* the terms can reference a healthy individual or an individual who is not experiencing any symptoms of a disease or condition).

Diseases and disorders that are characterized by altered (relative to a subject not suffering from the disease or disorder) Mer receptor tyrosine kinases, levels of this protein, or biological activity may be treated with therapeutics that antagonize (e.g., reduce or inhibit) the altered Mer receptor tyrosine kinase or its ligands. For example, the soluble extracellular form of Mer may block the activation of the full length native Mer or aberrant glycoforms of Mer by binding to Mer ligands including Protein S and Gas6. Therefore, in another embodiment of the invention, an effective amount of an inhibitor of a Protein S or Gas6 function or of a Gas6 receptor which is provided in the form of the soluble extracellular form of Mer herein described, and particularly, in the form of a particular soluble glycoform of Mer, may be used as a treatment for diseases and conditions associated with Mer expression, including aberrant Mer expression.

Accordingly, an additional aspect of the invention pertains to methods of modulating expression or activity of Mer glycoforms. In one aspect, this is achieved by modulating the ability of ligands of Mer, including Protein S and Gas 6, to bind to endogenous Mer receptors, for therapeutic purposes. The method of the invention for example, involves contacting a cell with an agent that modulates one or more of the activities of Mer. One such agent includes an anti-Mer antibody or antigen-binding fragment thereof that binds to and inhibits Mer activity in a cell. Another agent that modulates Mer activity is preferably a soluble Mer protein described herein and particularly, a soluble Mer glycoform that binds to Mer ligands and serves as a competitive inhibitor of Mer expressed by cells. Additionally, nucleic acid molecules, peptides and small molecules that target specific Mer glycoforms can be used in therapeutic embodiments of the invention. These methods can be performed *in vitro* (*e.g.,* by culturing the cell with the agent) or, alternatively, *ex vivo* or *in vivo* (*e.g.,* by administering the agent to a subject). As such, the invention provides methods of treating an individual afflicted with a disease or disorder, specifically a clotting disorder or a cancer. In a preferred embodiment, the method involves administering a reagent (*e.g*., a specific glycoform of soluble extracellular Mer polypeptide described herein or an antibody that selectively binds to a glycoform of Mer), or combination of reagents that modulate Mer activity (*e.g*., binding and/or activation of Mer by a cognate ligand).

In one embodiment of the invention, modulation of specific Mer glycoforms is contemplated to prevent thrombosis or any clotting disorder without causing bleeding side effects. According to the present invention, "modulation" refers to any type of regulation, including upregulation, stimulation, or enhancement of expression or activity, or downregulation, inhibition, reduction or blocking of expression or activity. Preferably, the method of the present invention specifically inhibits the activity of the Mer glycoform expressed by platelets. Inhibition can be accomplished through various means, most preferably, but not limited to, by providing variants of the sMer, including differentially glycosylated Mer proteins and sMer proteins and peptides which bind directly to the extracellular domain of Mer or bind and competitively inhibit Mer ligands including Protein S and Gas6. Inhibition is also accomplished through antibodies or antigen-binding fragments thereof that are specific for the platelet glycoform of Mer, and most preferably the 165-170kD glycoforms. Inhibition can also be achieved by providing to a patient small molecules, peptides, nucleic acids or other inhibitors which preferentially bind to and inhibit or block the glycosylated Mer extracellular domains that are expressed on platelets, or that bind to and inhibit or block the Mer ligands including protein S and Gas6. More preferably, all of these embodiments target the Mer glycoform that is specific to platelets as disclosed herein.

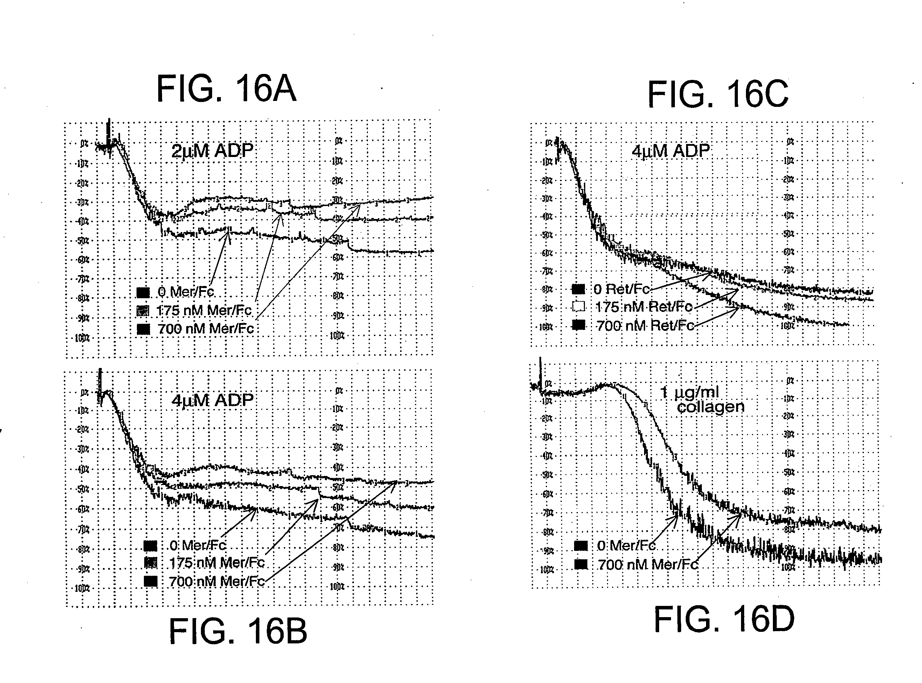

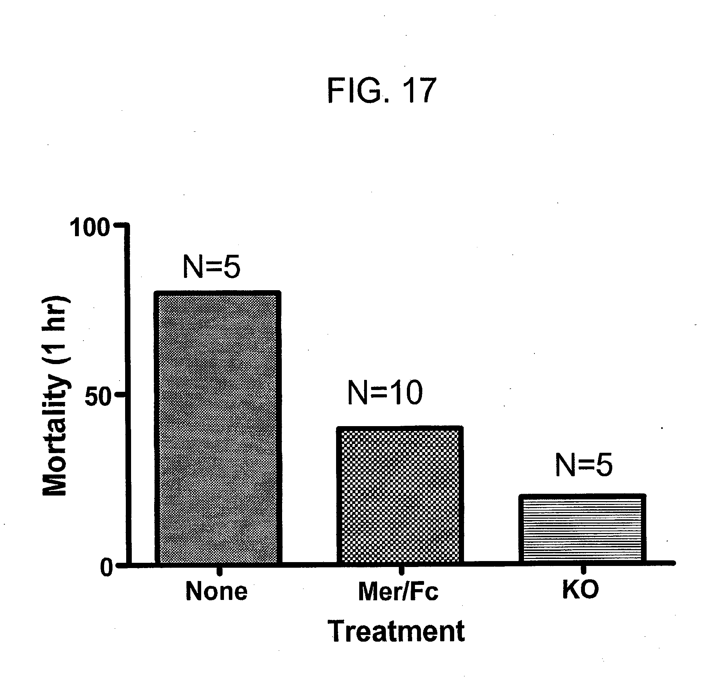

Modulation of Mer activity by administering antibodies to Mer ligand Gas6 has demonstrated protection of wild type mice against fatal thromboembolism to the same degree as genetic inactivation of this ligand, while not causing spontaneous bleeding. Further, the use of a version of sMer in the same mouse model as above inhibited platelet *in vitro* (Figure 16) aggregation and protected against fatal pulmonary embolism *in vivo* (Figure 17). Thus, the invention further contemplates modulating signaling through the Mer receptor as an anti-thrombotic approach, which is safer than currently available antiplatelet drugs. Thus, Mer, and in particular, the truncated or soluble form of Mer and its mimetics, and most preferably the soluble glycoform of Mer that is specific to platelets, are valuable prophylactic agents useful in the treatment and prevention of thrombotic events or disorders.

Clotting disorders that can be treated by the method of the invention include, but are not limited to, thrombophilia (including inherited traits predisposing an individual to have a higher risk of clotting), thrombosis or thrombo-embolic disorder. Specifically, this method of treatment could be applied to patients on medications (including, but not limited to, estrogens and chemotherapy) which increase the risk of clotting as well as diseases associated with thrombosis (including, but not limited to, cancer, myeloproliferative disorders, autoimmune disorders, cardiac disease, inflammatory disorders, atherosclerosis, hemolytic anemia, nephrosis, and hyperlipidemia). In addition, this method of treatment could be applied to predisposing factors to increased clotting including surgery, trauma, or pregnancy. Finally, this method of treatment may be appropriate for patients with adverse side effects from other anticoagulant or anti-platelet therapies, including heparin-induced thrombocytopenia (a severe immune-mediated drug reaction that occurs in 2-5% of patients exposed to heparin.)

Accordingly, the present invention provides for a method of treating an individual who has or is likely to develop a clotting disorder, comprising modulating the level of soluble extracellular Mer transmembrane receptor kinase in the blood. In one aspect, the invention includes a step of administering (*e.g*., by any suitable route, including infusion) an effective amount of a soluble extracellular Mer transmembrane receptor kinase into the individual, especially administration of the soluble extracellular domain of the Mer glycoform that is expressed by platelets (165-170 kD). An effective amount is any amount that achieves any detectable inhibition of the Mer receptor in the patient, or any detectable reduction in at least one symptom of the clotting disorder.

In another aspect, modulation occurs by administration of an agent that cleaves the extracellular domain of the Mer transmembrane receptor tyrosine kinase, preferably a metalloprotease and most preferably a TACE-like metalloprotease. Alternatively, modulation can be achieved by *inhibiting* cleavage of the Mer transmembrane receptor, preferably using a protease inhibitor, more preferably a metalloprotease inhibitor and most preferably using a TACE-like metalloprotease inhibitor.

In another aspect of the invention, clotting disorders are treated by administration of an agent that affects a post-translational proteolytic cleavage at the Mer extracellular domain. The cleaved extracellular domain becomes soluble after cleavage. This cleavage produces an inhibitory effect that is twofold: (1) competitive inhibition for Mer ligands (Figure 6) and (2) eliminating functional activity of the Mer receptor tyrosine kinase (Figures 8A and 8B). In a preferred embodiment, the agent is specific for the glycoform of Mer that is expressed by platelets.

In one embodiment of the invention, the cleavage agent is a protease capable of cleaving Mer, including TACE or a TACE-like metalloprotease. In an additional aspect of the invention, an sMer produced by cleavage of the membrane bound Mer directly binds to Mer ligands, such as Protein S or Gas6, and prevents activation of the full length Mer receptor tyrosine kinase. Additionally, treatment of patients with a clotting disorder may occur by modulation of sMer levels in the blood. This may be accomplished by sMer infusion (particularly using sMer glycoforms of the invention) or targeting the metalloprotease activity affecting endogenous sMer levels.

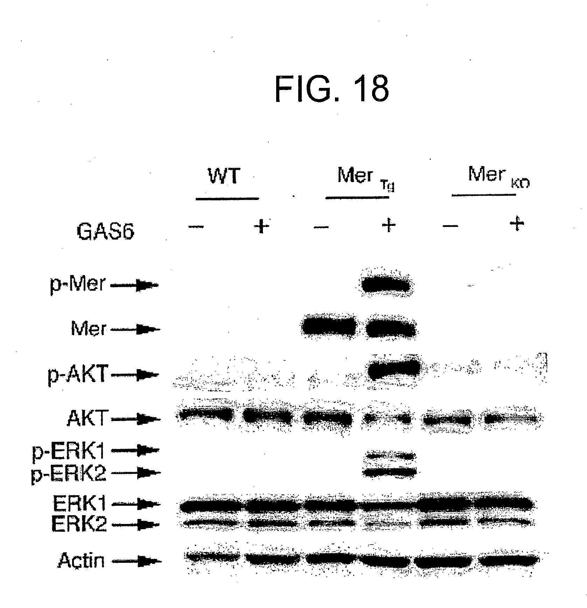

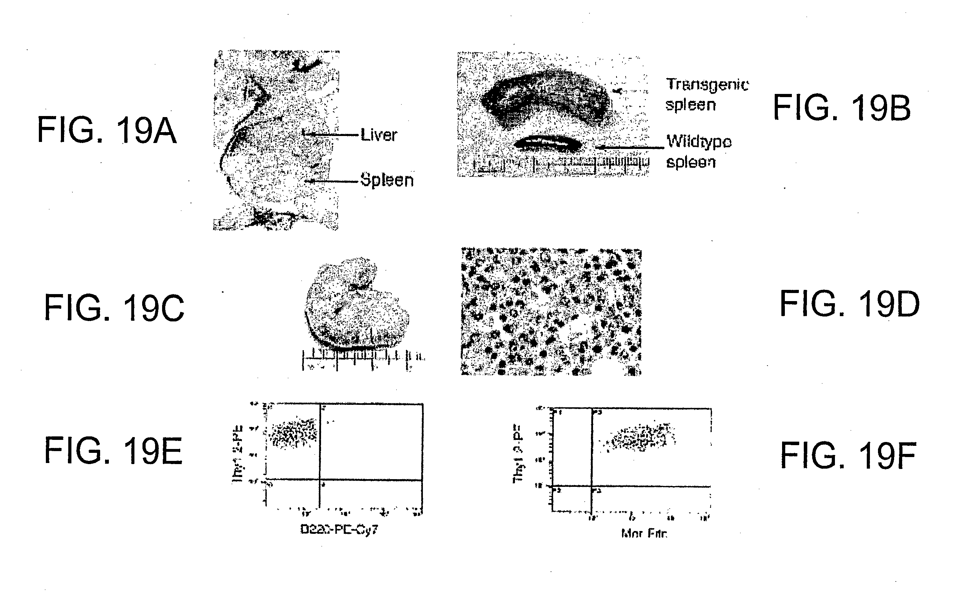

Mer activation leads to downstream activation of survival pathways (e.g., Akt and Erk 1/2) (Figure 18), and in some instances proliferation pathways, that are known to contribute to the development of cancer. In some cancers, overexpression or ectopic expression of Mer glycoforms has been noted on the surface of cancer cells, as described herein. Furthermore, the overexpression of Mer and activation of downstream signaling pathways leads to lymphoblastic leukemia and lymphoma in a Mer transgenic mouse model (Figure 19). The abnormal expression of Mer glycoforms makes this protein an attractive target for biologically targeted cancer therapy. Therefore, a further embodiment of the invention contemplates inhibition of the aberrantly glycosylated forms of the Mer receptor tyrosine kinase as part of a therapeutic strategy which selectively targets cancer cells. Any of the above-described methods and agents for treating a clotting disorder can be applied to the treatment of cancers. However, in these embodiments, the Mer that is targeted by the therapeutic method is preferably a Mer glycoform that is specifically expressed by a tumor cell (*i.e*., an aberrant Mer glycoform).

In one embodiment, the soluble extracellular portion of the Mer receptor tyrosine kinase, and particularly, a glycoform of sMer that selectively inhibits the Mer expressed by the target cancer cell, is used to block Mer activation and subsequent downstream signaling survival pathways, including AKT and Erk 1/2 as well as Mer specific proliferation pathways.

In another embodiment, small molecule inhibitors, nucleic acids, ribozymes, peptides or other drugs targeting aberrant Mer tyrosine kinase glycoforms or proteins directly downstream of Mer may be engineered and used in treatment regimens for Mer positive cancers. Antibodies (naked or coupled to toxins or radioactive' materials) that bind to leukemia, lymphoma, or other cancer-specific forms of Mer protein can also be used therapeutically to treat Mer positive patients with cancer. For example, antibodies or antigen-binding fragments that are specific for (selectively bind to) the glycoforms of Mer that are expressed by the leukemia and lymphoma cells as described herein are particularly useful therapeutic agents according to the present invention.

In another aspect of the invention, cancers positive for surface expression of Mer are treated by administration of an agent that affects a post-translational proteolytic cleavage at the Mer extracellular domain. Such agents have been described above with respect to the treatment of clotting disorders, but can also be applied to the treatment of cancer according to the invention.

In the therapeutic methods of the invention, suitable methods of administering a composition of the present invention to a subject include any route of *in vivo* administration that is suitable for delivering the composition. The preferred routes of administration will be apparent to those of skill in the art, depending on the type of delivery vehicle used, the target cell population, whether the compound is a protein, nucleic acid, or other compound (e.g., a drug or an antibody) and the disease or condition experienced by the patient.

When the agent to be administered to a patient is a protein, small molecule (i.e., the products of drug design) or antibody, a preferred single dose of such a compound typically comprises between about 0.01 microgram x kilogram⁻¹ and about 10 milligram x kilogram⁻¹ body weight of an animal. A more preferred single dose of an agent comprises between about 1 microgram x kilogram⁻¹ and about 10 milligram x kilogram⁻¹ body weight of an animal. An even more preferred single dose of an agent comprises between about 5 microgram x kilogram⁻¹ and about 7 milligram x kilogram⁻¹ body weight of an animal. An even more preferred single dose of an agent comprises between about 10 microgram x kilogram⁻¹ and about 5 milligram x kilogram⁻¹ body weight of an animal. Another particularly preferred single dose of an agent comprises between about 0.1 microgram x kilogram⁻¹ and about 10 microgram x kilogram⁻¹ body weight of an animal, if the agent is delivered parenterally.

The invention now being generally described will be more readily understood by reference to the following examples.

### Examples

### Example 1: Human Mer Monoclonal antibody 311 recognizes unique 185 kD protein in Jurkat Leukemia Cell Line

The human Mer monoclonal antibody 311 produced by standard techniques, as described above, was tested on a Western Blot against the protein component of the human monocyte macrophage cell line U937 and the T cell leukemia line Jurkat. The human cervical carcinoma cell line HeLa was also included as a negative control. Both U937 and Jurkat express Mer mRNA by testing with RT/PCR while HeLa does not express Mer mRNA (results not shown). The 311 antibody recognized distinct size differences in the Mer protein (Figure 1). The monocyte line U937 showed the expected banding at 205 kD. Unexpectedly, a 185 kD protein was found in the acute lymphocytic cell line (Jurkat).

Fig. 1 shows that the monoclonal antibody 311 directed against human Mer recognizes a 205 kD protein in the monocytic cell line U937 and a 185 kD protein in the Jurkat leukemia cell line on a Western blot. Hela cells, which do not express Mer mRNA by RT/PCR, were used as a negative control for this antibody.

### Example 2: Human Mer 185 kD protein in leukemia cell lines is distinct from Mer in other hematopoietic lineages

A Western Blot of the cell line U937 (human monocyte macrophage), K562 (human myeloid leukemia), Jurkat and HSB-2 (both myeloid leukemia) and normal human platelets was probed with the 311 monoclonal antibody. The Blot was probed with the anti-Mer antibody 311. The results are shown in Figure 2. The 311 antibody demonstrates distinctly migrating bands in all of the cell lines. Also seen in the platelet sample lane is soluble Mer extracellular domain (sMer, indicated by arrows), which is present in human plasma.

Fig. 2 is a Western blot probed with monoclonal antibody 311, demonstrating distinctly migrating forms of Mer in U937 (monocytic), K562 (myeloid), Jurkat (T-cell leukemia) and HSB-2 (T-cell leukemia) cell lines and in platelets. Also seen in the platelet sample lane is soluble Mer extracellular domain (sMer, arrows), which is present in human plasma.

### Example 3: Deglycosylation confirms unique Mer protein in Jurkat leukemia cells

U937, Jurkat, or K562 cell lysates were either untreated (-), digested with the glycosidase PNGase F (+ PNG), or digested with a mixture of 5 glycosidases (+ deglyc. mix). The 5-enzyme mix should remove all N-linked (Asn-linked) and most O-linked (Ser or Thr linked) carbohydrates from the glycoproteins. Digested lysates were fractionated by SDS-PAGE and probed with anti-Mer antibody 311 (Figure 3). The Mer glycoform present in Jurkat leukemia cells migrates differently after each of these treatments compared to Mer from the other two cell lines, revealing differences in glycosylation patterns.

### Example 4: Detection of aberrant Mer glycoforms in T cell ALL patient samples

Figure 4 illustrates a Western blot with anti-human Mer monoclonal antibody showing the control cell lines, A459 (lung carcinoma) and Jurkat (T-cell leukemia); 9 Mer positive T cell acute lymphoblastic leukemia (ALL) patient samples; 3 negative samples; and a thymus from a normal newborn. An additional 4 negative patient samples are not shown. The Blot was probed with the anit-Mer antibody 311. Two different glycosylation forms of Mer, 185kD and 140kD, are evident in the cell lines and patient samples. The middle panel demonstrates the presence of Axl, another member of the Mer tyrosine kinase family, only in the A459 cell line. There is no Axl detected in the T-ALL cell lines or patient samples. Actin standardization is shown in the bottom panel as a loading control.

### Example 5: Mutations/Truncations of Mer Protein Exist in Some Patient Samples

Figs. 5A-5C are Western blots showing the Jurkat T-ALL cell line (Fig. 5A) and two patient samples, 3877 (Fig. 5B) and 3554 (Fig. 5C), deglycosylated with the enzyme PNGase F. Mer is indicated by the arrowheads. The predominant Mer glycoform present in Jurkat cell line and patient 3877 is 185kD before deglycosylation and 110 kD after PNGase treatment. The predominant Mer glycoform in patient 3554 is 150kD prior to deglycosylation and 90 kD after treatment.

### Example 6: Protein S and Gas6 are Ligands for Mer

Fig. 6 shows that Mer can be activated by Protein S or Gas6. Thymocytes from Mer transgenic mice were starved in serum free medium for 2 hours and then treated with the indicated concentrations of hProtein S or mGas6. Inhibition of Mer activation by co-incubation with Mer/Fc was also performed where indicated. Activated Mer was assessed by immunoblot blot using anti-phospho-Mer antibody.

### Example 7: Soluble Mer is Shed into the Medium of Cultured Cells

Figs. 7A-7D show that Mer extracellular domain is released into the medium of cultured cell lines. J774 (mouse; Fig. 7A) or U937 (human; Fig. 7B) monocytic cells were grown overnight in serum-free medium. The conditioned medium (CM) was collected and concentrated 10 fold, cells were lysed and a sample of each CM was deglycosylated with PNGase F. Fig. 7C shows the concentrated CM from overnight cultures of human cell lines. Fig. 7D shows the concentrated CM from HSB-2 and U937 cells untreated and digested with PNGase F. Cell lysates (cells), medium (CM), and PNGase-digested medium (CM+PNG) were analyzed by SDS-PAGE and immunoblotting with antibodies against mouse (Fig. 7A) or human (Figs. 7B-7D) Mer extracellular domain.

As shown Figs. 7A-7D, a soluble Mer protein (sMer) of 120-140 kD was shed into the medium by these macrophage cell lines. The sMer was reduced to an apparent size of 60kD after PNGase treatment, which is consistent with the predicted size of unmodified extracellular domain cleaved close to the transmembrane sequence. Similar results indicate that the soluble protein is also release in human cells (U937).

### Example 8: LPS and PMA Induce Mer Ectodomain Shedding

Figs. 8A-8D show LPS or PMA stimulate release of Mer ectodomain. Surface expression of Mer on J774 cells treated with 100ng/ml LPS for 4 hr (Fig. 8A) or treated with 50 nM PMA for 45 hr (Fig. 8B)was evaluated by flow cytometry. Expression on untreated cells at each timepoint is shown for comparison. (Fig. 8C) J774 cells were incubated in serum free medium with or without 100ng/ml LPS or (Fig. 8D) treated with 50 nM PMA for the indicated times. Mer present in cell lysates and sMer released into the medium at each timepoint were analyzed by immunoblotting.

### Example 9: Soluble Mer in Mice

Soluble Mer shed from peritoneal macrophages (PMΦ) and from splenocytes, as well as Mer present in normal mouse serum was analyzed (Fig. 9). Wildtype mice were injected with thioglycollate to elicit macrophage accumulation in the peritoneal cavity. Three days after injection peritoneal macrophages were collected and then cultured in serum-free medium for 7 hours. Spleens from wildtype mice were passed through a cell strainer to yield a single cell suspension, and the splenocytes were also cultured in serum-free medium for 7 hours. Cell lysates and conditioned medium from the cultured macrophages and splenocytes, and serum from wildtype mice or Mer knockout mice (KO) (negative control) were analyzed by SDS-PAGE and immunoblotting. This experiment shows that the soluble form of Mer is also released from spleen cells (in addition to cultured cells). Large amounts of soluble Mer was also detected in the serum. No proteins were detected in serum of Mer KO mice using the anti-mouse Mer antibody.

Fig. 9 shows that sMer is shed from primary cells in culture and is detected in mouse blood. Three days after wildtype C57/B6 mice were injected with thioglycollate, peritoneal macrophages were harvested and cultured in serum-free medium for 7 hours. Spleens from wildtype mice were passed through a cell strainer to yield a single cell suspension, and the splenocytes were also cultured in serum-free medium for 7 hours. Cell lysates and conditioned medium from the cultured macrophages and splenocytes, and 5 01 serum from wildtype mice or mice with the Mer gene disrupted (KD) were analyzed by SDS-PAGE and immunoblotting.

### Example 10: Soluble Mer is present in human blood

Human lung microvascular endothelial cells (MVEC), human platelets and plasma samples were examined for the presence of Mer by Western blotting (Fig. 10). A 165 kD Mer protein was detected in pelleted platelets, and soluble Mer extracellular domain proteins of 110-140 kD were abundant in the plasma from this platelet preparation (labeled "platelet CM"). U937 cell lysate was a positive control for Mer receptor expression. The right panel shows platelet preparation plasma as well as two samples of pooled plasma (George King Bio-Medical, Inc.) from normal donors or from patients who had been treated with the anticoagulant coumadin. Gas6 is a vitamin K dependent ligand for Mer that are affected by coumadin. Treatment with the anticoagulant appears to increase Mer cleavage and more soluble Mer is detected.

Fig. 10 shows human lung microvascular endothelial cells (MVEC), platelets, and plasma samples were examined for the presence of Mer by Western blotting. Cultured MVEC express a 185 kD Mer glycoform, and soluble Mer was present in conditioned medium from these cells. A 165 kD Mer protein was detected in pelleted platelets, and soluble Mer extracellular domain proteins of 110-140 kD were abundant in pooled plasma from normal donors (George King Bio-Medical, Inc.) and in plasma from free-flowing blood (plasma), and subsequently after clotting, in serum from the same donor (serum).

### Example 11: Specific Metallopnotease Inhibitor (TAPI) Blocks Production of Soluble Mer

Cultured mouse J774 cells were treated with 50 ng/ ml lipopolysaccharide (LPS) to stimulate the cleavage of the Mer extracellular domain. Metalloprotease inhibitors were added to cell cultures as indicated. Mer remaining in cells and sMer released into the medium after each treatment were detected by Western blot with anti-mouse Mer (Fig. 11). As expected, the amount of soluble Mer increased in the medium after LPS treatment. Cleavage of Mer was reduced by the addition of EDTA and almost completely blocked by TAPI, a specific inhibitor of the metalloptotease TACE , which cleaves pro-TNFα and other transmembrane proteins. DMSO (which was used to dissolve TAPI) did not affect LPS-induced cleavage of Mer.

Fig. 11 shows LPS-induced production of sMer is blocked by metalloprotease inhibitors. J774 cells were treated with 50 ng/ml (LPS) to stimulate the cleavage of the Mer extracellular domain. 5 mM EDTA , 200 □M TAPI-0 or DMSO (vehicle used to dissolve TAPI) were added to cell cultures as indicated for 2 hours. Mer remaining in cells and sMer released into the medium after each treatment were detected by Western blot.

### Example 12: Mer/Fc (sMer) Binds to Gas6

The soluble Mer receptor ectodomain was shown to bind to its ligand Gas6 in this *in vitro* pulldown assay (Figs. 12A-12B). A chimeric protein consisting of the Mer extracellular domain fused to the Fc region of human IgG was incubated with purified Gas6. As a control for nonspecific binding, a parallel experiment was performed with a TNFα receptor ectodomain /Fc chimera and Gas6. Complexes were pulled down with protein A Sepharose beads, run on SDS-PAGE gels, and bound Gas6 was detected by immunoblotting with anti-mouse Gas6 antibody. Receptor/Fc proteins were purchased from R&D Systems.

Figs. 12A and 12B show that the soluble ectodomain of Mer binds to Gas6. A chimeric protein consisting of the Mer extracellular domain fused to the Fc region of human IgG was incubated with recombinant mouse Gas6. As a control for nonspecific binding, a parallel experiment was performed with a Ret receptor ectodomain /Fc chimera and mGas6. Complexes pulled down with protein G Sepharose beads and input rmGas6 were run on SDS-PAGE gels, and bound Gas6 was detected by immunoblotting with anti-mouse Gas6 antibody.

### Example 13: Mer/Fc (sMer) Inhibits Gas6 Signaling

Soluble Mer ectodomain can block Gas6 activation of Mer in mouse cells. Figs. 13A-13B show that the soluble ectodomain of Mer inhibits Gas6 signaling. J774 cells were starved for 2 hours in serum-free medium and then treated for 10 min. with 200 nM mGas6 and 200 nM Mer/Fc or Ret/Fc as indicated. Cell lysates were analyzed for phospho-Mer and total Mer content. As shown in Figs. 13A and B, J774 cells incubated with or without murine Gas6 and Mer/Fc (Fig. 13A) and phospho- AKT and total AKT (Fig. 13B) was monitored.

### Example 14: Mer/Fc (sMer) Proteins are Glycosylated Differently in Mammalian and Insect Cells

Fig. 14 shows that Mer/Fc (sMer) proteins are glycosylated differently in mammalian and insect cells. HEK293 cells were transfected with a plasmid encoding the human Mer extracellular domain coupled to the Fc region of human IgG. Shown is Mer/Fc (sMer) secreted into the culture medium by transfected HEK293 cells and a sample of Mer/Fc expressed from a baculovirus vector in Sf21 insect cells (R&D Systems).

### Example 15: Mer. Expression is Associated with Lack of Surface CD3

Fig. 15 shows that in a double-blinded retrospective chart review of 16 T cell ALL patients, there was a statistically significant association between the detection of Mer in lymphoblasts and the lack of surface expression of CD3. Lymphoblasts lacking CD3 are derived from an immature stage of thymic differentiation and have been associated with decreased event-free survival compared to CD3 surface positive lymphoblasts.

### Example 16: Mer/Fc (sMer) Inhibits Platelet Aggregation In Vitro

Figs. 16A-16D show that Mer extracellular domain inhibits platelet aggregation induced by ADP and collagen. *In vitro* platelet aggregation was performed using human platelet rich plasma and was analyzed on a BioData aggregometer. As shown in Figs. 15A and 15B, aggregation response of platelets in response to 2 mM ADP (Fig. 16A) or 4 mM ADP (Fig. 16B) following preincubation with different concentrations of Mer/Fc. Fig. 16C shows the platelet aggregation induced by 4 mM ADP after pretreatment with Ret/Fc. Fig. 16D shows the aggregation of platelets in response to 10 □ g/ml collagen with and without preincubation with Mer/Fc. Squares on the X axis represent 15 second intervals. Data shown are representative of three independent experiments.

### Example 17: Mer/Fc (sMer) Protects Against Fatal Thromboembolism

Fig. 17 shows the inactivation of the Mer gene or inhibition of Mer activation protects mice against thrombosis. Thromboembolism induced by collagen-epinephrine injection was monitored in control wildtype mice, wildtype mice pretreated with Mer/Fc protein and in mice with the Mer gene disrupted (KO).

### Example 18: Activation of Mer with Gas6 Leads to Activation of Pro-Survival Pathways AKT and ERK ½

Fig. 18 shows that the activation of Mer with Gas6 leads to activation of pro-survival pathways AKT and ERK 1/2. Wildtype (WT), Mer knockout (Mer KO), and Mer transgenic (Mer Tg) thymocytes were serum starved in the serum free medium for three hours and treated with 150 nM Gas6 for 10 minutes. Cell lysates were analyzed for phospho-Mer, Mer, phospho-AKT, AKT, phospho-ERK 1/2, ERK 1/2 and actin.

### Example 19: Mer Transgenic Mice Develop Lymphoblastic Leukemia/Lymphoma

Fig. 19 shows that Mer transgenic mice develop lymphoblastic leukemia/lymphoma. Fig. 19A shows hepatosplenomegaly in Mer transgenic mouse with lympoblastic leukemia/lymphoma. As shown in Figs. 19B and 19C, splenomegaly (Fig. 19B) and intra-abdominal lymphoma (Fig. 19C) are noted in Mer transgenic mice with lympoblastic leukemia/lymphoma. Fig. 19D shows an H and E stain of intra-abdominal lymphoma confirming the presence of lymphoblasts. In Figs. 19E and 19F, lymphoblasts from Mer transgenic mice have been characterized by flow cytometry as T cell (Thy 1.2 positive) (Fig. 19E) and Mer positive (Fig. 19F).

## Claims

1. A method of diagnosing leukemia or lymphoma in a human, comprising detecting glycoforms of human Mer transmembrane receptor tyrosine kinase (Mer) in lymphocytes obtained from a human individual, wherein the expression of glycoforms of the human Mer transmembrane receptor tyrosine kinase selected from the group consisting of a Mer glycoform having a molecular weight between 170 kD and 190 kD, a Mer glycoform having a molecular weight between 135 kD and 140 kD, and a Mer glycoform having a molecular weight between 190 kD and 195kD, by lymphocytes in the individual is indicative of leukemia or lymphoma.

2. The method of claim 1, wherein the leukemia is a lymphoblastic leukemia, and wherein the method includes the detection of at least one Mer glycoform having a molecular weight of between 170 kD and 190 kD, or having a molecular weight between 135 kD and 140 kD.

3. The method of claim 2, wherein when a Mer glycoform having a molecular weight of between 170 kD and 190 kD is detected, the method further comprises detecting the amount of the Mer glycoform expressed by the cells, wherein expression of Mer and lack of CD3 expression by the cells, indicates a poor prognosis for the individual.

4. The method of claim 1, wherein the leukemia is a myelogenous leukemia or lymphoma, and wherein the method includes the detection of a Mer glycoform having a molecular weight of between 190 kD and 195 kD.

5. The method of any one of claims 1 to 4, wherein the step of detection comprises contacting the sample with an antibody or antigen binding fragment thereof that selectively binds to said Mer transmembrane receptor tyrosine kinase glycoform.

## Patentansprüche

1. Verfahren zur Diagnose von Leukämie oder Lymphom, bei dem man anomale Glykoformen von menschlicher Mer-Transmembranrezeptor-Tyrosinkinase (Mer) in von einem menschlichen Individuum erhaltenen Lymphozyten nachweist, wobei die Expression von Glykoformen der menschlichen Mer-Transmembranrezeptor-Tyrosinkinase, ausgewählt aus der Gruppe bestehend aus einer Mer-Glykoform mit einem Molekulargewicht zwischen 170 kD und 190 kD, einer Mer-Glykoform mit einem Molekulargewicht zwischen 135 kD und 140 kD und einer Mer-Glykoform mit einem Molekulargewicht zwischen 190 kD und 195 kD, durch Lymphozyten in dem Individuum Leukämie oder Lymphom anzeigt.

2. Verfahren nach Anspruch 1, wobei es sich bei der Leukämie um eine lymphoblastische Leukämie handelt und wobei das Verfahren den Nachweis wenigstens einer Mer-Glykoform mit einem Molekulargewicht zwischen 170 kD und 190 kD oder mit einem Molekulargewicht zwischen 135 kD und 140 kD enthält.

3. Verfahren nach Anspruch 2, wobei, wenn eine Mer-Glykoform mit einem Molekulargewicht zwischen 170 kD und 190 kD nachgewiesen wird, das Verfahren ferner das Nachweisen der Menge der durch die Zellen exprimierten Mer-Glykoform umfasst, wobei die Expression von Mer und das Fehlen von CD3-Expression durch die Zellen eine schlechte Prognose für das Individuum anzeigt.

4. Verfahren nach Anspruch 1, wobei es sich bei der Leukämie um eine myeloische Leukämie handelt und wobei das Verfahren den Nachweis einer Mer-Glykoform mit einem Molekulargewicht zwischen 190 kD und 195 kD enthält.

5. Verfahren nach einem der Ansprüche 1 bis 4, wobei der Nachweisschritt das Inkontaktbringen der Probe mit einem Antikörper oder einem antigenbindenden Fragment davon, der bzw. das selektiv an die Mer-Transmembranrezeptor-Tyrosinkinase-Glykoform bindet, umfasst.

## Revendications

1. Procédé de diagnostic d'une leucémie ou d'un lymphome chez un humain, comprenant la détection de glycoformes de récepteur tyrosine kinase transmembranaire Mer humain (Mer) dans des lymphocytes obtenus à partir d'un individu humain, dans lequel l'expression de glycoformes du récepteur tyrosine kinase transmembranaire Mer humain choisi dans le groupe constitué d'une glycoforme Mer ayant un poids moléculaire entre 170 kD et 190 kD, une glycoforme de Mer ayant un poids moléculaire entre 135 kD et 140 kD, et une glycoforme de Mer ayant un poids moléculaire entre 190 kD et 195 kD, par des lymphocytes dans l'individu est indicatrice d'une leucémie ou un lymphome.

2. Procédé de la revendication 1, dans lequel la leucémie est une leucémie lymphoblastique, et le procédé comprenant la détection d'au moins une glycoforme de Mer ayant un poids moléculaire entre 170 kD et 190 kD, ou ayant un poids moléculaire entre 135 kD et 140 kD.

3. Procédé de la revendication 2, dans lequel, lorsqu'une glycoforme de Mer ayant un poids moléculaire entre 170 kD et 190 kD est détectée, le procédé comprend en outre la détection de la quantité de la glycoforme de Mer exprimée par les cellules, où l'expression de Mer et l'absence d'expression de CD3 par les cellules, indique un pronostic médiocre pour l'individu.

4. Procédé de la revendication 1, dans lequel la leucémie est une leucémie ou un lymphome myéloïde, et le procédé comprenant la détection d'une glycoforme de Mer ayant un poids moléculaire entre 190 kD et 195 kD.

5. Procédé de l'une quelconque des revendications 1 à 4, dans lequel l'étape de détection comprend la mise en contact de l'échantillon avec un anticorps ou un fragment de liaison d'antigène de celui-ci qui se lie sélectivement à ladite glycoforme de récepteur tyrosine kinase transmembranaire Mer.
